# EUROPEAN PATENT SPECIFICATION

(11) **EP 3 277 674 B1**
(45) Date of publication and mention of the grant of the patent: **11.09.2019**
(21) Application number: 16711861.1
(22) Date of filing: 29.03.2016
(51) Int. Cl.: C07D 401/06, A01N 43/653

(54) **TRIAZOL DERIVATIVES AS FUNGICIDES**
TRIAZOLDERIVATE ALS FUNGIZIDE
DÉRIVÉS DE TRIAZOLE COMME FUNGICIDES

(30) Priority: 02.04.2015 EP 15162422
(43) Date of publication of application: 07.02.2018
(73) Proprietor: Bayer CropScience Aktiengesellschaft, 40789 Monheim am Rhein (DE)
(72) Inventor: SUDAU, Alexander, 40764 Langenfeld (DE); HOFFMANN, Sebastian, 41470 Neuss (DE); DAHMEN, Peter, 41470 Neuss (DE); WACHENDORFF-NEUMANN, Ulrike, 56566 Neuwied (DE); BERNIER, David, 69004 Lyon (FR); MILLER, Ricarda, 69002 Lyon (FR); COQUERON, Pierre-Yves, 69009 Lyon (FR); GENIX, Pierre, 69004 Lyon (FR); WITTROCK, Sven, 10117 Berlin (DE); VORS, Jean-Pierre, 69110 Sainte Foy Les Lyon (FR); KENNEL, Philippe, 06410 Biot (FR); BRUNET, Stephane, 01390 St Andre De Corcy (FR); NAUD, Sébastien, 69003 Lyon (FR); MEISSNER, Ruth, 51375 Leverkusen (DE)
(74) Representative: BIP Patents
(86) International application number: PCT/EP2016/056768
(87) International publication number: WO 2016/156294

(56) References cited:
- EP-A1- 0 440 372
- WO-A1-95/06047
- WO-A1-2014/167009
- WO-A2-2012/177603
- DICKINSON, ROGER P. ET AL: "Novel antifungal 2-aryl-1-(1H-1,2,4-triazol-1-yl)butan-2-ol derivatives with high activity against Aspergillus fumigatus", BIOORGANIC & MEDICINAL CHEMISTRY LETTERS , 6(16), 2031-2036 CODEN: BMCLE8; ISSN: 0960-894X, 1996, XP055267964, DOI: 10.1016/0960-894X(96)00363-0 10.1016/0960-894X(96)00363-0

## Description

The present invention relates to novel triazole derivatives, to processes for preparing these compounds, to compositions comprising these compounds, and to the use thereof as biologically active compounds, especially for control of harmful microorganisms in crop protection and in the protection of materials and as plant growth regulators.

It is already known that particular alkyl-substituted triazole derivatives can be used in crop protection as fungicides (cf. CN 1760193 A). It is also known that particular triazole derivatives can be used in several pharmaceutical indications and in crop protection as fungicides (cf. EP-A 0440372, WO-A 95/06047, WO-A 2012/177635, WO-A 2012/177638, WO-A 2012/177603, WO-A 2012/177608, WO-A 2012/177725, WO-A 2012/177728, WO-A 2014/167008, WO-A 2014/167009).

Since the ecological and economic demands made on modern active ingredients, for example fungicides, are increasing constantly, for example with respect to activity spectrum, toxicity, selectivity, application rate, formation of residues and favourable manufacture, and there can also be problems, for example, with resistances, there is a constant need to develop novel fungicidal compositions which have advantages over the known compositions at least in some areas.

Accordingly, the present invention provides novel triazole derivatives of the formula (I) 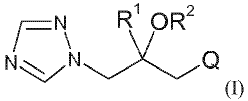 wherein
- R¹: represents phenyl or naphthyl; wherein the phenyl or naphthyl may be non-substituted or substituted by one or more group(s) selected from halogen; hydroxyl; cyano; amino; sulfanyl; pentafluoro-λ⁶-sulfanyl; carboxaldehyde, hydroxycarbonyl, C₁-C₈-alkyl; C₁-C₈-haloalkyl; C₁-C₈-cyanoalkyl; C₁-C₈-alkyloxy; C₁-C₈-halogenalkyloxy; tri(C₁-C₈-alkyl)silyl; tri(C₁-C₈-alkyl)silyl-C₁-C₈-alkyl; C₃-C₇-cycloalkyl; C₃-C₇-halogencycloalkyl; C₃-C₇-cycloalkenyl; C₃-C₇-halogencycloalkenyl; C₄-C₁₀-cycloalkylalkyl; C₄-C₁₀-halocycloaLkylaU<yl; C₆-C₁₂-cycloalkylcycloalkyl; C₁-Cs-akl-C₃-C₇-cycloalkyl; C₁-C₈-alkoxy-C₃-C₇-cycloalkyl; tri(C₁-C₈-alkyl)silyl-C₃-C₇-cycloalkyl; C₂-C₈-alkenyl; C₂-C₈-alkynyl; C₂-C₈-alkenyloxy; C₂-C₈-halogenalkenyloxy; C₃-C₈-alkynyloxy; C₃-C₈-halogenoalkynyloxy; C₁-C₈-alkylamino; C₁-C₈-halogenalkylamino; C₁-C₈-cyanoalkoxy; C₄-C₈-cycloalkylalkoxy; C₃-C₆-cycloalkoxy; C₁-C₈-alkylsulfanyl; C₁-C₈-halogenoalkylsulfanyl; C₁-C₈-alkylcarbonyl; C₁-C₈-halogenoalkylcarbonyl; arylcarbonyl; C₃-C₈-cycloalkylcarbonyl; C₃-C₈-halogenocycloalkylcarbonyl; C₁-C₈-alkylcarbamoyl; di-C₁-C₈-alkylcarbamoyl; N-C₁-C₈-alkyloxycarbamoyl; C₁-C₈-alkoxycarbamoyl; N-C₁-C₈-alkyl-C₁-C₈-alkoxycarbamoyl; C₁-C₈-alkoxycarbonyl; C₁-C₈-halogenoalkoxycarbonyl; C₃-C₈-cycloalkoxycarbonyl; C₂-C₈-alkoxyalkylcarbonyl; C₂-C₈-halogenoalkoxyalkylcarbonyl; C₃-C₁₀-cycloalkoxyalkylcarbonyl; C₁-C₈-alkylaminocarbonyl; di-C₁-C₈-alkylaminocarbonyl; C₃-C₈-cycloalkylaminocarbonyl; C₁-C₈-alkylcarbonyloxy; C₁-C₈-halogenoalkylcarbonyloxy; C₃-C₈-cycloalkylcarbonyloxy; C₁-C₈-alkylcarbonylamino; C₁-C₈-halogenoalkylcarbonylamino; C₁-C₈-alkylaminocarbonyloxy; di-C₁-C₈-alkylaminocarbonyloxy; C₁-C₈-alkyloxycarbonyloxy; C₁-C₈-alkylsulfinyl; C₁-C₈-halogenoalkylsulfinyl; C₁-C₈-alkylsulfonyl; C₁-C₈-halogenoalkylsulfonyl; C₁-C₈-alkylsulfonyloxy; C₁-C₈-halogenoalkylsulfonyloxy; C₁-C₈-alkylaminosulfamoyl; di-C₁-C₈-alkylaminosulfamoyl; (C₁-C₈-alkoxyimino)-C₁-C₈-alkyl; (C₃-C₇-cycloalkoxyimino)-C₁-C₈-alkyl; hydroxyimino-C₁-C₈-alkyl; (C₁-C₈-alkoxyimmo)-C₃-C₇-cycloalkyl; hydroxyimino-C₃-C₇-cycloalkyl; (C₁-C₈-alkylinimo)-oxy; (C₁-C₈-alkylimino)-oxy-C₁-C₈-alkyl; (C₃-C₇-cycloalkylimino)-oxy-C₁-C₈-alkyl; (C₁-C₆-alkylimino)-oxy-C₃-C₇-cycloalkyl; (C₁-C₈-alkenyloxyimino)-C₁-C₈-alkyl; (C₁-C₈-alkynyloximino)-C₁-C₈-alkyl; (benzyloxyimino)-C₁-C₈-alkyl; C₁-C₈-alkoxyalkyl; C₁-C₈-alkylthioalkyl; C₁-C₈-alkoxyalkoxyalkyl; C₁-C₈-halogenoaloxyakyl; benzyl; phenyl; 5-membered heteroaryl; 6-membered heteroaryl; benzyloxy; phenyloxy; benzylsulfanyl; benzylamino; phenylsulfanyl; or phenylamino;
- R²: represents H, C₁-C₈-alkyl, -Si(R^{3a})(R^{3b})(R^{3c}), -P(O)(OH)₂, -CH₂-O-P(O)(OH)₂, -C(O)-C₁-C₈-alkyl, -C(O)-C₃-C₇-cycloalkyl, -C(O)NH-C₁-C₈-alkyl; C(O)N-di-C₁-C₈-alkyl; -C(O)O-C₁-C₈-alkyl; wherein the -C(O)-C₁-C₈-alkyl, -C(O)-C₃-C₇-cycloalkyl, -C(O)NH-C₁-C₈-alkyl; -C(O)N-di-C₁-C₈-alkyl or C(O)O-C₁-C₈-alkyl may be non-substituted or substituted by one or more group(s) selected from halogen or C₁-C₈-alkoxy;
wherein
R^{3a}, R^{3b}, R^{3c} represent independently from each other a phenyl or C₁-C₈-alkyl;
- Q: represents a substituted 6-membered aromatic heterocycle of formula (Q-I) containing one nitrogen atom 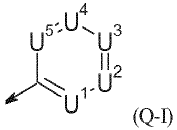
wherein
- U¹: represents CX¹ or N;
wherein X¹ represents hydrogen, halogen, C₁-C₈-alkyl; C₁-C₈-haloalkyl; C₁-C₈-alkoxy; or C₁-C₈-halogenalkyloxy;

- U²: represents CX² or N;
wherein X² represents hydrogen, halogen, C₁-C₈-alkyl; C₁-C₈-haloalkyl; C₁-C₈-alkoxy; or C₁-C₈-halogenalkyloxy;
- U³: represents CX³ or N;
wherein X³ represents hydrogen, halogen, C₁-C₈-alkyl; C₁-C₈-haloalkyl; C₁-C₈-alkoxy; or C₁-C₈-halogenalkyloxy;
- U⁴: represents CX⁴ or N;
wherein X⁴ represents hydrogen, halogen, C₁-C₈-alkyl; C₁-C₈-haloalkyl ; C₁-C₈-alkoxy; or C₁-C₈-halogenalkyloxy;
- U⁵: represents CX⁵ or N;
wherein X⁵ represents hydrogen, halogen, C₁-C₈-alkyl; C₁-C₈-haloalkyl; C₁-C₈-alkoxy; or C₁-C₈-halogenalkyloxy;
and wherein one of U¹, U², U³, U⁴ or U⁵ represents N and wherein at least one of X¹, X², X³, X⁴ or X⁵ is different from hydrogen;
and its salts or N-oxides.

The term substituted in substituted 6-membered aromatic heterocycle of formula (Q-I) refers to the fact, that the heterocycle needs to comprise at least one X¹, X², X³, X⁴ or X⁵ being different from hydrogen.

The salts or N-oxides of the triazole derivatives of formula (I) also have fungicidal properties.

The formula (I) provides a general definition of the triazole derivatives according to the invention. Preferred radical definitions for the formulae shown above and below are given below. These definitions apply to the end products of the formula (I) and likewise to all intermediates.
- R¹: preferably represents substituted or non-substituted phenyl.
- R¹: more preferably represents non-substituted phenyl or phenyl which is substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino; wherein the benzyl, phenyl, 5-membered heteroaryl, 6-membered heteroaryl, benzyloxy or phenyloxy may be optionally substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino.
- R¹: most preferably represents non-substituted phenyl or phenyl which is substituted by one or more group(s) selected from halogen, C₁-C₈-haloalkyl; C₁-C₈-halogenalkyloxy; C₂-C₈-alkynyl; phenyl; phenyloxy; wherein the phenyl or phenyloxy may be optionally substituted by one or more group(s) selected from halogen, C₁-C₈-haloalkyl.

In preferred embodiments of the present invention R¹ represents non-substituted phenyl or phenyl which is substituted by 1 or 2 substituents.

In more preferred embodiments of the present invention R¹ represents non-substituted phenyl or phenyl which is substituted by 1 substituent in 2-position or by 1 substituent in 4-position.

In other more preferred embodiments of the present invention R¹ represents phenyl which is substituted by 2 substituents in 2-,4-positions or by 2 substituents in 2-,5-positions.
- R²: preferably represents H, C₁-C₈-alkyl, halogen- or C₁-C₈-alkoxy-substituted or non-substituted -C(O)-C₁-C₈-alkyl.
- R²: more preferably represents H, C₁-C₄-alkyl, non-substituted -C(O)-C₁-C₄-alkyl.
- R²: most preferably represents H.

In such embodiments of the present invention wherein R² represents -Si(R^{3a})(R^{3b})(R^{3c})
- R^{3a}, R^{3b}, R^{3c}: preferably represent independent from each other methyl, ethyl or tert-butyl,
- R^{3a}, R^{3b}, R^{3c}: more preferably represents methyl.
- Q: preferably represents a substituted 6-membered aromatic heterocycle containing one nitrogen atom of formula (Q-I-1) to (Q-I-3)

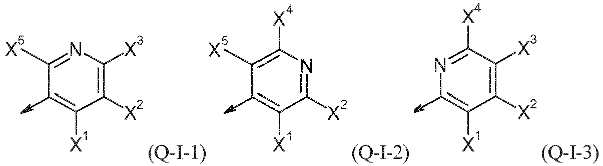

wherein X¹, X², X³, X⁴ or X⁵ have the same definition as given for formula (I) above. X¹, X², X³, X⁴ or X⁵ preferably represent hydrogen or halogen, wherein at least one of X¹, X², X³, X⁴ or X⁵, being present in the heterocycle, is different from hydrogen.
Q more preferably represents a substituted 3-pyridyl or 4-pyridyl of formula (Q-I-1) to (Q-I-2) 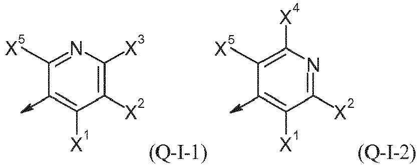
wherein X¹, X², X³, X⁴ or X⁵ have the same definition as given for formula (I) above. X¹, X², X³, X⁴ or X⁵ preferably represent hydrogen or halogen, wherein at least one of X¹, X², X³, X⁴ or X⁵, being present in the heterocycle, is different from hydrogen.
   - Q: most preferably represents a substituted 3-pyridyl of formula (Q-I-1a) or (Q-I-2a) to (Q-I-2d) 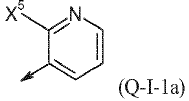 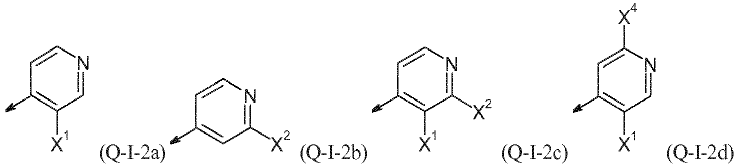 wherein X¹, X², X⁴ or X⁵ have the same definition as given for formula (I) above except for hydrogen. X¹, X², X⁴ or X⁵ preferably represent halogen.

In the definitions of the symbols given in the above formulae, collective terms were used which are generally representative of the following substituents:
The definition C₁-C₈-alkyl comprises the largest range defined here for an alkyl radical. Specifically, this definition comprises the meanings methyl, ethyl, n-, isopropyl, n-, iso-, sec-, tert-butyl, and also in each case all isomeric pentyls, hexyls, heptyls and octyls, such as methyl, ethyl, propyl, 1-methylethyl, butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, l-ethyl-3-methylpropyl, n-heptyl, 1-methylhexyl, 1-ethylpentyl, 2-ethylpentyl, 1-propylbutyl, octyl, 1-methylheptyl, 2-methylheptyl, 1-ethylhexyl, 2-ethylhexyl, 1-propylpentyl and 2-propylpentyl, in particular propyl, 1-methylethyl, butyl, 1-methylbutyl, 2-methylbutyl, 3-methylbutyl, 1,1-dimethylethyl, 1,2-dimethylbutyl, 1,3-dimethylbutyl, pentyl, 1-methylbutyl, 1-ethylpropyl, hexyl, 3-methylpentyl, heptyl, 1-methylhexyl, 1-ethyl-3-methylbutyl, 1-methylheptyl, 1,2-dimethylhexyl, 1,3-dimethyloctyl, 4-methyloctyl, 1,2,2,3-tetramethylbutyl, 1,3,3-trimethylbutyl, 1,2,3-trimethylbutyl, 1,3-dimethylpentyl, 1,3-dimethylhexyl, 5-methyl-3-hexyl, 2-methyl-4-heptyl and 1-methyl-2-cyclopropylethyl. A preferred range is C₁-C₄-alkyl, such as methyl, ethyl, n-, isopropyl, n-, iso-, sec-, tert-butyl. The definition C₁-C₃-alkyl comprises methyl, ethyl, n-, isopropyl.

The definition halogen comprises fluorine, chlorine, bromine and iodine.

Halogen-substituted alkyl - referred to as C₁-C₈-haloalkyl - represents, for example, C₁-C₈-alkyl as defined above substituted by one or more halogen substituents which can be the same or different. Preferably C₁-C₈-haloalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2- fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 1-fluoro-1-methylethyl, 2- fluoro-1,1-dimethylethyl, 2-fluoro-1-fluoromethyl- 1-methylethyl, 2-fluoro-1, 1-di(fluoromethyl)-ethyl, 3-chloro-1-methylbutyl, 2-chloro-1-methylbutyl, 1-chlorobutyl, 3,3-dichloro-1-methylbutyl, 3-chloro-1-methylbutyl, 1-methyl-3-trifluoromethylbutyl, 3-methyl-1-trifluoromethylbutyl.

Mono- or multiple fluorinated C₁-C₄-alkyl represents, for example, C₁-C₄-alkyl as defined above substituted by one or more fluorine substituent(s). Preferably mono- or multiple fluorinated C₁-C₄-alkyl represents fluoromethyl, difluoromethyl, trifluoromethyl, 1-fluoroethyl, 2-fluoroethyl, 2,2-difluoroethyl, 2,2,2-trifluoroethyl, pentafluoroethyl, 1-fluoro-1-methylethyl, 2-tluoro-1,1-dimethylethyl, 2-fluoro-1-fluoromethyl-1-methylethyl, 2-fluoro-1,1-di(fluoromethyl)-ethyl, 1-methyl-3-trifluoromethylbutyl, 3-methyl-1-trifluoromethylbutyl.

The definition C₂-C₈-alkenyl comprises the largest range defined here for an alkenyl radical. Specifically, this definition comprises the meanings ethenyl, n-, isopropenyl, n-, iso-, sec-, tert-butenyl, and also in each case all isomeric pentenyls, hexenyls, heptenyls, octenyls, 1-methyl-1-propenyl, 1-ethyl-1-butenyl, 2,4-dimethyl-1-pentenyl, 2,4-dimethyl-2-pentenyl. Halogen-substituted alkenyl - referred to as C₂-C₈-haloalkenyl - represents, for example, C₂-C₈-alkenyl as defined above substituted by one or more halogen substituents which can be the same or different. A preferred range is C₂-C₄-alkenyl, such as ethenyl, n-, isopropenyl, n-, iso-, sec- or tert-butenyl.

The definition C₂-C₈-alkynyl comprises the largest range defined here for an alkynyl radical. Specifically, this definition comprises the meanings ethynyl, n-, isopropynyl, n-, iso-, sec-, tert-butynyl, and also in each case all isomeric pentynyls, hexynyls, heptynyls, octynyls. Halogen-substituted alkynyl - referred to as C₂-C₈-haloalkynyl - represents, for example, C₂-C₈-alkynyl as defined above substituted by one or more halogen substituents which can be the same or different. A preferred range is C₂-C₄-alkynyl, such as ethynyl, n-, isopropynyl, n-, iso-, sec- or tert-butynyl

The definition C₃-C₇-cycloalkyl comprises monocyclic saturated hydrocarbyl groups having 3 to 7 carbon ring members, such as cyclopropyl, cyclobutyl, cyclopentyl, cyclohexyl and cycloheptyl.

The definition halogen-substituted cycloalkyl and halocycloalkyl comprises monocyclic saturated hydrocarbyl groups having 3 to 7 carbon ring members, such as 1-fluoro-cyclopropyl and 1-chloro-cyclopropyl.

The definition aryl comprises aromatic, mono-, bi- or tricyclic ring, for example phenyl, naphthyl, anthracenyl (anthryl), phenanthracenyl (phenanthryl).

The definition hetaryl or heteroaryl comprises unsaturated, benzoannulated or not benzoannulated heterocyclic 5- to 10-membered ring containing up to 4 heteroatoms selected from N, O and S. Preferably, the definition hetaryl or heteroaryl comprises unsaturated heterocyclic 5- to 7-membered rings containing up to 4 heteroatoms selected from N, O and S: for example 2-furyl, 3-furyl, 2-thienyl, 3-thienyl, 2-pyrrolyl, 3-pyrrolyl, 1-pyrrolyl, 3-pyrazolyl, 4-pyrazolyl, 5-pyrazolyl, 1-pyrazolyl, 1H-imidazol-2-yl, 1H-imidazol-4-yl, 1H-imidazol-5-yl, 1H-imidazol-1-yl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-3-yl, 1H-1,2,4-triazol-5-yl, 1H-1,2,4-triazol-1-yl, 4H-1,2,4-triazol-3-yl, 4H-1,2,4-triazol-4-yl, 1H-tetrazol-1-yl, 1H-tetrazol-5-yl, 2H-tetrazol-2-yl, 2H-tetrazol-5-yl, 1,2,4-oxadiazol-3-yl, 1,2,4-oxadiazol-5-yl, 1,2,4-thiadiazol-3-yl, 1,2,4-thiadiazol-5-yl, 1,3,4-oxadiazol-2-yl, 1,3,4-thiadiazol-2-yl, 1,2,3-oxadiazol-4-yl, 1,2,3-oxadiazol-5-yl, 1,2,3-thiadiazol-4-yl, 1,2,3-thiadiazol-5-yl, 1,2,5-oxadiazol-3-yl, 1,2,5-thiadiazol-3-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1,3,5-triazin-2-yl, 1,2,4-triazin-3-yl, 1,2,4-triazin-5-yl, 1,2,4-triazin-6-yl.

The definition 5-membered heteroaryl comprises a unsaturated heterocyclic 5-membered ring containing up to 4 heteroatoms selected from N, O and S: for example 2-furyl, 3-furyl, 2-thienyl, 3-thienyl, 2-pyrrolyl, 3-pyrrolyl, 1-pyrrolyl, 3-pyrazolyl, 4-pyrazolyl, 5-pyrazolyl, 1-pyrazolyl, 1H-imidazol-2-yl, 1H-imidazol-4-yl, 1H-imidazol-5-yl, 1H-imidazol-1-yl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-3-yl, 1H-1,2,4-triazol-5-yl, 1H-1,2,4-triazol-1-yl, 4H-1,2,4-triazol-3-yl, 4H-1,2,4-triazol-4-yl, 1H-tetrazol-1-yl, 1H-tetrazol-5-yl, 2H-tetrazol-2-yl, 2H-tetrazol-5-yl, 1,2,4-oxadiazol-3-yl, 1,2,4-oxadiazol-5-yl, 1,2,4-thiadiazol-3-yl, 1,2,4-thiadiazol-5-yl, 1,3,4-oxadiazol-2-yl, 1,3,4-thiadiazol-2-yl, 1,2,3-oxadiazol-4-yl, 1,2,3-oxadiazol-5-yl, 1,2,3-thiadiazol-4-yl, 1,2,3-thiadiazol-5-yl, 1,2,5-oxadiazol-3-yl, 1,2,5-thiadiazol-3-yl.

The definition 6-membered heteroaryl comprises a unsaturated heterocyclic 6-membered ring containing up to 4 heteroatoms selected from N, O and S: for example 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1,3,5-triazin-2-yl, 1,2,4-triazin-3-yl, 1,2,4-triazin-5-yl, 1,2,4-triazin-6-yl.

Optionally substituted radicals may be mono- or polysubstituted, where in the case of polysubstitution, the substituents may be identical or different.

Depending on the nature of the substituents, the compounds according to the invention can be present as mixtures of different possible isomeric forms, in particular of stereoisomers, such as, for example, E and Z, threo and erythro, and also optical isomers, and, if appropriate, also of tautomers. What is claimed are both the E and the Z isomers, and also the threo and erythro, and the optical isomers, any mixtures of these isomers, and the possible tautomeric forms.

Depending on the nature of the substituents, the compounds of the present invention can exist in one or more optical or chiral isomer forms depending on the number of asymmetric centres in the compound. The invention thus relates equally to all the optical isomers and to their racemic or scalemic mixtures (the term "scalemic" denotes a mixture of enantiomers in different proportions) and to the mixtures of all the possible stereoisomers, in all proportions. The diastereoisomers and/or the optical isomers can be separated according to the methods which are known per se by the man ordinary skilled in the art.

Depending on the nature of the substituents, the compounds of the present invention can also exist in one or more geometric isomer forms depending on the number of double bonds in the compound. The invention thus relates equally to all geometric isomers and to all possible mixtures, in all proportions. The geometric isomers can be separated according to general methods, which are known per se by the man ordinary skilled in the art.

Depending on the nature of the substituents, the compounds of the present invention can also exist in one or more geometric isomer forms depending on the relative position (syn/anti or cis/trans) of the substituents of ring B. The invention thus relates equally to all syn/anti (or cis/trans) isomers and to all possible syn/anti (or cis/trans) mixtures, in all proportions. The syn/anti (or cis/trans) isomers can be separated according to general methods, which are known per se by the man ordinary skilled in the art.

The compounds of formula (I) wherein Q is substituted by a hydroxy, a sulfanyl or an amino substituent may be found in its tautomeric form resulting from the shift of the proton of said hydroxy, sulfanyl or amino group. All tautomeric forms of such compounds of the present invention) wherein Q is substituted by a hydroxy, a sulfanyl or an amino substituent are also part of the present invention.

### Îllustration of the processes and intermediates

The present invention furthermore related to processes for preparing compounds of formula **(I).** The present invention furthermore relates to intermediates such as compounds of formula **(XII)** and the preparation thereof.

The compounds **(I)** can be obtained by various routes in analogy to prior art processes known (see e.g. EP-A 461 502, DE-A 40 27 608, DE-A 32 35 935, WO-A 2014/167008, WO-A 2014/167009 and references therein) and by synthesis routes shown schematically below and in the experimental part of this application. Unless indicated otherwise, the radicals Q, R¹ and R² have the meanings given above for the compounds of formula (I). These definitions apply not only to the end products of the formula (I) but likewise to all intermediates.

**Process A (Scheme 1):**

| | |
|---|---|
| 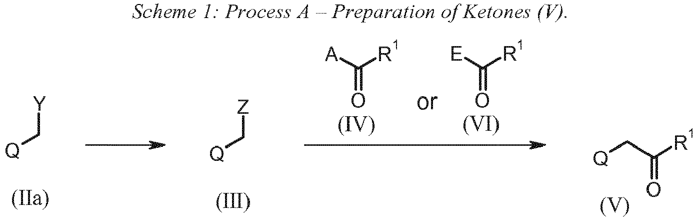 | |
| Y | = -H or -OH |
| Z | = halogen, -OSO₂-C₁-C₈-alkyl, -OSO₂-aryl, -OP(O)(O-C₁-C₈-alkyl)₂ or -OP(O)(O-aryl)₂, preferably Cl or Br |
| A | = halogen, preferably Cl |
| E | = -O-C₁-C₈-alkyl, preferably -O-methyl, -O-ethyl; -O-aryl; -S-C₁-C₈-alkyl; -S-aryl; -NHR^{a}; -NR^{a}R^{b}; R^{a}: is aryl, C₁-C₈-alkyl or C₃-C₇-cycloalkyl, R^{b}: is C₁-C₈-alkyl or C₁-C₈-alkyloxy, preferably -NMe₂, -NMeOMe; or heterocyclic leaving groups, such as imidazole, triazole and hydroxybenzotriazole. |

Compounds **(IIa)** and_{/}or **(III)** are either commercially available or producible by processes described in the literature (see, for example, "Comprehensive Heterocyclic Chemistry III", Pergamon Press, 2008; vol. 7, pages 101-169; 217-308 & vol. 7, pages 1-331 and references cited therein; , "Comprehensive Heterocyclic Chemistry II", Pergamon Press, 1996; vol. 5, pages 37-243 & vol. 6, pages 1-278 and references cited therein; "Comprehensive Heterocyclic Chemistry I", Pergamon Press, 1984; vol. 2, pages 395-510 & vol. 3, pages 1-197 and references cited therein; "Comprehensive Heterocyclic Chemistry III", Pergamon Press, 2008; vol. 3, pages 45-388 & vol. 4, pages 1-364 and references cited therein; , "Comprehensive Heterocyclic Chemistry II", Pergamon Press, 1996; vol. 2, pages 39-257 & vol. 3, pages 1-220 and references cited therein; "Comprehensive Heterocyclic Chemistry I", Pergamon Press, 1984; vol. 4, pages 155-376 & vol. 5, pages 167-498 and references cited therein).

The compounds **(IIa)** (Scheme 1) can be converted by means of methods described in the literature to the corresponding compounds **(III)** and subsequently to compounds **(V).** In a first process, for example, compounds **(IIa)** are halogenated.

In case Y stands for hydrogen, the compounds **(IIa)** can be halogenated e.g. with Bromo- or Chlorosuccinimide (see e.g. WO-A 2011/012622, WO-A 2008/003622, WO-A 2005/111003; Synthesis, 18, 2008, 2996 and references cited therein), preferably in the presence of a radical initiator such as Azobisisobutyronitrile or dibenzoyl peroxide and in the presence of an organic solvent, e.g. a chlorinated organic solvent such as tetrachloromethane. Alternatively, compounds **(IIa)** undergo side-chain halogenation in the presence of bromine or chlorine (see e.g. EP-A 557967) to obtain compounds **(III).** Optionally, a radical initiator such as Azobisisobutyronitrile or dibenzoyl peroxide can be used. Alternatively, compounds **(IIa)** are reacted with a base, e.g. methyl lithium, and subsequently with a halogen source such as Magnesiumbromide to obtain compounds **(III)** (see e.g. WO-A 2012/087784)

Compounds **(IIa)** where Y stands for -OH are reacted with halogenating agents, such as PBr₃, PC1₃ or thionyl chloride, to obtain compounds **(III)** (see e.g. WO-A 2009/153554, Bioorganic & Medicinal Chemistry Letters, 22, 2012, 901-906, WO-A 2010/132999 and references cited therein). Alternatively, compounds **(IIa)** can be reacted with sulfonyl halides, such as e.g. Mesylchloride or Tosylchloride, or with phosphonic acid halides, such as e.g. diphenylphosphoryl chloride, to obtain the respective sulfonates and phosphates (see e.g. J. Org. Chem. 1992,57, 5425-5431 and references cited therein)

The compounds **(III)** can subsequently be reacted with compounds **(IV)** or **(VI)** wherein A and E represent a replaceable group such as halide, -OR, NHR^{a} or NR^{a}R^{b}, preferably chloro, -O-methyl, -O-ethyl, -NMe₂ or-NMeOMe. To obtain compounds **(V),** compounds **(III)** are reacted in a first step with e.g. zinc, magnesium or isopropylmagnesium chloride, followed by a carbonyl compound **(IV)** or **(VI)** preferably under anhydrous conditions and optionally in the presence of a metal catalyst, such as palladium- or nickel-based catalysts. The metal catalyst can be used such as (Ph₃P)₂PdCl₂ (e.g. WO-A 2012/087784, EP-A 461 502), PEPPSI-IPr (Chem. Eur. J. 2006, 12, 4743 - 4748) or prepared in-situ by the mixing of a metals salt (e.g. Pd(OAc)₂) and a ligand (such as e.g. PPh₃, 2-dicyclohexylphosphino-2',6'-dimethoxybiphenyl (S-Phos)). The Insertion of the metal can be enhanced by the addition of ionic salts, such as LiBr, LiCl, LiI, CuI, Zn(OPiv)₂, MgCh, CuCN (see e.g. Dissertation Albrecht Metzer 2010 (University Munich); Angew. Chem. Int. Ed. 2011, 50, 9205 - 9209), or by activation of the metal using halogenated alkanes (1,2-dibromoethane) or halogenated alkylsilanes (TMSCl). Alternatively this sequence may be carried out in a one-pot fashion (see e.g. Beller et al., Chem. Asian J., 2011, 7(1) 40-44).

The reactions are preferably performed at temperatures between room temperature and refluxing temperature of the solvent.

As the solvent, all common solvents inert under the reaction conditions, such as for example ethers (such as e.g. tetrahydrofurane, diethyl ether) can be used and the reaction can be effected in mixtures of two or more of these solvents.

**Process B (Scheme 2):**

| | |
|---|---|
| 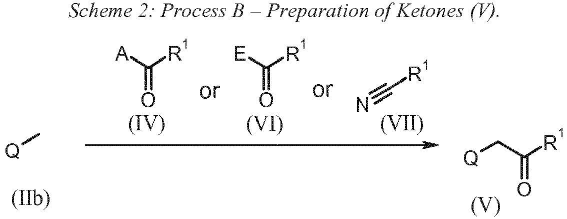 | |
| A = halogen, preferably Cl | |
| E = -O-C₁-C₈-alkyl, preferably -O-methyl, -O-ethyl; -O-aryl; -S-C₁-C₈-alkyl; -S-aryl; -NHR^{a}; -NR^{a}R^{b}; R^{a}: is aryl, C₁-C₈-alkyl or C₃-C₇-cycloalkyl, R^{b}: is C₁-C₈-alkyl or C₁-C₈-alkyloxy, preferably -NMe₂, -NMeOMe; or heterocyclic leaving groups, such as imidazole, triazole and hydroxybenzotriazole. | |

Compounds **(IIb)** are either commercially available or producible by processes described in the literature (see, for example, "Comprehensive Heterocyclic Chemistry III", Pergamon Press, 2008; vol. 7, pages 101-169; 217-308 & vol. 7, pages 1-331 and references cited therein; , "Comprehensive Heterocyclic Chemistry II", Pergamon Press, 1996; vol. 5, pages 37-243 & vol. 6, pages 1-278 and references cited therein; "Comprehensive Heterocyclic Chemistry I", Pergamon Press, 1984; vol. 2, pages 395-510 & vol. 3, pages 1-197 and references cited therein; "Comprehensive Heterocyclic Chemistry III", Pergamon Press, 2008; vol. 3, pages 45-388 & vol. 4, pages 1-364 and references cited therein; , "Comprehensive Heterocyclic Chemistry II", Pergamon Press, 1996; vol. 2, pages 39-257 & vol. 3, pages 1-220 and references cited therein; "Comprehensive Heterocyclic Chemistry I", Pergamon Press, 1984; vol. 4, pages 155-376 & vol. 5, pages 167-498 and references cited therein).

There are numerous literature methods for the preparation of ketones (see e.g. WO-A 2012/055942, WO-A 2012/100342, WO-A 2012/087784, WO-A 2012/087833, US-A 2012/0010190, Dalton Transaction, 2011, 2366-2374, Journal of the American Chemical Society, 1955, 3858-3860, Journal of the American Chemical Society, 1937, 1494-1497, WO-A 2012/085815, WO-A 2011/042389, WO-A 2003/026663, Heterocycles, 1998, 2103-2109, Bioorganic & Medicinal Chemistry Letters, 2010, 2634-2640).

In general, it is possible to prepare compounds of the formula **(V)** from corresponding compounds **(IIb)** and **(IV)** and/or from corresponding compounds **(IIb)** and **(VI)** with suitable groups A and E (see Scheme 2, process B). Compounds **(IIb)** are optionally reacted sequentially with a base, e.g. n-butyllithium, lithium-diisopropylamide, lithium bis(trimethylsilyl)amide, sodium bis(trimethylsilyl)amide, potassium bis(trimethylsilyl)amide sodium amide, potassium amide, potassium tert-butoxide, methyl lithium, TMP2Zn·2MgCl2·2LiCl (see e.g. Dissertation Albrecht Metzer 2010, University Munich), followed by compounds **(IV)** or **(VI),** preferably under anhydrous conditions. Optionally, the reaction of compounds **(IIb)** and compounds **(IV)** or **(VI)** is carried out in the presence of a base in a one-pot fashion. The possible groups for A and E are, for example, halide, -OR, NHR^{a} or NR^{a}R^{b}, preferably chloro, -O-methyl, -O-ethyl, -NMe₂ or -NMeOMe, etc., which can act as appropriate leaving groups to form the desired ketones **(V)** under suitable reaction conditions (Scheme 2).

In an alternative route compounds **(IIb)** are reacted with compounds **(VII)** in the presence of a base, e.g. phenyl lithium or methyl lithium, to obtain compounds **(V)** (see e.g. Journal of the American Chemical Society, 2011, 11194-11204; Journal of Medicinal Chemistry 1963, 205-207 and references cited therein).

**Process C (Scheme 3):**

| | |
|---|---|
| 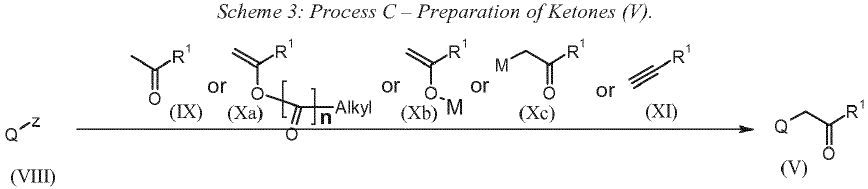 | |
| Z = halogen, preferably Cl or Br | |
| n = 0 or 1 | |
| M = Li, MgZ^{M}, ZnZ^{M}, Si(C₁-C₈-alkyl)₃ or Sn(C₁-C₈-alkyl)₃ | |
| Z^{M} = halogen or hydroxyl, preferably Cl or Br | |

One means of preparing compounds of the formula **(V)** from corresponding compounds **(VIII)** with the compounds **(IX)** or **(X)** or **(XI)** is shown in Scheme 3 (*Process C*). Compounds **(X)** include compounds **(Xa), (Xb)** and **(Xc)**

Compounds **(VIII)** are either commercially available or producible by processes described in the literature (see, for example, "Comprehensive Heterocyclic Chemistry III", Pergamon Press, 2008; vol. 7, pages 101-169; 217-308 & vol. 7, pages 1-331 and references cited therein; , "Comprehensive Heterocyclic Chemistry II", Pergamon Press, 1996; vol. 5, pages 37-243 & vol. 6, pages 1-278 and references cited therein; "Comprehensive Heterocyclic Chemistry I", Pergamon Press, 1984; vol. 2, pages 395-510 & vol. 3, pages 1-197 and references cited therein; "Comprehensive Heterocyclic Chemistry III", Pergamon Press, 2008; vol. 3, pages 45-388 & vol. 4, pages 1-364 and references cited therein; , "Comprehensive Heterocyclic Chemistry II", Pergamon Press, 1996; vol. 2, pages 39-257 & vol. 3, pages 1-220 and references cited therein; "Comprehensive Heterocyclic Chemistry I", Pergamon Press, 1984; vol. 4, pages 155-376 & vol. 5, pages 167-498 and references cited therein).

Compounds **(IX), (X)** and **(XI)** are either commercially available or producible by processes described in the literature (see, for example, WO-A 2010/029066; Chemische Berichte, 1986, 2995-3026 and references cited therein).

A compound having the general formula **(V)** can be synthesized analogously to methods described in the literature (see, for example Organic letters, 2009, 1773-1775; European Journal of Organic Chemistry, 2011, 1570-1574), by a coupling reaction of a compound with the corresponding general formula **(VIII)** with a substrate of the general formula **(IX), (X)** or **(XI)** where Z is halogen, preferably chlorine or bromine.

Compounds **(VIII)** are reacted with compounds of the general structure **(IX)** or **(X)** to obtain compounds **(V)** analogously to methods described in the literature (e.g. Organic letters, 2009, 1773-1775, European Journal of Organic Chemistry, 2011, 1570-1574, Chemical & Pharmaceutical Bulletin, 1970, 1457-1464, Chemical & Pharmaceutical Bulletin, 1980, 337-342, WO-A 2005/044785). Those reactions can be optionally carried out in the presence of a catalyst and a base.

As catalysts for the reaction various metal based catalysts can be used which are either used directly or being in situ prepared from a metal precursor (e.g. Pd₂dba₃, Pd(OAc)₂) and a ligand (e.g. phosphine based ligands like Xanthphos, 2-(dicyclohexylphosphino)-2'-methylbiphenyl, 2-Diphenylphosphino-2'-(N,N-dimethylamino)biphenyl, tri-t-butylphosphine, Tri-o-tolylphosphine) (see e.g. WO-A 2008/147544, WO-A 2005/027837).

As bases, various organic and inorganic bases can be used such as potassium phosphate, base, e.g. sodium amide, sodium hydride or sodium tert-butoxide. Alternatively, silicon-containing bases can be used (e.g.

NaHMDS, KHMDS, LiHMDS).

Compounds **(VIII)** are reacted with compounds of the general structure **(XI)** to obtain compounds **(V)** analogously to methods described in the literature (e.g. WO-A 2012/080476). The intermediary alkynes can be further converted to the corresponding ketones **(V)** by methods known in the literature (see e.g. Chemistry - A European Journal, 2011, 1261-1267; European Journal of Organic Chemistry, 2008, 5277-5282; Journal of the Chemical Society, 1944, 612-615 and references cited therein).

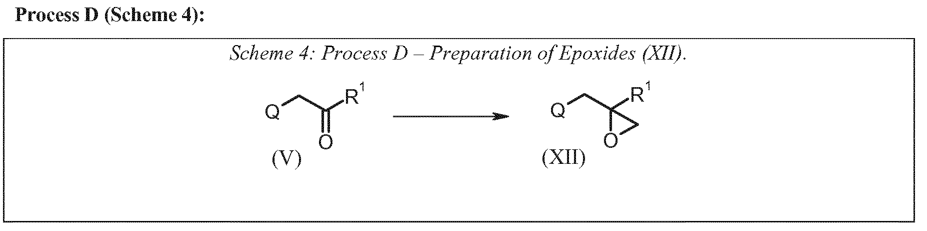

The compounds **(V)** (Scheme 4) can be converted by means of methods described in the literature to the corresponding compounds **(XII)** (see e.g. EP-A 461 502, DE-A 33 15 681, EP-A 291 797). Intermediates **(V)** are preferably reacted with trimethylsulfoxonium- or trimethylsulfonium-salts, preferably trimethylsulfoxonium halides, trimethylsulfonium halides, trimethylsulfoxonium methylsulfates or trimethylsulfonium methylsulfates, preferably in the presence of a base such as sodium hydroxide.

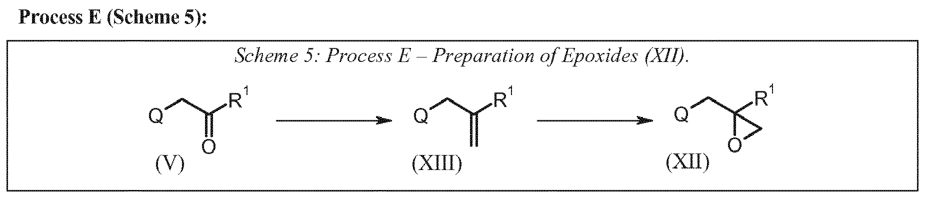

Alternatively, compounds **(V)** can be first converted to the corresponding olefins **(XIII),** followed by an epoxidation to obtain epoxides **(XII)** (see e.g. EP-A 291 797).

**Process F (Scheme 6):**

| | |
|---|---|
| 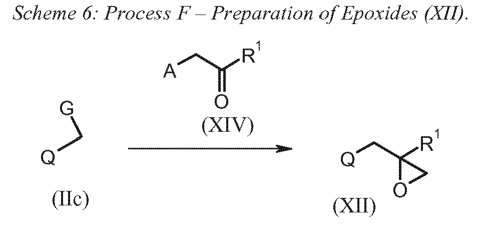 | |
| G = halogen or hydrogen | |
| A = halogen, O-SO₂-C₁-C₈-alkyl or O-SO₂-aryl, preferably Cl or Br | |

Alternatively, a compound having the general formula **(XII)** can be synthesized analogously to methods described in the literature by a coupling reaction of a compound having the corresponding general formula **(IIc)** with a substrate of the general formula **(XIV)** (see e.g. DE-A 40 27 608, WO-A 93/02086, WO-A 93/12121, Journal of Organic Chemistry, 2001, 2149-2153 and references cited therein).

Compounds **(IIc)** are either commercially available or producible by processes described in the literature (see, for example, "Comprehensive Heterocyclic Chemistry III", Pergamon Press, 2008; vol. 7, pages 101-169; 217-308 & vol. 7, pages 1-331 and references cited therein; , "Comprehensive Heterocyclic Chemistry II", Pergamon Press, 1996; vol. 5, pages 37-243 & vol. 6, pages 1-278 and references cited therein; "Comprehensive Heterocyclic Chemistry I", Pergamon Press, 1984; vol. 2, pages 395-510 & vol. 3, pages 1-197 and references cited therein; "Comprehensive Heterocyclic Chemistry III", Pergamon Press, 2008; vol. 3, pages 45-388 & vol. 4, pages 1-364 and references cited therein; , "Comprehensive Heterocyclic Chemistry II", Pergamon Press, 1996; vol. 2, pages 39-257 & vol. 3, pages 1-220 and references cited therein; "Comprehensive Heterocyclic Chemistry I", Pergamon Press, 1984; vol. 4, pages 155-376 & vol. 5, pages 167-498 and references cited therein).

If G represents halogen, preferably chloride or bromide, compounds **(IIc)** are first transformed into Grignard reagents by the reaction with magnesium or with halogen/metal exchange reagents such as isopropylmagnesium halides and subsequently reacted with ketones **(XIV)** preferably under anhydrous conditions to obtain compounds of the general formula **(XV)** (see e.g. DE4027608). Alternatively, if G represents halogen, the halides **(IIc)** can be converted to the corresponding zinc reagents and subsequently reacted with ketones **(XIV)** (e.g. ChemComm, 2008, 5824-5826; Journal of Organic Chemistry, 2004, 908-914 and references cited therein).

In an alternative route, if G represents hydrogen, compounds **(IIc)** are reacted with compounds **(XIV)** preferably in the presence of a base. When G represents hydrogen, compounds **(IIc)** are optionally reacted with a base upfront, e.g. n-butyllithium, lithium-diisopropylamide, lithium bis(trimethylsilyl)amide, sodium bis(trimethylsilyl)amide, potassium bis(trimethylsilyl)amide sodium amide, potassium amide, potassium tert-butoxide, methyl lithium, TMP₂Zn·2MgCl₂·2LiCl (see e.g. Dissertation Albrecht Metzer 2010, University Munich), followed by compounds of the general structure **(XIV)** preferably under anhydrous conditions. The possible groups for A are, for example, halides which can act as appropriate leaving groups to form the desired compounds **(XII)** under suitable reaction conditions.

**Process G (Scheme 7):**

| | |
|---|---|
| 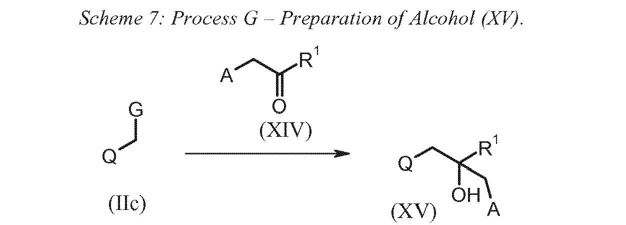 | |
| A = halogen, O-SO₂-C₁-C₈-alkyl or O-SO₂-aryl, preferably Cl or Br | |

A compound having the general formula **(XV)** can be synthesized analogously to methods described in the literature by a coupling reaction of a compound having the corresponding general formula **(IIc)** with a substrate of the general formula **(XIV)** (see e.g. DE-A 40 27 608, WO-A 93/02086, WO-A 93/12121, Journal of Organic Chemistry, 2001, 2149-2153).

If G represents halogen, preferably chloride or bromide, compounds **(IIc)** are first transformed into Grignard reagents by the reaction with magnesium or with halogen/metal exchange reagents, such as isopropylmagnesium halides, and subsequently reacted with ketones **(XIV)** preferably under anhydrous conditions to obtain compounds of the general formula **(XV)** (see e.g. DE4027608). Alternatively, if G represents halogen, the halides **(IIc)** can be converted to the corresponding zinc reagents and subsequently reacted with ketones **(XIV)** (e.g. ChemComm, 2008, 5824-5826; Journal of Organic Chemistry, 2004, 908-914 and references cited therein).

In an alternative route, if G represents hydrogen, compounds **(IIc)** are reacted with compounds **(XIV)** preferably in the presence of a base. When G represents hydrogen, compounds **(IIc)** are optionally reacted with a base upfront, e.g. n-butyllithium, lithium-di-isopropylamide, lithium bis(trimethylsilyl)amide, methyl lithium, followed by compounds of the general structure **(XIV)** preferably under anhydrous conditions. The possible groups for A are, for example, halides which can act as appropriate leaving groups to form the desired compounds **(XV)** under suitable reaction conditions.

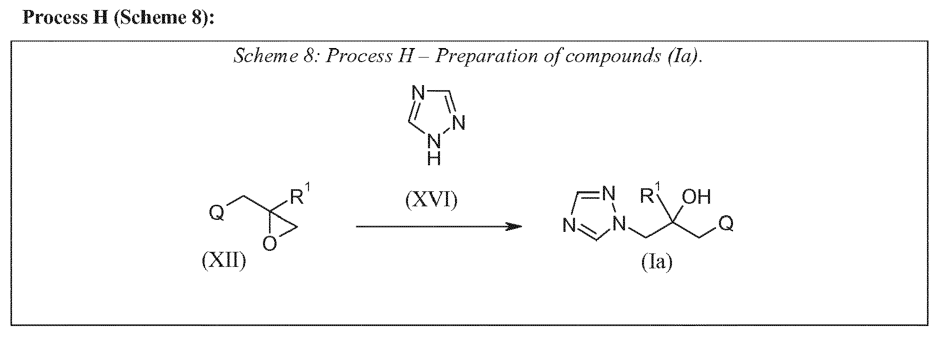

The compounds **(XII)** obtained according to Process D, E or F can be converted by means of methods described in the literature to the corresponding compounds **(Ia)** (see e.g. DE-A 40 27 608, EP-A 461 502, DE-A 33 15 681, EP-A 291 797, WO9529901, EP0291797). The starting materials **(XII)** can be reacted with 1*H-*1,2,4-triazole **(XVI)** preferably in the presence of a base, such as potassium phosphate, potassium carbonate and/or potassium *tert*-butoxide, and preferably in the presence of an organic solvent, such as DMF, to obtain compounds **(Ia).**

**Process I (Scheme 9):**

| | |
|---|---|
| 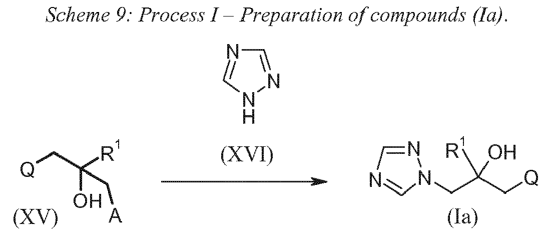 | |
| A = halogen, O-SO₂-C₁-C₈-alkyl or O-SO₂-aryl, preferably Cl or Br | |

The compounds **(XV)** obtained according to Process G can be converted by means of methods described in the literature to the corresponding compounds **(Ia)** (see e.g. DE-A 40 27 608). The starting materials **(XV)** can be reacted with 1*H*-1,2,4-triazole **(XVI)** preferably in the presence of a base, such as potassium carbonate and/or potassium *tert*-butoxide, and preferably in the presence of an organic solvent, such as dimethyl formamide, to obtain compounds **(Ia).**

**Process J (Scheme 10):**

| | |
|---|---|
| 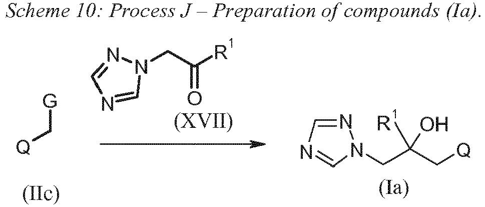 | |
| G = halogen or hydrogen | |

Many triazole ketones of the formula **(XVII)** are known or can be prepared by literature known methods (e.g. DE-A 24 31 407, DE-A 26 10 022, DE-A 26 38 470, DE-A 42 04 816, EP-A 0 470 463, US 4 486 218, DE-A 31 44 670). The compounds of the formula **(XVII)** which have not hitherto been described in the literature can be prepared by customary methods. For instance, they are obtained by reacting the corresponding halo-ketones with 1*H*-1,2,4-triazole in the presence of a base.

In a process according to Scheme 10, for example, ketones **(XVII)** are reacted with derivatives **(IIc),** wherein G represents halogen or hydrogen. If G represents halogen, compounds **(IIc)** are first transformed into Grignard reagents by the reaction with magnesium or with transmetallation reagents such as isopropylmagenesium halides and subsequently reacted with ketone **(XVII),** preferably under anhydrous conditions to obtain compounds **(Ia).**

In case G represents hydrogen, compounds **(IIc)** can be reacted with an organolithium reagent such as methyllithium or n-butyllithium preferably under anhydrous conditions to obtain a lithiated species. Optionally, a base such as lithiumdiisopropy*l*amide or lithium bis(trimethylsilyl)amide, can be used. The obtained intermediates are subsequently reacted with ketones **(XVII),** preferably under anhydrous conditions to obtain compounds of the general formula **(Ia).**

**Process K (Scheme 11):**

| | |
|---|---|
| 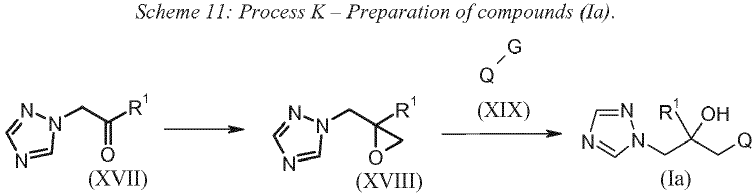 | |
| G = halogen or hydrogen | |

The compounds **(XVII)** (Scheme 11) can be converted by means of methods described in the literature to the corresponding compounds **(XVIII)** (see e.g. DE-A 31 11 238, DE-A 33 07 217). Compounds of the general formula **(XVII)** are preferably reacted with trimethylsulfoxonium halides, trimethylsulfonium halides, trimethylsulfoxonium methylsulfates or trimethylsulfonium methylsulfates, preferably in the presence of a base, such as sodium hydroxide, to obtain compounds **(XVIII).**

Compounds **(XIX)** are either commercially available or producible by processes described in the literature (see, for example, "Comprehensive Heterocyclic Chemistry III", Pergamon Press, 2008; vol. 7, pages 101-169; 217-308 & vol. 7, pages 1-331 and references cited therein; , "Comprehensive Heterocyclic Chemistry II", Pergamon Press, 1996; vol. 5, pages 37-243 & vol. 6, pages 1-278 and references cited therein; "Comprehensive Heterocyclic Chemistry I", Pergamon Press, 1984; vol. 2, pages 395-510 & vol. 3, pages 1-197 and references cited therein; "Comprehensive Heterocyclic Chemistry III", Pergamon Press, 2008; vol. 3, pages 45-388 & vol. 4, pages 1-364 and references cited therein; , "Comprehensive Heterocyclic Chemistry II", Pergamon Press, 1996; vol. 2, pages 39-257 & vol. 3, pages 1-220 and references cited therein; "Comprehensive Heterocyclic Chemistry I", Pergamon Press, 1984; vol. 4, pages 155-376 & vol. 5, pages 167-498 and references cited therein).

Subsequently, compounds **(Ia)** can be obtained by the reaction of **(XVIII)** with **(XIX).** If G represents halogen, preferably chloride or bromide, compounds **(XIX)** are first transformed into Grignard reagents by the reaction with magnesium or with transmetallation reagents such as isopropylmagnesium halides and subsequently reacted with epoxides **(XVIII)** preferably under anhydrous conditions.

In an alternative route, if G represents halogen or hydrogen, compounds **(XIX)** are reacted with compounds **(XVIII)** preferably in the presence of a base. Compounds **(XIX)** wherein G represents hydrogen or halogen, are optionally reacted with a base upfront, e.g. n-butyllithium, lithium-di-isopropylamide, lithium bis(trimethylsilyl)amide, methyl lithium, followed by compounds of the general structure **(XVIII)** preferably under anhydrous conditions to form the desired compounds **(Ia).**

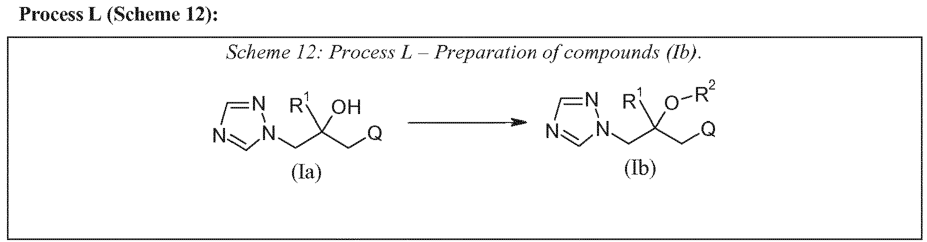

The compounds **(Ia)** obtained according to Processes H, I, J or K can be converted by means of methods described in the literature to the corresponding compounds **(Ib)** (see e.g. DE-A 3202604, JP-A 02101067, EP-A 225 739, CN-A 101824002, FR-A 2802772; WO-A 2012/175119, Bioorganic & Medicinal Chemistry Letters, 7207-7213, 2012; Journal of the American Chemical Society, 19358-19361, 2012, Journal of Organic Chemistry, 9458-9472, 2012; Organic Letters, 554-557, 2013; Journal of the American Chemical Society, 15556, 2012). Compounds of the general structure **(Ia)** are preferably reacted with alkylhalides, dialkylsulfates, anhydrides, acid chlorides, phosphorylchloride, alkylisocyanate, carbamoyl chlorides, carbono chloridates or imidocarbonates preferably in the presence of a base to obtain compounds **(Ib).**

### General

The processes A to L according to the invention for preparing compounds of the formula **(I)** are optionally performed using one or more reaction auxiliaries.

Useful reaction auxiliaries are, as appropriate, inorganic or organic bases or acid acceptors. These preferably include alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, n-butyllithium, sec-butyllithium, tert-butyllithium, lithium diisopropylamide, lithium bis(trimethylsilyl)amide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) or 1,8-diazabicyclo[5.4.0]-undec-7-ene (DBU).

Useful reaction auxiliaries are, as appropriate, inorganic or organic acids. These preferably include inorganic acids, for example hydrogen fluoride, hydrogen chloride, hydrogen bromide and hydrogen iodide, sulphuric acid, phosphoric acid and nitric acid, and acidic salts such as NaHSO₄ and KHSO₄, or organic acids, for example, formic acid, carbonic acid and alkanoic acids such as acetic acid, trifluoroacetic acid, trichloroacetic acid and propionic acid, and also glycolic acid, thiocyanic acid, lactic acid, succinic acid, citric acid, benzoic acid, cinnamic acid, oxalic acid, saturated or mono- or diunsaturated C₆-C₂₀ fatty acids, alkylsulphuric monoesters, alkylsulphonic acids (sulphonic acids having straight-chain or branched alkyl radicals having 1 to 20 carbon atoms), arylsulphonic acids or aryldisulphonic acids (aromatic radicals, such as phenyl and naphthyl, which bear one or two sulphonic acid groups), alkylphosphonic acids (phosphonic acids having straight-chain or branched alkyl radicals having 1 to 20 carbon atoms), arylphosphonic acids or aryldiphosphonic acids (aromatic radicals, such as phenyl and naphthyl, which bear one or two phosphonic acid radicals), where the alkyl and aryl radicals may bear further substituents, for example p-toluenesulphonic acid, salicylic acid, p-aminosalicylic acid, 2-phenoxybenzoic acid, 2-acetoxybenzoic acid, etc.

The processes A to L according to the invention are optionally performed using one or more diluents. Useful diluents are virtually all inert organic solvents. Unless otherwise indicated for the above described processes A to L, these preferably include aliphatic and aromatic, optionally halogenated hydrocarbons, such as pentane, hexane, heptane, cyclohexane, petroleum ether, benzine, ligroin, benzene, toluene, xylene, methylene chloride, ethylene chloride, chloroform, carbon tetrachloride, chlorobenzene and o-dichlorobenzene, ethers such as diethyl ether, dibutyl ether and methyl tert-butyl ether, glycol dimethyl ether and diglycol dimethyl ether, tetrahydrofuran and dioxane, ketones such as acetone, methyl ethyl ketone, methyl isopropyl ketone and methyl isobutyl ketone, esters, such as methyl acetate and ethyl acetate, nitriles, for example acetonitrile and propionitrile, amides, for example dimethylformamide, dimethylacetamide and N-methylpyrrolidone, and also dimethyl sulphoxide, tetramethylenesulphone and hexamethylphosphoramide and DMPU.

In the processes according to the invention, the reaction temperatures can be varied within a relatively wide range. In general, the temperatures employed are between -78°C and 250°C, preferably temperatures between -78°C and 150°C.

The reaction time varies as a function of the scale of the reaction and of the reaction temperature, but is generally between a few minutes and 48 hours.

The processes according to the invention are generally performed under standard pressure. However, it is also possible to work under elevated or reduced pressure.

For performance of the processes according to the invention, the starting materials required in each case are generally used in approximately equimolar amounts. However, it is also possible to use one of the components used in each case in a relatively large excess.

After a reaction has ended, the compounds are optionally separated from the reaction mixture by one of the customary separation techniques. If necessary, the compounds are purified by recrystallization or chromatography.

If appropriate, in the processes A to L according to the invention also salts and/or N-oxides of the starting compounds can be used.

The invention further relates to novel intermediates of the compounds of formula (I), which form part of the invention.

Novel intermediates according to the present invention are novel epoxides of formula (XII) 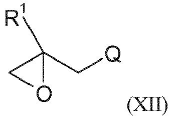 wherein
Q represents a substituted 6-membered aromatic heterocycle of formula (Q-I) containing one nitrogen atom 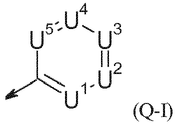 wherein
- U¹: represents CX¹ or N;
wherein X¹ represents hydrogen, halogen, C₁-C₈-alkyl; C₁-C₈-haloalkyl; C₁-C₈-alkoxy; or C₁-C₈-halogenalkyloxy;
- U²: represents CX² or N;
wherein X² represents hydrogen, halogen, C₁-C₈-alkyl; C₁-C₈-haloalkyl; C₁-C₈-alkoxy; or C₁-C₈-halogenalkyloxy;
- U³: represents CX³ or N;
wherein X³ represents hydrogen, halogen, C₁-C₈-alkyl; C₁-C₈-haloalkyl; C₁-C₈-alkoxy; or C₁-C₈-halogenalkyloxy;

- U⁴: represents CX⁴ or N;
wherein X⁴ represents hydrogen, halogen, C₁-C₈-alkyl; C₁-C₈-haloalkyl; C₁-C₈-alkoxy; or C₁-C₈-halogenalkyloxy;
- U⁵: represents CX⁵ or N;
wherein X⁵ represents hydrogen, halogen, C₁-C₈-alkyl; C₁-C₈-haloalkyl; C₁-C₈-alkoxy; or C₁-C₈-halogenalkyloxy;
and wherein one of U¹, U², U³, U⁴ or U⁵ represents N and wherein at least one of X¹, X², X³, X⁴ or X⁵ is different from hydrogen;
and
- R¹: represents phenyl or naphthyl; wherein the phenyl or naphthyl may be non-substituted or substituted by one or more group(s) selected from halogen; hydroxyl; cyano; amino; sulfanyl; pentafluoro-λ⁶-sulfanyl; carboxaldehyde, hydroxycarbonyl, C₁-C₈-alkyl; C₁-C₈-haloalkyl; C₁-C₈-cyanoalkyl; C₁-C₈-alkyloxy; C₁-C₈-halogenalkyloxy; tri(C₁-C₈-alkyl)silyl; tri(C₁-C₈-arkyl)silyl-C₁-C₈-alkyl; C₃-C₇-cycloalkyl; C₃-C₇-halogencycloalkyl; C₃-C₇-cycloalkenyl; C₃-C₇-halogencycloalkenyl; C₄-C₁₀-cycloalkylalkyl; C₄-C₁₀-halocycloalkylalkyl; C₆-C₁₂-cycloalkylcycloalkyl; C₁-C₈-alkyl-C₃-C₇-cycloalkyl; C₁-C₈-alkoxy-C₃-C₇-cycloalkyl; tri(C₁-C₈-alkyl)silyl-C₃-C₇-cycloalkyl; C₂-C₈-alkenyl; C₂-C₈-alkynyl; C₂-C₈-alkenyloxy; C₂-C₈-halogenalkenyloxy; C₃-C₈-alkynyloxy; C₃-C₈-halogenoalkynyloxy; C₁-C₈-alkylamino; C₁-C₈-halogenalkylamino; C₁-C₈-cyanoalkoxy; C₄-C₈-cycloalkylalkoxy; C₃-C₆-cycloalkoxy; C₁-C₈-alkylsulfanyl; C₁-C₈-halogenoalkylsulfanyl; C₁-C₈-alkylcarbonyl; C₁-C₈-halogenoalkylcarbonyl; arylcarbonyl; C₃-C₈-cycloalkylcarbonyl; C₃-C₈-halogenocycloalkylcarbonyl; C₁-C₈-alkylcarbamoyl; di-C₁-C₈-alkylcarbamoyl; N-C₁-C₈-alkyloxycarbamoyl; C₁-C₈-alkoxycarbamoyl; N-C₁-C₈-alkyl-C₁-C₈-alkoxycarbamoyl; C₁-C₈-alkoxycarbonyl; C₁-C₈-halogenoalkoxycarbonyl; C₃-C₈-cycloalkoxycarbonyl; C₂-C₈-alkoxyalkylcarbonyl; C₂-C₈-halogenoalkoxylcarbonyl; C₃-C₁₀-cycloalkoxyalkylcarbonyl; C₁-C₈-alkylaminocarbonyl; di-C₁-C₈-alkylaminocarbonyl; C₃-C₈-cycloalkylaminocarbonyl; C₁-C₈-alkylcarbonyloxy; C₁-C₈-halogenoalkylcarbonyloxy; C₃-C₈-cycloalkylcarbonyloxy; C₁-C₈-alkylcarbonylamino; C₁-C₈-halogenoalkylcarbonylamino; C₁-C₈-alkylaminocarbonyloxy; di-C₁-C₈-alkylaminocarbonyloxy; C₁-C₈-alkyloxycarbonyloxy; C₁-C₈-alkylsulfinyl; C₁-C₈-halogenoalkylsulfinyl; C₁-C₈-alkylsulfonyl; C₁-C₈-halogenoalkylsulfonyl; C₁-C₈-alkylsulfonyloxy; C₁-C₈-halogenoalkysulfonyloxy; C₁-C₈-alkylaminosulfamoyl; di-C₁-C₈-alkylaminosulfamoyl; (C₁-C₈-alkoxyimino)-C₁-C₈-alkyl; (C₃-C₇-cycloalkoxyimino)-C₁-C₈-alkyl; hydroxyimino-C₁-C₈-alkyl; (C₁-C₈-alkoxyimino)-C₃-C₇-cycloalkyl; hydroxyimino-C₃-C₇-cycloalkyl; (C₁-C₈-alkylimino)-oxy; (C₁-C₈-alkylimino)-oxy-C₁-C₈-alkyl; (C₃-C₇-cycloalkylimino)-oxy-C₁-C₈-alkyl; (C₁-C₆-alkylimino)-oxy-C₃-C₇-cycloalkyl; (C₁-C₈-alkenyloxyimino)-C₁-C₈-alkyl; (C₁-C₈-alkynyloxyimino)-C₁-C₈-alkyl; (benzyloxyimino)-C₁-C₈-alkyl; C₁-C₈-alkoxyalkyl; C₁-C₈-alkylthioalkyl; C₁-C₈-alkoxyalkoxyalkyl; C₁-C₈-halogenoalkoxyalkyl; benzyl; phenyl; 5-membered heteroaryl; 6-membered heteroaryl; benzyloxy; phenyloxy; benzylsulfanyl; benzylamino; phenylsulfanyl; or phenylamino;
and its salts or N-oxides.

Preferred radical definitions for Q and R¹ have already been given above for the compounds of formula (I). Such preferred radical definitions shall also apply for the epoxides of formula (XII).

The compounds of the formula (I) according to the invention can be converted into physiologically acceptable salts, e.g. as acid addition salts or metal salt complexes.

Depending on the nature of the substituents defined above, the compounds of the formula (I) have acidic or basic properties and can form salts, if appropriate also inner salts, or adducts with inorganic or organic acids or with bases or with metal ions. If the compounds of the formula (I) carry amino, alkylamino or other groups which induce basic properties, these compounds can be reacted with acids to give salts, or they are directly obtained as salts in the synthesis. If the compounds of the formula (I) carries hydroxyl, carboxyl or other groups which induce acidic properties, these compounds can be reacted with bases to give salts. Suitable bases are, for example, hydroxides, carbonates, bicarbonates of the alkali metals and alkaline earth metals, in particular those of sodium, potassium, magnesium and calcium, furthermore ammonia, primary, secondary and tertiary amines having (C₁-C₄)-alkyl groups, mono-, di- and trialkanolamines of (C₁-C₄)-alkanols, choline and also chlorocholine.

The salts obtainable in this manner also have fungicidal properties.

Examples of inorganic acids are hydrohalic acids, such as hydrogen fluoride, hydrogen chloride, hydrogen bromide and hydrogen iodide, sulphuric acid, phosphoric acid and nitric acid, and acidic salts, such as NaHSO₄ and KHSO₄. Suitable organic acids are, for example, formic acid, carbonic acid and alkanoic acids, such as acetic acid, trifluoroacetic acid, trichloroacetic acid and propionic acid, and also glycolic acid, thiocyanic acid, lactic acid, succinic acid, citric acid, benzoic acid, cinnamic acid, maleic acid, fumaric acid, tartaric acid, sorbic acid oxalic acid, alkylsulphonic acids (sulphonic acids having straight-chain or branched alkyl radicals of 1 to 20 carbon atoms), arylsulphonic acids or aryldisulphonic acids (aromatic radicals, such as phenyl and naphthyl, which carry one or two sulphonic acid groups), alkylphosphonic acids (phosphonic acids having straight-chain or branched alkyl radicals of 1 to 20 carbon atoms), arylphosphonic acids or aryldiphosphonic acids (aromatic radicals, such as phenyl and naphthyl, which carry one or two phosphonic acid radicals), where the alkyl and aryl radicals may carry further substituents, for example p-toluenesulphonic acid, 1,5-naphthalenedisulphonic acid, salicylic acid, p-aminosalicylic acid, 2-phenoxybenzoic acid, 2-acetoxybenzoic acid, etc.

Suitable metal ions are in particular the ions of the elements of the second main group, in particular calcium and magnesium, of the third and fourth main group, in particular aluminium, tin and lead, and also of the first to eighth transition group, in particular chromium, manganese, iron, cobalt, nickel, copper, zinc and others. Particular preference is given to the metal ions of the elements of the fourth period. Here, the metals can be present in various valencies that they can assume.

The acid addition salts of the compounds of the formula (I) can be obtained in a simple manner by customary methods for forming salts, for example by dissolving a compound of the formula (I) in a suitable inert solvent and adding the acid, for example hydrochloric acid, and be isolated in a known manner, for example by filtration, and, if required, be purified by washing with an inert organic solvent.

Suitable anions of the salts are those which are preferably derived from the following acids: hydrohalic acids, such as, for example, hydrochloric acid and hydrobromic acid, furthermore phosphoric acid, nitric acid and sulphuric acid.

The metal salt complexes of compounds of the formula (I) can be obtained in a simple manner by customary processes, for example by dissolving the metal salt in alcohol, for example ethanol, and adding the solution to the compound of the formula (I). Metal salt complexes can be isolated in a known manner, for example by filtration, and, if required, be purified by recrystallization.

Salts of the intermediates can also be prepared according to the processes mentioned above for the salts of compounds of formula (I).

N-oxides of compounds of the formula (I) or intermediates thereof can be obtained in a simple manner by customary processes, for example by N-oxidation with hydrogen peroxide (H₂O₂), peracids, for example peroxy sulfuric acid or peroxy carboxylic acids, such as meta-chloroperoxybenzoic acid or peroxymonosulfuric acid (Caro's acid).

### Methods and uses

The invention also describes to a method for controlling unwanted microorganisms, characterized in that the compounds of the formula (I) are applied to the microorganisms and/or in their habitat.

The invention further relates to seed which has been treated with at least one compound of the formula (I). The invention finally provides a method for protecting seed against unwanted microorganisms by using seed treated with at least one compound of the formula (I).

The compounds of the formula (I) have potent microbicidal activity and can be used for control of unwanted microorganisms, such as fungi and bacteria, in crop protection and in the protection of materials.

The compounds of the formula (I) have very good fungicidal properties and can be used in crop protection, for example for control of Plasmodiophoromycetes, Oomycetes, Chytridiomycetes, Zygomycetes, Ascomycetes, Basidiomycetes and Deuteromycetes.

Bactericides can be used in crop protection, for example, for control of Pseudomonadaceae, Rhizobiaceae, Enterobacteriaceae, Corynebacteriaceae and Streptomycetaceae.

The compounds of the formula (I) can be used for curative or protective control of phytopathogenic fungi. The invention therefore also relates to curative and protective methods for controlling phytopathogenic fungi by the use of the inventive active ingredients or compositions, which are applied to the seed, the plant or plant parts, the fruit or the soil in which the plants grow.

### Plants

All plants and plant parts can be treated in accordance with the invention. Plants are understood here to mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the transgenic plants and including the plant cultivars which are protectable and non-protectable by plant breeders' rights. Plant parts are understood to mean all parts and organs of plants above and below the ground, such as shoot, leaf, flower and root, examples of which include leaves, needles, stalks, stems, flowers, fruit bodies, fruits and seeds, and also roots, tubers and rhizomes. The plant parts also include harvested material and vegetative and generative propagation material, for example cuttings, tubers, rhizomes, slips and seeds.

Plants which can be treated in accordance with the invention include the following: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

### Pathogens

Non-limiting examples of pathogens of fungal diseases which can be treated in accordance with the invention include:
diseases caused by powdery mildew pathogens, for example Blumeria species, for example Blumeria graminis; Podosphaera species, for example Podosphaera leucotricha; Sphaerotheca species, for example Sphaerotheca fuliginea; Uncinula species, for example Uncinula necator;
diseases caused by rust disease pathogens, for example Gymnosporangium species, for example Gymnosporangium sabinae; Hemileia species, for example Hemileia vastatrix; Phakopsora species, for example Phakopsora pachyrhizi or Phakopsora meibomiae; Puccinia species, for example Puccinia recondita, Puccinia graminis oder Puccinia striiformis; Uromyces species, for example Uromyces appendiculatus;
diseases caused by pathogens from the group of the Oomycetes, for example Albugo species, for example Albugo candida; Bremia species, for example Bremia lactucae; Peronospora species, for example Peronospora pisi or P. brassicae; Phytophthora species, for example Phytophthora infestans; Plasmopara species, for example Plasmopara viticola; Pseudoperonospora species, for example Pseudoperonospora humuli or Pseudoperonospora cubensis; Pythium species, for example Pythium ultimum;
leaf blotch diseases and leaf wilt diseases caused, for example, by Alternaria species, for example Alternaria solani; Cercospora species, for example Cercospora beticola; Cladiosporium species, for example Cladiosporium cucumerinum; Cochliobolus species, for example Cochliobolus sativus (conidial form: Drechslera, syn: Helminthosporium) or Cochliobolus miyabeanus; Colletotrichum species, for example Colletotrichum lindemuthanium; Cycloconium species, for example Cycloconium oleaginum; Diaporthe species, for example Diaporthe citri; Elsinoe species, for example Elsinoe fawcettii; Gloeosporium species, for example Gloeosporium laeticolor; Glomerella species, for example Glomerella cingulata; Guignardia species, for example Guignardia bidwelli; Leptosphaeria species, for example Leptosphaeria maculans; Magnaporthe species, for example Magnaporthe grisea; Microdochium species, for example Microdochium nivale; Mycosphaerella species, for example Mycosphaerella graminicola, Mycosphaerella arachidicola or Mycosphaerella fijiensis; Phaeosphaeria species, for example Phaeosphaeria nodorum; Pyrenophora species, for example Pyrenophora teres or Pyrenophora tritici repentis; Ramularia species, for example Ramularia collo-cygni or Ramularia areola; Rhynchosporium species, for example Rhynchosporium secalis; Septoria species, for example Septoria apii or Septoria lycopersici; Stagonospora species, for example Stagonospora nodorum; Typhula species, for example Typhula incarnata; Venturia species, for example Venturia inaequalis;
root and stem diseases caused, for example, by Corticium species, for example Corticium graminearum; Fusarium species, for example Fusarium oxysporum; Gaeumannomyces species, for example Gaeumannomyces graminis; Plasmodiophora species, for example Plasmodiophora brassicae; Rhizoctonia species, for example Rhizoctonia solani; Sarocladium species, for example Sarocladium oryzae; Sclerotium species, for example Sclerotium oryzae; Tapesia species, for example Tapesia acuformis; Thielaviopsis species, for example Thielaviopsis basicola;
ear and panicle diseases (including corn cobs) caused, for example, by Alternaria species, for example Alternaria spp.; Aspergillus species, for example Aspergillus flavus; Cladosporium species, for example Cladosporium cladosporioides; Claviceps species, for example Claviceps purpurea; Fusarium species, for example Fusarium culmorum; Gibberella species, for example Gibberella zeae; Monographella species, for example Monographella nivalis; Stagnospora species, for example Stagnospora nodorum;
diseases caused by smut fungi, for example Sphacelotheca species, for example Sphacelotheca reiliana; Tilletia species, for example Tilletia caries or Tilletia controversa; Urocystis species, for example Urocystis occulta; Ustilago species, for example Ustilago nuda;
fruit rot caused, for example, by Aspergillus species, for example Aspergillus flavus; Botrytis species, for example Botrytis cinerea; Penicillium species, for example Penicillium expansum or Penicillium purpurogenum; Rhizopus species, for example Rhizopus stolonifer; Sclerotinia species, for example Sclerotinia sclerotiorum; Verticilium species, for example Verticilium alboatrum;
seed- and soil-borne rot and wilt diseases, and also diseases of seedlings, caused, for example, by Alternaria species, for example Alternaria brassicicola; Aphanomyces species, for example Aphanomyces euteiches; Ascochyta species, for example Ascochyta lentis; Aspergillus species, for example Aspergillus flavus; Cladosporium species, for example Cladosporium herbarum; Cochliobolus species, for example Cochliobolus sativus (conidial form: Drechslera, Bipolaris Syn: Helminthosporium); Colletotrichum species, for example Colletotrichum coccodes; Fusarium species, for example Fusarium culmorum; Gibberella species, for example Gibberella zeae; Macrophomina species, for example Macrophomina phaseolina; Microdochium species, for example Microdochium nivale; Monographella species, for example Monographella nivalis; Penicillium species, for example Penicillium expansum; Phoma species, for example Phoma lingam; Phomopsis species, for example Phomopsis sojae; Phytophthora species, for example Phytophthora cactorum; Pyrenophora species, for example Pyrenophora graminea; Pyricularia species, for example Pyricularia oryzae; Pythium species, for example Pythium ultimum; Rhizoctonia species, for example Rhizoctonia solani; Rhizopus species, for example Rhizopus oryzae; Sclerotium species, for example Sclerotium rolfsii; Septoria species, for example Septoria nodorum; Typhula species, for example Typhula incarnata; Verticillium species, for example Verticillium dahliae;
cancers, galls and witches' broom caused, for example, by Nectria species, for example Nectria galligena;
wilt diseases caused, for example, by Monilinia species, for example Monilinia laxa;
deformations of leaves, flowers and fruits caused, for example, by Exobasidium species, for example Exobasidium vexans; Taphrina species, for example Taphrina deformans;
degenerative diseases in woody plants, caused, for example, by Esca species, for example Phaeomoniella chlamydospora, Phaeoacremonium aleophilum or Fomitiporia mediterranea; Ganoderma species, for example Ganoderma boninense;
diseases of flowers and seeds caused, for example, by Botrytis species, for example Botrytis cinerea;
diseases of plant tubers caused, for example, by Rhizoctonia species, for example Rhizoctonia solani; Helminthosporium species, for example Helminthosporium solani;
diseases caused by bacterial pathogens, for example Xanthomonas species, for example Xanthomonas campestris pv. oryzae; Pseudomonas species, for example Pseudomonas syringae pv. lachrymans; Erwinia species, for example Erwinia amylovora.

Preference is given to controlling the following diseases of soya beans:
Fungal diseases on leaves, stems, pods and seeds caused, for example, by Alternaria leaf spot (Alternaria spec. atrans tenuissima), Anthracnose (Colletotrichum gloeosporoides dematium var. truncatum), brown spot (Septoria glycines), cercospora leaf spot and blight (Cercospora kikuchii), choanephora leaf blight (Choanephora infundibulifera trispora (Syn.)), dactuliophora leaf spot (Dactuliophora glycines), downy mildew (Peronospora manshurica), drechslera blight (Drechslera glycini), frogeye leaf spot (Cercospora sojina), leptosphaerulina leaf spot (Leptosphaerulina trifolii), phyllostica leaf spot (Phyllosticta sojaecola), pod and stem blight (Phomopsis sojae), powdery mildew (Microsphaera diffusa), pyrenochaeta leaf spot (Pyrenochaeta glycines), rhizoctonia aerial, foliage, and web blight (Rhizoctonia solani), rust (Phakopsora pachyrhizi, Phakopsora meibomiae), scab (Sphaceloma glycines), stemphylium leaf blight (Stemphylium botryosum), target spot (Corynespora cassiicola).

Fungal diseases on roots and the stem base caused, for example, by black root rot (Calonectria crotalariae), charcoal rot (Macrophomina phaseolina), fusarium blight or wilt, root rot, and pod and collar rot (Fusarium oxysporum, Fusarium orthoceras, Fusarium semitectum, Fusarium equiseti), mycoleptodiscus root rot (Mycoleptodiscus terrestris), neocosmospora (Neocosmospora vasinfecta), pod and stem blight (Diaporthe phaseolorum), stem canker (Diaporthe phaseolorum var. caulivora), phytophthora rot (Phytophthora megasperma), brown stem rot (Phialophora gregata), pythium rot (Pythium aphanidermatum, Pythium irregulare, Pythium debaryanum, Pythium myriotylum, Pythium ultimum), rhizoctonia root rot, stem decay, and damping-off (Rhizoctonia solani), sclerotinia stem decay (Sclerotinia sclerotiorum), sclerotinia southern blight (Sclerotinia rolfsii), thielaviopsis root rot (Thielaviopsis basicola).

### Plant Growth Regulation

In some cases, the compounds of the formula (I) can, at particular concentrations or application rates, also be used as growth regulators or agents to improve plant properties, or as microbicides, for example as fungicides, antimycotics, bactericides, viricides (including compositions against viroids) or as compositions against MLO (Mycoplasma-like organisms) and RLO (Rickettsia-like organisms).

The compounds of the formula (I) intervene in physiological processes of plants and can therefore also be used as plant growth regulators. Plant growth regulators may exert various effects on plants. The effect of the substances depends essentially on the time of application in relation to the developmental stage of the plant, and also on the amounts of active ingredient applied to the plants or their environment and on the type of application. In each case, growth regulators should have a particular desired effect on the crop plants. Growth regulating effects, comprise earlier germination, better emergence, more developed root system and/or improved root growth, increased ability of tillering, more productive tillers, earlier flowering, increased plant height and/or biomass, shorting of stems, improvements in shoot growth, number of kernels/ear, number of ears/m², number of stolons and/or number of flowers, enhanced harvest index, bigger leaves, less dead basal leaves, improved phyllotaxy, earlier maturation / earlier fruit finish, homogenous riping, increased duration of grain filling, better fruit finish, bigger fruit/vegetable size, sprouting resistance and reduced lodging.

Increased or improved yield is referring to total biomass per hectare, yield per hectare, kernel/fruit weight, seed size and/or hectolitre weight as well as to improved product quality, comprising:
improved processability relating to size distribution (kernel, fruit, etc.), homogenous riping, grain moisture, better milling, better vinification, better brewing, increased juice yield, harvestability, digestibility, sedimentation value, falling number, pod stability, storage stability, improved fiber length/strength/uniformity, increase of milk and/or meet quality of silage fed animals, adaption to cooking and frying;
further comprising improved marketability relating to improved fruit/grain quality, size distribution (kernel, fruit, etc.), increased storage / shelf-life, firmness / softness, taste (aroma, texture, etc.), grade (size, shape, number of berries, etc.), number of berries/fruits per bunch, crispness, freshness, coverage with wax, frequency of physiological disorders, colour, etc.;
further comprising increased desired ingredients such as e.g. protein content, fatty acids, oil content, oil quality, aminoacid composition, sugar content, acid content (pH), sugar/acid ratio (Brix), polyphenols, starch content, nutritional quality, gluten content/index, energy content, taste, etc.;
and further comprising decreased undesired ingredients such as e.g. less mycotoxines, less aflatoxines, geosmin level, phenolic aromas, lacchase, polyphenol oxidases and peroxidases, nitrate content etc.

Plant growth-regulating compounds can be used, for example, to slow down the vegetative growth of the plants. Such growth depression is of economic interest, for example, in the case of grasses, since it is thus possible to reduce the frequency of grass cutting in ornamental gardens, parks and sport facilities, on roadsides, at airports or in fruit crops. Also of significance is the inhibition of the growth of herbaceous and woody plants on roadsides and in the vicinity of pipelines or overhead cables, or quite generally in areas where vigorous plant growth is unwanted.

Also important is the use of growth regulators for inhibition of the longitudinal growth of cereal. This reduces or completely eliminates the risk of lodging of the plants prior to harvest. In addition, growth regulators in the case of cereals can strengthen the culm, which also counteracts lodging. The employment of growth regulators for shortening and strengthening culms allows the deployment of higher fertilizer volumes to increase the yield, without any risk of lodging of the cereal crop.

In many crop plants, vegetative growth depression allows denser planting, and it is thus possible to achieve higher yields based on the soil surface. Another advantage of the smaller plants obtained in this way is that the crop is easier to cultivate and harvest.

Reduction of the vegetative plant growth may also lead to increased or improved yields because the nutrients and assimilates are of more benefit to flower and fruit formation than to the vegetative parts of the plants.

Alternatively, growth regulators can also be used to promote vegetative growth. This is of great benefit when harvesting the vegetative plant parts. However, promoting vegetative growth may also promote generative growth in that more assimilates are formed, resulting in more or larger fruits.

Furthermore, beneficial effects on growth or yield can be achieved through improved nutrient use efficiency, especially nitrogen (N)-use efficiency, phosphours (P)-use efficiency, water use efficiency, improved transpiration, respiration and/or CO2 assimilation rate, better nodulation, improved Ca-metabolism etc.

Likewise, growth regulators can be used to alter the composition of the plants, which in turn may result in an improvement in quality of the harvested products. Under the influence of growth regulators, parthenocarpic fruits may be formed. In addition, it is possible to influence the sex of the flowers. It is also possible to produce sterile pollen, which is of great importance in the breeding and production of hybrid seed.

Use of growth regulators can control the branching of the plants. On the one hand, by breaking apical dominance, it is possible to promote the development of side shoots, which may be highly desirable particularly in the cultivation of ornamental plants, also in combination with an inhibition of growth. On the other hand, however, it is also possible to inhibit the growth of the side shoots. This effect is of particular interest, for example, in the cultivation of tobacco or in the cultivation of tomatoes.

Under the influence of growth regulators, the amount of leaves on the plants can be controlled such that defoliation of the plants is achieved at a desired time. Such defoliation plays a major role in the mechanical harvesting of cotton, but is also of interest for facilitating harvesting in other crops, for example in viticulture. Defoliation of the plants can also be undertaken to lower the transpiration of the plants before they are transplanted.

Furthermore, growth regulators can modulate plant senescence, which may result in prolonged green leaf area duration, a longer grain filling phase, improved yield quality, etc.

Growth regulators can likewise be used to regulate fruit dehiscence. On the one hand, it is possible to prevent premature fruit dehiscence. On the other hand, it is also possible to promote fruit dehiscence or even flower abortion to achieve a desired mass ("thinning"). In addition it is possible to use growth regulators at the time of harvest to reduce the forces required to detach the fruits, in order to allow mechanical harvesting or to facilitate manual harvesting.

Growth regulators can also be used to achieve faster or else delayed ripening of the harvested material before or after harvest. This is particularly advantageous as it allows optimal adjustment to the requirements of the market. Moreover, growth regulators in some cases can improve the fruit colour. In addition, growth regulators can also be used to synchronize maturation within a certain period of time. This establishes the prerequisites for complete mechanical or manual harvesting in a single operation, for example in the case of tobacco, tomatoes or coffee.

By using growth regulators, it is additionally possible to influence the resting of seed or buds of the plants, such that plants such as pineapple or ornamental plants in nurseries, for example, germinate, sprout or flower at a time when they are normally not inclined to do so. In areas where there is a risk of frost, it may be desirable to delay budding or germination of seeds with the aid of growth regulators, in order to avoid damage resulting from late frosts.

Finally, growth regulators can induce resistance of the plants to frost, drought or high salinity of the soil. This allows the cultivation of plants in regions which are normally unsuitable for this purpose.

### Resistance Induction /Plant Health and other effects

The compounds of the formula (I) also exhibit a potent strengthening effect in plants. Accordingly, they can be used for mobilizing the defences of the plant against attack by undesirable microorganisms.

Plant-strengthening (resistance-inducing) substances in the present context are substances capable of stimulating the defence system of plants in such a way that the treated plants, when subsequently inoculated with undesirable microorganisms, develop a high degree of resistance to these microorganisms.

Further, in context with the present invention plant physiology effects comprise the following:
Abiotic stress tolerance, comprising tolerance to high or low temperatures, drought tolerance and recovery after drought stress, water use efficiency (correlating to reduced water consumption), flood tolerance, ozone stress and UV tolerance, tolerance towards chemicals like heavy metals, salts, pesticides etc.

Biotic stress tolerance, comprising increased fungal resistance and increased resistance against nematodes, viruses and bacteria. In context with the present invention, biotic stress tolerance preferably comprises increased fungal resistance and increased resistance against nematodes.

Increased plant vigor, comprising plant health / plant quality and seed vigor, reduced stand failure, improved appearance, increased recovery after periods of stress, improved pigmentation (e.g. chlorophyll content, stay-green effects, etc.) and improved photosynthetic efficiency.

### Mycotoxins

In addition, the compounds of the formula (I) can reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides* etc., and also by *Aspergillus* spec., such as *A. flavus, A. parasiticus, A. nomius, A. ochraceus, A. clavatus, A. terreus, A. versicolor, Penicillium* spec., such as *P. verrucosum, P. viridicatum, P. citrinum, P. expansum, P. claviforme, P. roqueforti, Claviceps* spec., such as *C. purpurea, C. fusiformis, C. paspali, C. africana, Stachybotrys* spec. and others.

### Material Protection

The compounds of the formula (I) can also be used in the protection of materials, for protection of industrial materials against attack and destruction by phytopathogenic fungi.

In addition, the compounds of the formula (I) can be used as antifouling compositions, alone or in combinations with other active ingredients.

Industrial materials in the present context are understood to mean inanimate materials which have been prepared for use in industry. For example, industrial materials which are to be protected by inventive compositions from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The compounds of the formula (I) may prevent adverse effects, such as rotting, decay, discoloration, decoloration or formation of mould.

In the case of treatment of wood the compounds of the formula (I) may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting a composition according to the invention; this includes for example direct application, spraying, dipping, injection or any other suitable means.

In addition, the compounds of the formula (I) can be used to protect objects which come into contact with saltwater or brackish water, especially hulls, screens, nets, buildings, moorings and signalling systems, from fouling.

The compounds of the formula (I) can also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, can be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The inventive compositions may prevent adverse effects, such as rotting, decay, discoloration, decoloration or formation of mould.

Microorganisms capable of degrading or altering the industrial materials include, for example, bacteria, fungi, yeasts, algae and slime organisms. The compounds of the formula (I) preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis*; *Aspergillus,* such as *Aspergillus niger; Chaetomium,* such as *Chaetomium globosum*; *Coniophora,* such as *Coniophora puetana*; *Lentinus,* such as *Lentinus tigrinus; Penicillium,* such as *Penicillium glaucum*; *Polyporus,* such as *Polyporus versicolor*; *Aureobasidium,* such as *Aureobasidium pullulans; Sclerophoma,* such as *Sclerophoma pityophila*; *Trichoderma,* such as *Trichoderma viride; Ophiostoma spp., Ceratocystis spp., Humicola spp., Petriella spp., Trichurus spp., Coriolus spp., Gloeophyllum spp., Pleurotus spp., Poria spp., Serpula spp.* and *Tyromyces spp., Cladosporium spp., Paecilomyces spp. Mucor spp., Escherichia,* such as *Escherichia coli*; *Pseudomonas,* such as *Pseudomonas aeruginosa*; *Staphylococcus,* such as *Staphylococcus aureus, Candida spp.* and *Saccharomyces spp.,* such as *Saccharomyces cerevisae.*

### Formulations

The present invention further relates to a composition for controlling unwanted microorganisms, comprising at least one of the compounds of the formula (I). These are preferably fungicidal compositions which comprise agriculturally suitable auxiliaries, solvents, carriers, surfactants or extenders.

According to the invention, a carrier is a natural or synthetic, organic or inorganic substance with which the active ingredients are mixed or combined for better applicability, in particular for application to plants or plant parts or seed. The carrier, which may be solid or liquid, is generally inert and should be suitable for use in agriculture.

Useful solid carriers include: for example ammonium salts and natural rock flours, such as kaolins, clays, talc, chalk, quartz, attapulgite, montmorillonite or diatomaceous earth, and synthetic rock flours, such as finely divided silica, alumina and silicates; useful solid carriers for granules include: for example, crushed and fractionated natural rocks such as calcite, marble, pumice, sepiolite and dolomite, and also synthetic granules of inorganic and organic flours, and granules of organic material such as paper, sawdust, coconut shells, maize cobs and tobacco stalks; useful emulsifiers and/or foam-formers include: for example nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP POE esters, alkylaryl and/or POP POE ethers, fat and/or POP POE adducts, POE- and/or POP-polyol derivatives, POE- and/or POP-sorbitan or -sugar adducts, alkyl or aryl sulphates, alkyl- or arylsulphonates and alkyl or aryl phosphates or the corresponding PO-ether adducts. Additionally suitable are oligo- or polymers, for example those derived from vinylic monomers, from acrylic acid, from EO and/or PO alone or in combination with, for example, (poly)alcohols or (poly)amines. It is also possible to use lignin and its sulphonic acid derivatives, unmodified and modified celluloses, aromatic and/or aliphatic sulphonic acids and also their adducts with formaldehyde.

The active ingredients can be converted to the customary formulations, such as solutions, emulsions, wettable powders, water- and oil-based suspensions, powders, dusts, pastes, soluble powders, soluble granules, granules for broadcasting, suspoemulsion concentrates, natural products impregnated with active ingredient, synthetic substances impregnated with active ingredient, fertilizers and also microencapsulations in polymeric substances.

The active ingredients can be applied as such, in the form of their formulations or the use forms prepared therefrom, such as ready-to-use solutions, emulsions, water- or oil-based suspensions, powders, wettable powders, pastes, soluble powders, dusts, soluble granules, granules for broadcasting, suspoemulsion concentrates, natural products impregnated with active ingredient, synthetic substances impregnated with active ingredient, fertilizers and also microencapsulations in polymeric substances. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

The formulations mentioned can be prepared in a manner known per se, for example by mixing the active ingredients with at least one customary extender, solvent or diluent, emulsifier, dispersant and/or binder or fixing agent, wetting agent, a water repellent, if appropriate siccatives and UV stabilizers and if appropriate dyes and pigments, antifoams, preservatives, secondary thickeners, stickers, gibberellins and also other processing auxiliaries.

The present invention includes not only formulations which are already ready for use and can be deployed with a suitable apparatus to the plant or the seed, but also commercial concentrates which have to be diluted with water prior to use.

The compounds of the formula (I) may be present as such or in their (commercial) formulations and in the use forms prepared from these formulations as a mixture with other (known) active ingredients, such as insecticides, attractants, sterilants, bactericides, acaricides, nematicides, fungicides, growth regulators, herbicides, fertilizers, safeners and/or semiochemicals.

The auxiliaries used may be those substances which are suitable for imparting particular properties to the composition itself or and/or to preparations derived therefrom (for example spray liquors, seed dressings), such as certain technical properties and/or also particular biological properties. Typical auxiliaries include: extenders, solvents and carriers.

Suitable extenders are, for example, water, polar and nonpolar organic chemical liquids, for example from the classes of the aromatic and nonaromatic hydrocarbons (such as paraffins, alkylbenzenes, alkylnaphthalenes, chlorobenzenes), the alcohols and polyols (which may optionally also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

Liquefied gaseous extenders or carriers are understood to mean liquids which are gaseous at standard temperature and under standard pressure, for example aerosol propellants such as halohydrocarbons, or else butane, propane, nitrogen and carbon dioxide.

In the formulations it is possible to use tackifiers such as carboxymethylcellulose, natural and synthetic polymers in the form of powders, granules or latices, such as gum arabic, polyvinyl alcohol and polyvinyl acetate, or else natural phospholipids such as cephalins and lecithins and synthetic phospholipids. Further additives may be mineral and vegetable oils.

If the extender used is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example petroleum fractions, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, or else water.

Compositions comprising compounds of the formula (I) may additionally comprise further components, for example surfactants. Suitable surfactants are emulsifiers and/or foam formers, dispersants or wetting agents having ionic or nonionic properties, or mixtures of these surfactants. Examples thereof are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. The presence of a surfactant is necessary if one of the active ingredients and/or one of the inert carriers is insoluble in water and when application is effected in water. The proportion of surfactants is between 5 and 40 per cent by weight of the inventive composition.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Further additives may be perfumes, mineral or vegetable, optionally modified oils, waxes and nutrients (including trace nutrients), such as salts of iron, manganese, boron, copper, cobalt, molybdenum and zinc.

Additional components may be stabilizers, such as cold stabilizers, preservatives, antioxidants, light stabilizers, or other agents which improve chemical and/or physical stability.

If appropriate, other additional components may also be present, for example protective colloids, binders, adhesives, thickeners, thixotropic substances, penetrants, stabilizers, sequestering agents, complex formers. In general, the active ingredients can be combined with any solid or liquid additive commonly used for formulation purposes.

The formulations contain generally between 0.05 and 99% by weight, 0.01 and 98% by weight, preferably between 0.1 and 95% by weight, more preferably between 0.5 and 90% of active ingredient, most preferably between 10 and 70 per cent by weight.

The formulations described above can be used for controlling unwanted microorganisms, in which the compositions comprising compounds of the formula (I) are applied to the microorganisms and/or in their habitat.

### Mixtures

Compounds of the formula (I) can be used as such or in formulations thereof and can be mixed with known fungicides, bactericides, acaricides, nematicides or insecticides, in order thus to broaden, for example, the activity spectrum or to prevent development of resistance.

Useful mixing partners include, for example, known fungicides, insecticides, acaricides, nematicides or else bactericides (see also Pesticide Manual, 14th ed.).

A mixture with other known active ingredients, such as herbicides, or with fertilizers and growth regulators, safeners and/or semiochemicals, is also possible.

### Seed Treatment

The invention furthermore includes a method for treating seed.

A further aspect of the present invention relates in particular to seeds (dormant, primed, pregerminated or even with emerged roots and leaves) treated with at least one of the compounds of the formula (I). The inventive seeds are used in methods for protection of seeds and emerged plants from the seeds from phytopathogenic harmful fungi. In these methods, seed treated with at least one inventive active ingredient is used.

The compounds of the formula (I) are also suitable for the treatment of seeds and young seedlings. A large part of the damage to crop plants caused by harmful organisms is triggered by the infection of the seeds before sowing or after germination of the plant. This phase is particularly critical since the roots and shoots of the growing plant are particularly sensitive, and even small damage may result in the death of the plant. Accordingly, there is great interest in protecting the seed and the germinating plant by using appropriate compositions.

It is also desirable to optimize the amount of the active ingredient used so as to provide the best possible protection for the seeds, the germinating plants and emerged seedlings from attack by phytopathogenic fungi, but without damaging the plants themselves by the active ingredient used. In particular, methods for the treatment of seed should also take into consideration the intrinsic phenotypes of transgenic plants in order to achieve optimum protection of the seed and the germinating plant with a minimum of crop protection compositions being employed.

The present invention therefore also relates to a method for protecting seeds, germinating plants and emerged seedlings against attack by animal pests and/or phytopathogenic harmful microorganisms by treating the seeds with an inventive composition. The invention also relates to the use of the compositions according to the invention for treating seeds for protecting the seeds, the germinating plants and emerged seedlings against animal pests and/or phytopathogenic microorganisms. The invention further relates to seeds which has been treated with an inventive composition for protection from animal pests and/or phytopathogenic microorganisms.

One of the advantages of the present invention is that the treatment of the seeds with these compositions not only protects the seed itself, but also the resulting plants after emergence, from animal pests and/or phytopathogenic harmful microorganisms. In this way, the immediate treatment of the crop at the time of sowing or shortly thereafter protect plants as well as seed treatment in prior to sowing. It is likewise considered to be advantageous that the inventive active ingredients or compositions can be used especially also for transgenic seed, in which case the plant which grows from this seed is capable of expressing a protein which acts against pests, herbicidal damage or abiotic stress. The treatment of such seeds with the inventive active ingredients or compositions, for example an insecticidal protein, can result in control of certain pests. Surprisingly, a further synergistic effect can be observed in this case, which additionally increases the effectiveness for protection against attack by pests., microorganisms, weeds or abiotic stress.

The compounds of the formula (I) are suitable for protection of seed of any plant variety which is used in agriculture, in the greenhouse, in forests or in horticulture. More particularly, the seed is that of cereals (such as wheat, barley, rye, millet and oats), oilseed rape, maize, cotton, soybeen, rice, potatoes, sunflower, beans, coffee, beet (e.g. sugar beet and fodder beet), peanut, vegetables (such as tomato, cucumber, onions and lettuce), lawns and ornamental plants. Of particular significance is the treatment of the seed ofwheat, soybean, oilseed rape, maize and rice.

As also described below, the treatment of transgenic seed with the inventive active ingredients or compositions is of particular significance. This refers to the seed of plants containing at least one heterologous gene which allows the expression of a polypeptide or protein, e.g. having insecticidal properties. These heterologous genes in transgenic seeds may originate, for example, from microorganisms of the species Bacillus, Rhizobium, Pseudomonas, Serratia, Trichoderma, Clavibacter, Glomus or Gliocladium. These heterologous genes preferably originates from Bacillus sp., in which case the gene product is effective against the European corn borer and/or the Western corn rootworm. Particularly preferably, the heterologous genes originate from Bacillus thuringiensis.

In the context of the present invention, the inventive composition is applied to seeds either alone or in a suitable formulation. Preferably, the seed is treated in a state in which it is sufficiently stable for no damage to occur in the course of treatment. In general, seeds can be treated at any time between harvest and some time after sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15% by weight. Alternatively, it is also possible to use seed which, after drying, for example, has been treated with water and then dried again, or seeds just after priming, or seeds stored in primed conditions or pre-germinated seeds, or seeds sown on nursery trays, tapes or paper.

When treating the seeds, it generally has to be ensured that the amount of the inventive composition applied to the seed and/or the amount of further additives is selected such that the germination of the seed is not impaired, or that the resulting plant is not damaged. This must be ensured particularly in the case of active ingredients which can exhibit phytotoxic effects at certain application rates.

The compounds of the formula (I) can be applied directly, i.e. without containing any other components and without having been diluted. In general, it is preferable to apply the compositions to the seed in the form of a suitable formulation. Suitable formulations and methods for seed treatment are known to those skilled in the art. The compounds of the formula (I) can be converted to the customary formulations relevant to on-seed applications, such as solutions, emulsions, suspensions, powders, foams, slurries or combined with other coating compositions for seed, such as film forming materials, pelleting materials, fine iron or other metal powders, granules, coating material for inactivated seeds, and also ULV formulations.

These formulations are prepared in a known manner, by mixing the active ingredients or active ingredient combinations with customary additives, for example customary extenders and solvents or diluents, dyes, wetting agents, dispersants, emulsifiers, antifoams, preservatives, secondary thickeners, adhesives, gibberellins, and also water.

Useful dyes which may be present in the seed dressing formulations usable in accordance with the invention are all dyes which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of active agrochemical ingredients. Usable with preference are alkylnaphthalenesulphonates, such as diisopropyl- or diisobutylnaphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Useful nonionic dispersants include especially ethylene oxide/propylene oxide block polymers, alkylphenol polyglycol ethers and tristryrylphenol polyglycol ether, and the phosphated or sulphated derivatives thereof. Suitable anionic dispersants are especially lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Silicone antifoams and magnesium stearate can be used with preference.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances usable for such purposes in agrochemical compositions. Preferred examples include cellulose derivatives, acrylic acid derivatives, xanthan, modified clays and finely divided silica.

Adhesives which may be present in the seed dressing formulations usable in accordance with the invention are all customary binders usable in seed dressing products. Preferred examples include polyvinylpyrrolidone, polyvinyl acetate, polyvinyl alcohol and tylose.

The formulations for on-seed applications usable in accordance with the invention can be used to treat a wide variety of different kinds of seed either directly or after prior dilution with water. For instance, the concentrates or the preparations obtainable therefrom by dilution with water can be used to dress the seed of cereals, such as wheat, barley, rye, oats, and triticale, and also seeds of maize, soybean, rice, oilseed rape, peas, beans, cotton, sunflowers, and beets, or else a wide variety of different vegetable seeds. The formulations usable in accordance with the invention, or the dilute preparations thereof, can also be used for seeds of transgenic plants. In this case, additional synergistic effects may also occur in interaction with the substances formed by expression.

For treatment of seeds with the formulations usable in accordance with the invention, or the preparations prepared therefrom by adding water, all mixing units usable customarily for on-seed applications are useful. Specifically, the procedure in on-seed applications is to place the seeds into a mixer, to add the particular desired amount of the formulations, either as such or after prior dilution with water, and to mix everything until all applied formulations are distributed homogeneously on the seeds. If appropriate, this is followed by a drying operation.

The application rate of the formulations usable in accordance with the invention can be varied within a relatively wide range. It is guided by the particular content of the active ingredients in the formulations and by the seeds. The application rates of each single active ingredient is generally between 0.001 and 15 g per kilogram of seed, preferably between 0.01 and 5 g per kilogram of seed.

### Antimycotic Effects

In addition, the compounds of the formula (I) also have very good antimycotic effects. They have a very broad antimycotic activity spectrum, especially against dermatophytes and yeasts, moulds and diphasic fungi (for example against Candida species, such as Candida albicans, Candida glabrata), and Epidermophyton floccosum, Aspergillus species, such as Aspergillus niger and Aspergillus fumigatus, Trichophyton species, such as Trichophyton mentagrophytes, Microsporon species such as Microsporon canis and audouinii. The enumeration of these fungi by no means constitutes a restriction of the mycotic spectrum covered, and is merely of illustrative character.

The compounds can be used also to control important fungal pathogens in fish and crustacea farming, e.g. saprolegnia diclina in trouts, saprolegnia parasitica in crayfish.

The compounds of the formula (I) can therefore be used both in medical and in non-medical applications.

The compounds of the formula (I) can be used as such, in the form of their formulations or the use forms prepared therefrom, such as ready-to-use solutions, suspensions, wettable powders, pastes, soluble powders, dusts and granules. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

### GMO

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts" or "parts of plants" or "plant parts" have been explained above. More preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance.

Plants or plant cultivars (obtained by plant biotechnology methods such as genetic engineering) which may also be treated according to the invention show altered quantity, quality and/or storage-stability of the harvested product and/or altered properties of specific ingredients of the harvested product.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics.

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics.

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering.

Plants or plant cultivars (that can be obtained by plant biotechnology methods such as genetic engineering) which may also be treated according to the invention are plants, such as Tobacco plants, with altered post-translational protein modification patterns.

### Application Rates

When using the compounds of the formula (I) as fungicides, the application rates can be varied within a relatively wide range, depending on the kind of application. The application rate of the inventive active ingredients is
- in the case of treatment of plant parts, for example leaves: from 0.1 to 10 000 g/ha, preferably from 10 to 1000 g/ha, more preferably from 50 to 300 g/ha (in the case of application by watering or dripping, it is even possible to reduce the application rate, especially when inert substrates such as rockwool or perlite are used);
- in the case of seed treatment: from 0.1 to 200 g per 100 kg of seed, preferably from 1 to 150 g per 100 kg of seed, more preferably from 2.5 to 25 g per 100 kg of seed, even more preferably from 2.5 to 12.5 g per 100 kg of seed;
- in the case of soil treatment: from 0.1 to 10 000 g/ha, preferably from 1 to 5000 g/ha.

These application rates are merely by way of example and are not limiting for the purposes of the invention.

The invention is illustrated by the examples below. However, the invention is not limited to the examples.

### Examples

### Preparation examples

### Preparation of compounds of the formula (I) according to process J:

### Example 1: Preparation of 1-(2-chloro-3-fluoro-4-pyridyl)-2-(2,4-difluorophenyl)-3-(1,2,4-triazol-1-yl)propan-2-ol (I-08)

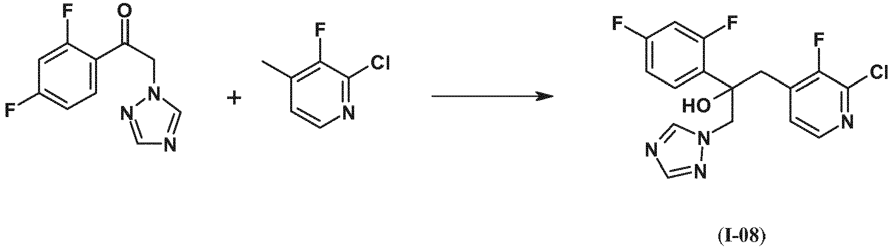

To a solution of 2,2,6,6-tetramethylpiperidinylmagnesium chloride (11.9 g, 2.2 eq, 49.3 mmol) in THF (120 mL) at -70 °C was added a solution of 2-chloro-3-fluoro-4-methyl-pyridine (7.14 g, 2.2 eq, 49.3 mmol) in THF (40 mL) over 10 min. The reaction was stirred for 30 min at -78 °C for 30 min, before a solution of 1-(2,4-difluorophenyl)-2-(1,2,4-triazol-1-yl)ethanone (5.0 g, 1 eq, 22.4 mmol) in THF (100 mL) was added over 20 min. After complete addition, the reaction was stirred for 30 min at -70 °C, subsequently warmed to rt (room temperature = 21°C) and stirred for 18 h at rt. The reaction was then quenched with NH₄Cl (sat. aq. solution), diluted with water, extracted with ethyl acetate, washed with brine, dried (MgSO₄), and concentrated. The crude product was treated with di-isopropyl ether (50 mL) and filtered, the solid was then taken up in ethyl acetate (40 mL), heated to 50 °C for 10 min, cooled down with an ice bath, and filtered. The obtained solid was washed with di-isopropyl ether (2x20 mL), and dried *in vacuo* to give 5.20 g (63% yield, 100% pure) of the target compound (**I-08**) as colourless solid.
MS (ESI): 369.0 ([M+H]⁺)

### Preparation of compounds of the formula (I) according to process H:

### Example 2: Preparation of 2-(4-chloro-2-fluoro-phenyl)-1-(2-chloro-3-pyridyl)-3-(1,2,4-triazol-1-yl)propan-2-ol (I-29)

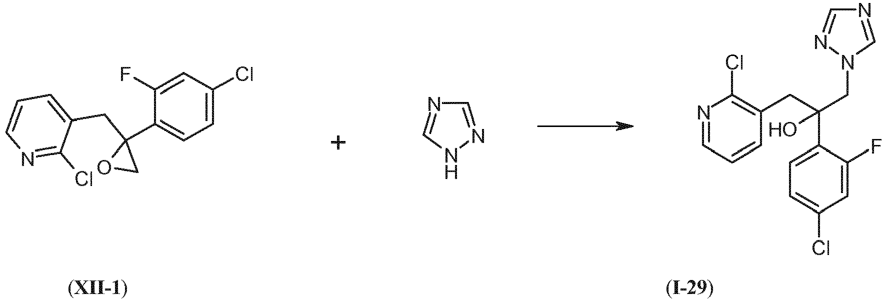

To a solution of 1*H*-1,2,4-triazole (339 mg, 3 eq, 4.9 mmol) in dimethyl formamide (0.5 mL) was added potassium carbonate (679 mg, 3 eq, 4.9 mmol), and the mixture was stirred for 10 min at rt, before 2-chloro-3-[[2-(4-chloro-2-fluoro-phenyl)oxiran-2-yl]methyl]pyridine (**XII-1**) (610 mg, 1 eq, 1.64 mmol, 80% purity) in dimethyl formamide (1 mL) and potassium *tert*-butoxide (1.8 mg, 0.01 eq) were added. The whole was warmed to 40 °C for 20 h, after which the solvent was evaporated and the remains were taken up in ethyl acetate and water. The crude product was purified by chromatography over silica. After evaporation of the solvent 260 mg (42% yield, 97% pure) of the target compound (**I-29**) were obtained.
MS (ESI): 367.0 ([M+H]⁺)

### Preparation of compounds of the formula (XII) according to process D:

### Example 3: Preparation of 2-chloro-3-[[2-(4-chloro-2-fluoro-phenyl)oxiran-2-yl]methyl]pyridine (XII-1)

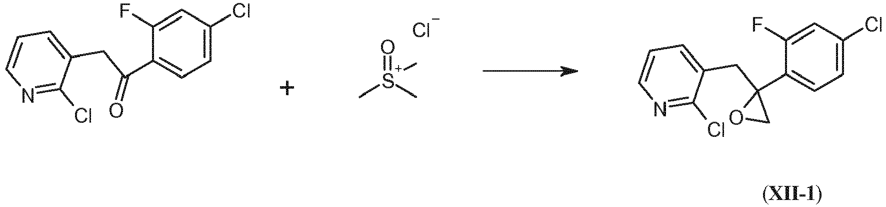

To a solution of 1-(4-chloro-2-fluoro-phenyl)-2-(2-chloro-3-pyridyl)ethanone (1.1 g, 1 eq, 3.8 mmol) and trimethylsulfoxonium chloride (548 mg, 1.1 eq, 4.2 mmol) in toluene (4 g) was added NaOH (378 mg, 1.1 eq, 4.2 mmol), and the whole was heated at 60 °C for 16 h. Water was added, the organic layer was dried and concentrated. The crude product was purified by chromatography over silica. After evaporation of the solvent 610 mg (42% yield, 80% pure) of the target compound were obtained.
MS (ESI): 298.0 ([M+H]⁺)

The following Table 1 illustrates in a non -limiting manner examples of compounds according to formula (I).

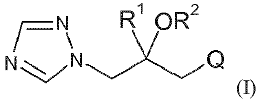

**Table 1**

| **Ex N°** | **R¹** | **R²** | **Q** | **LogP** |
|---|---|---|---|---|
| I-01 | 4-phenoxyphenyl | H | 3-chloropyridin-4-yl | 2,46^{[a]} |
| I-02 | biphenyl-4-yl | H | 3-chloropyridin-4-yl | 2,46^{[a]} |
| I-03 | 4-phenoxyphenyl | H | 3-fluoropyridin-4-yl | 2,13^{[a]} |
| I-04 | biphenyl-4-yl | H | 3-fluoropyridin-4-yl | 2,13^{[a]} |
| I-05 | 4-fluorophenyl | H | 2-chloropyridin-4-yl | 1,69^{[a]} |
| I-06 | 4-(4-chlorophenoxy)phenyl | H | 3-chloropyridin-4-yl | 2,90^{[a]} |
| I-07 | 2-chlorophenyl | H | 2-chloro-3-fluoropyridin-4-yl | 2,27^{[a]} |
| I-08(*) | 2,4-difluorophenyl | H | 2-chloro-3-fluoropyridin-4-yl | 2,14^{[a]} |
| I-09 | 2,4-dichlorophenyl | H | 2-chloro-3-fluoropyridin-4-yl | 2,69^{[a]} |
| I-10 | 4-chlorophenyl | H | 2-chloro-3-fluoropyridin-4-yl | 2,23^{[a]} |
| I-11 | 4-chlorophenyl | H | 3-chloropyridin-4-yl | 1,86^{[a]} |
| I-12 | 2-chlorophenyl | H | 3-chloropyridin-4-yl | 1,93^{[a]}; 1,97^{[b]} |
| I-13 | 4-fluorophenyl | H | 3-fluoropyridin-4-yl | 1,26^{[a]} |
| I-14 | phenyl | H | 5-chloro-2-fluoropyridin-4-yl | 2,02^{[a]} |
| I-15 | 4-fluorophenyl | H | 5-chloro-2-fluoropyridin-4-yl | 2,12^{[a]} |
| I-16 | 4-(trifluoromethyl)phenyl | H | 2-chloropyridin-3-yl | 2,22^{[a]} |
| I-17 | 3-chlorophenyl | H | 2-chloropyridin-3-yl | 1,97^{[a]} |
| I-18 | 4- fluorophenyl | H | 3-chloropyridin-4-yl | 1,57^{[a]} |
| I-19 | 4-(2,4-difluorophenoxy)phenyl | H | 3-chloropyridin-4-yl | 2,57^{[a]} |
| I-20 | 4-(2,4-difluorophenoxy)phenyl | H | 3-fluoropyridin-4-yl | 2,27^{[a]} |
| I-21 | 3-chlorophenyl | H | 3-chloropyridin-4-yl | 1,85^{[a]}; 1,90^{[b]} |
| I-22 | 3-phenoxyphenyl | H | 3-chloropyridin-4-yl | 2,43^{[a]}; 2,42^{[b]} |
| I-23 | 4-fluorophenyl | H | 2-chloro-3-fluoropyridin-4-yl | 1,91^{[a]} |
| I-24 | 4-phenoxyphenyl | H | 2-chloropyridin-4-yl | 2,57^{[a]} |
| I-25 | 4-(2,4-difluorophenoxy)phenyl | H | 2-chloropyridin-4-yl | 2,69^{[a]} |
| I-26 | biphenyl-4-yl | H | 2-chloro-5-fluoropyridin-4-yl | 2,88^{[a]} |
| I-27 | biphenyl-4-yl | H | 5-chloro-2-fluoropyridin-4-yl | 3,02^{[a]} |
| I-28 | 4-chlorophenyl | H | 5-chloro-2-fluoropyridin-4-yl | 2,43^{[a]} |
| I-29 | 4-chloro-2-fluorophenyl | H | 2-chloropyridin-3-yl | 2,15^{[a]} |
| I-30 | 2,4-difluorophenyl | H | 3-chloropyridin-4-yl | 1,76^{[a]} |
| I-31 | 4-chlorophenyl | H | 3-fluoropyridin-4-yl | 1,54^{[a]} |
| I-32 | 4-fluorophenyl | H | 2-chloro-5-fluoropyridin-4-yl | 1,96^{[a]} |
| I-33 | phenyl | H | 3-chloropyridin-4-yl | 1,48^{[a]}; 1,59^{[b]} |
| I-34 | 4-phenoxyphenyl | H | 5-chloro-2-fluoropyridin-4-yl | 3,02^{[a]} |
| I-35 | 4-cyanophenyl | H | 2-chloropyridin-3-yl | 1,47^{[a]} |
| I-36 | phenyl | H | 3-fluoropyridin-4-yl | 1,13^{[a]} |
| I-37 | phenyl | H | 2-chloropyridin-4-yl | 1,60^{[a]} |
| I-38 | 4-(3-tert-butyl-l,2,4-oxadiazol-5-yl)phenyl | H | 3-chloropyridin-4-yl | 2,65^{[a]} |
| I-39 | biphenyl-4-yl | H | 2-chloropyridin-4-yl | 2,57^{[a]} |
| I-40 | 4-chlorophenyl | H | 2-chloro-5-fluoropyridin-4-yl | 2,27^{[a]} |
| I-41 | 2,4-difluorophenyl | H | 5-chloro-2-fluoropyridin-4-yl | 2,35^{[a]} |
| I-42 | 2,4-dichlorophenyl | H | 3-chloropyridin-4-yl | 2,38^{[a]} |
| I-43 | 2,4-difluorophenyl | H | 3-fluoropyridin-4-yl | 1,47^{[a]} |
| I-44 | 2,4-difluorophenyl | H | 2-chloropyridin-4-yl | 1,86^{[a]} |
| I-45 | 2,4-difluorophenyl | H | 2-chloro-5-fluoropyridin-4-yl | 2,13^{[a]} |
| I-46 | 4-phenoxyphenyl | H | 2-chloro-3-fluoropyridin-4-yl | 2,84^{[a]} |
| I-47 | 4-chlorophenyl | H | 2-chloropyridin-4-yl | 1,96^{[a]} |
| I-48 | phenyl | H | 2-chloro-5-fluoropyridin-4-yl | 1,86^{[a]} |
| I-49 | phenyl | H | 2-chloro-3-fluoropyridin-4-yl | 1,84^{[a]} |
| I-50 | 2,4-dichlorophenyl | H | 2-chloropyridin-4-yl | 2,42^{[a]} |
| I-51 | phenyl | H | 2-chloropyridin-3-yl | 1,61^{[a]} |
| I-52 | 4-chlorophenyl | H | 2 -chloropyridin-3 -yl | 1,99^{[a]} |
| I-53 | 2,4-dichlorophenyl | H | 3-fluoropyridin-4-yl | 2,00^{[a]} |
| I-54 | 4-(2,4-difluorophenoxy)phenyl | H | 5-chloro-2-fluoropyridin-4-yl | 3,02^{[a]} |
| I-55(*) | 2,4-difluorophenyl | H | 2-chloro-3-fluoropyridin-4-yl | 2,21^{[a]} |
| I-56(*) | 2,4-difluorophenyl | H | 2-chloro-3-fluoropyridin-4-yl | 2,21^{[a]} |
| I-57 | 2,4-difluorophenyl | methyl | 2-chloro-3-fluoropyridin-4-yl | 2,47^{[a]} |

| | | | | |
|---|---|---|---|---|
| Optical rotation Concentration c is expressed in g/100 mL **(*) Ex I-55 and 1-56 are the 2 enantiomers of Ex I-08** Ex I-55: Optical rotation: + 3.1 (c = 1,129; DCM, 25°C) Ex I-56: Optical rotation: - 3.4 (c = 1,160; DCM, 25°C) | | | | |

The following Table 2 illustrates in a non -limiting manner examples of compounds according to formula (XII).

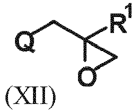

**Table 2**

| **Ex N°** | **R¹** | **Q** | **LogP** |
|---|---|---|---|
| XII-1 | 4-chloro-2- fluorophenyl | 2-chloropyridin-3-yl | 3,31^{[a]} |
| XII-2 | 4-chlorophenyl | 3-fluoropyridin-4-yl | 2,65^{[a]} |
| XII-3 | 2,4-dichlorophenyl | 3-fluoropyridin-4-yl | 3,19^{[a]} |
| XII-4 | 4-fluorophenyl | 3-fluoropyridin-4-yl | 2,23^{[a]} |
| XII-5 | 4-(2,4-difluorophenoxy)phenyl | 3-fluoropyridin-4-yl | 3,37^{[a]} |
| XII-6 | 2,4-difluorophenyl | 3-fluoropyridin-4-yl | 2,38^{[a]} |
| XII-7 | 4-phenoxyphenyl | 3-fluoropyridin-4-yl | 3,37^{[a]} |
| XII-8 | biphenyl-4-yl | 3-fluoropyridin-4-yl | 3,37^{[a]} |
| XII-9 | 4-phenoxyphenyl | 2-chloropyridin-4-yl | 3,37^{[a]} |
| XII-10 | 2,4-dichlorophenyl | 2-chloropyridin-4-yl | 3,85^{[a]} |
| XII-11 | 2,4-difluorophenyl | 2-chloropyridin-4-yl | 2,93^{[a]} |
| XII-12 | 4-(2,4-difluorophenoxy)phenyl | 2-chloropyridin-4-yl | 3,85^{[a]} |
| XII-13 | biphenyl-4-yl | 2-chloropyridin-4-yl | 3,89^{[a]} |
| XII-14 | 4-fluorophenyl | 2-chloropyridin-4-yl | 2,76^{[a]} |
| XII-15 | 4-chlorophenyl | 2-chloropyridin-4-yl | 3,19^{[a]} |
| XII-16 | 4-fluorophenyl | 2-chloro-5-fluoropyridin-4-yl | 3,15^{[a]} |
| XII-17 | 4-chlorophenyl | 2-chloro-5-fluoropyridin-4-yl | 3,56^{[a]} |
| XII-18 | 4-phenoxyphenyl | 2-chloro-5-fluoropyridin-4-yl | 4,23^{[a]} |
| XII-19 | 2,4-dichlorophenyl | 2-chloro-5-fluoropyridin-4-yl | 4,17^{[a]} |
| XII-20 | 2,4-difluorophenyl | 2-chloro-5-fluoropyridin-4-yl | 3,24^{[a]} |
| XII-21 | 4-(2, 4-difluorophenoxy)phenyl | 2-chloro-5-fluoropyridin-4-yl | 4,17^{[a]} |
| XII-22 | biphenyl-4-yl | 2-chloro-5-fluoropyridin-4-yl | 4,28^{[a]} |
| XII-23 | 3-chlorophenyl | 2-chloropyridin-3-yl | 3,24^{[a]} |
| XII-24 | 4-chlorophenyl | 2-chloropyridin-3-yl | 3,25^{[a]} |
| XII-25 | 4 -(trifluoromethyl)phenyl | 2-chloropyridin-3-yl | 3,46^{[a]} |
| XII-26 | 4-cyanophenyl | 2-chloropyridin-3-yl | 2,35^{[a]} |
| XII-27 | 4-fluorophenyl | 3-chloropyridin-4-yl | 2,65^{[a]} |
| XII-28 | biphenyl-4-yl | 3-chloropyridin-4-yl | 3,85^{[a]} |
| XII-29 | 2-chlorophenyl | 3-chloropyridin-4-yl | 3,02^{[a]} |
| XII-30 | 4-chlorophenyl | 3-chloropyridin-4-yl | 3,15^{[a]} |
| XII-31 | 2,4-dichlorophenyl | 3-chloropyridin-4-yl | 3,06^{[a]} |
| XII-32 | 4-phenoxyphenyl | 3-chloropyridin-4-yl | 3,80^{[a]} |
| XII-33 | 4-(2,4-difluorophenoxy)phenyl | 3-chloropyridin-4-yl | 3,80^{[a]} |
| XII-34 | 2,4-difluorophenyl | 3-chloropyridin-4-yl | 2,76^{[a]} |
| XII-35 | 2,4-difluorophenyl | 5-chloro-2-fluoropyridin-4-yl | 3,46^{[a]} |
| XII-36 | 2,4-dichlorophenyl | 5-chloro-2-fluoropyridin-4-yl | 4,45^{[a]} |
| XII-37 | 4-chlorophenyl | 5-chloro-2-fluoropyridin-4-yl | 3,85^{[a]} |
| XII-38 | 2-chlorophenyl | 5-chloro-2-fluoropyridin-4-yl | 3,80^{[a]} |
| XII-39 | 4-fluorophenyl | 5-chloro-2-fluoropyridin-4-yl | 3,37^{[a]} |
| XII-40 | 4-phenoxyphenyl | 5-chloro-2-fluoropyridin-4-yl | 4,57^{[a]} |
| XII-41 | 4-(2,4-difluorophenoxy)phenyl | 5-chloro-2-fluoropyridin-4-yl | 4,51^{[a]} |
| XII-42 | biphenyl-4-yl | 5-chloro-2-fluoropyridin-4-yl | 4,64^{[a]} |
| XII-43 | 4-chlorophenyl | 2-chloro-3-fluoropyridin-4-yl | 3,58^{[a]} |
| XII-44 | 4-(2,4-difluorophenoxy)phenyl | 2-chloro-3-fluoropyridin-4-yl | 4,20^{[a]} |
| XII-45 | biphenyl-4-yl | 2-chloro-3-fluoropyridin-4-yl | 4,25^{[a]} |
| XII-46 | 2,4-dichlorophenyl | 2-chloro-3-fluoropyridin-4-yl | 3,02^{[a]} |
| XII-47 | 2,4-difluorophenyl | 2-chloro-3-fluoropyridin-4-yl | 3,26^{[a]} |
| XII-48 | 4-phenoxyphenyl | 2-chloro-3-fluoropyridin-4-yl | 4,20^{[a]} |
| XII-49 | 2-chlorophenyl | 2-chloro-3-fluoropyridin-4-yl | 3,47^{[a]} |
| XII-50 | 4-fluorophenyl | 2-chloro-3-fluoropyridin-4-yl | 3,11^{[a]} |
| XII-51 | 4-phenoxyphenyl | 3,5-dichloropyridin-4-yl | 4,76^{[a]} |
| XII-52 | 4-(2,4-difluorophenoxy)phenyl | 3,5-dichloropyridin-4-yl | 4,70^{[a]} |
| XII-53 | biphenyl-4-yl | 3,5-dichloropyridin-4-yl | 4,89^{[a]} |
| XII-54 | 2,4-dichlorophenyl | 3,5-dichloropyridin-4-yl | 4,76^{[a]} |
| XII-55 | 2,4-difluorophenyl | 3,5-dichloropyridin-4-yl | 3,68^{[a]} |
| XII-56 | 4-fluorophenyl | 3,5-dichloropyridin-4-yl | 3,58^{[a]} |
| XII-57 | 4-chlorophenyl | 3,5-dichloropyridin-4-yl | 4,10^{[a]} |
| XII-58 | phenyl | 3,5-dichloropyridin-4-yl | 3,53^{[a]} |
| XII-59 | 2-chlorophenyl | 3,5-dichloropyridin-4-yl | 4,04^{[a]} |
| XII-60 | mesityl | 3-chloropyridin-4-yl | 4,15^{[a]} |
| XII-61 | 2,3-dichlorophenyl | 3-chloropyridin-4-yl | 3,53^{[a]} |
| XII-62 | 2,6-dichlorophenyl | 3-chloropyridin-4-yl | 3,02^{[a]} |
| XII-63 | 3-[(2-chlorobenzyl)oxy]phenyl | 3-chloropyridin-4-yl | 4,30^{[a]} |
| XII-64 | 4-[(2-chlorobenzyl)oxy]phenyl | 3-chloropyridin-4-yl | 4,30^{[a]} |
| XII-65 | 4-(3-tert-butyl-1,2,4-oxadiazol-5-yl)phenyl | 3-chloropyridin-4-yl | 4,25^{[a]} |
| XII-66 | 2,6-difluorophenyl | 3-chloropyridin-4-yl | 2,50^{[a]} |

| | | | |
|---|---|---|---|
| Measurement of LogP values was performed according to EEC directive 79/831 Annex V.A8 by HPLC (High Performance Liquid Chromatography) on reversed phase columns with the following methods: ^{[a]} LogP value is determined by measurement of LC-UV, in an acidic range, with 0.1% formic acid in water and acetonitrile as eluent (linear gradient from 10% acetonitrile to 95% acetonitrile). ^{[b]} LogP value is determined by measurement of LC-UV, in a neutral range, with 0.001 molar ammonium acetate solution in water and acetonitrile as eluent (linear gradient from 10% acetonitrile to 95% acetonitrile). ^{[c]} LogP value is determined by measurement of LC-UV, in an acidic range, with 0.1% phosphoric acid and acetonitrile as eluent (linear gradient from 10% acetonitrile to 95% acetonitrile). | | | |

If more than one LogP value is available within the same method, all the values are given and separated by "+".

Calibration was done with straight-chain alkan2-ones (with 3 to 16 carbon atoms) with known LogP values (measurement of LogP values using retention times with linear interpolation between successive alkanones). Lambda-max-values were determined using UV-spectra from 200 nm to 400 nm and the peak values of the chromatographic signals.

### NMR-Peak lists

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ₁ (intensity₁); δ₂ (intensity₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, we use tetramethylsilane and/or the chemical shift of the solvent used, especially in the case of spectra measured in DMSO. Therefore in NMR peak lists, tetramethylsilane peak can occur but not necessarily.

The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity.

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists you find in the publication "Citation of NMR Peaklist Data within Patent Applications" of the Research Disclosure Database Number 564025.

| |
|---|
| Example I-01: ¹H-NMR (400.0 MHz, d₆-DMSO): |
| δ= 8.433 (11.0); 8.294 (7.1); 8.282 (16.0); 7.922 (13.5); 7.406 (0.8); 7.401 (4.9); 7.395 (1.8); 7.388 (1.1); 7.382 (7.1); 7.379 (6.7); 7.373 (1.1); 7.366 (2.3); 7.361 (6.3); 7.355 (1.8); 7.348 (8.6); 7.342 (2.8); 7.331 (3.0); 7.326 (9.5); 7.318 (1.1); 7.286 (5.3); 7.273 (5.2); 7.140 (1.6); 7.138 (2.9); 7.135 (1.7); 7.119 (4.8); 7.103 (1.3); 7.101 (2.4); 7.098 (1.3); 6.944 (1.5); 6.940 (6.5); 6.938 (8.1); 6.932 (2.2); 6.923 (2.3); 6.921 (3.9); 6.919 (7.0); 6.916 (6.1); 6.910 (0.7); 6.889 (1.2); 6.881 (9.9); 6.876 (2.9); 6.864 (2.9); 6.859 (9.2); 6.852 (0.9); 5.949 (0.5); 5.839 (12.0); 4.726 (1.8); 4.690 (5.6); 4.668 (5.6); 4.632 (1.8); 3.431 (3.2); 3.395 (4.1); 3.345 (20.4); 3.204 (4.1); 3.168 (3.2); 2.545 (45.2); 2.523 (0.4); 2.514 (5.0); 2.510 (10.2); 2.505 (13.4); 2.501 (9.5); 2.496 (4.4); 0.000 (1.1) |
| Example I-02: ¹H-NMR (400.0 MHz, d₆-DMSO): |
| δ= 8.532 (6.7); 8.422 (12.3); 8.315 (0.7); 8.273 (14.3); 8.266 (7.6); 8.254 (7.7); 8.152 (4.5); 8.131 (5.6); 8.035 (6.6); 7.925 (5.6); 7.907 (16.0); 7.808 (2.7); 7.804 (3.8); 7.787 (3.9); 7.665 (5.3); 7.662 (7.0); 7.644 (7.9); 7.581 (7.9); 7.559 (11.2); 7.549 (2.3); 7.532 (4.5); 7.528 (2.3); 7.512 (2.9); 7.486 (0.4); 7.475 (2.0); 7.461 (4.9); 7.456 (4.4); 7.446 (13.1); 7.442 (12.2); 7.425 (10.8); 7.378 (0.3); 7.363 (3.4); 7.349 (1.6); 7.344 (4.4); 7.340 (1.4); 7.326 (1.6); 7.293 (5.7); 7.281 (5.7); 6.030 (11.3); 5.881 (12.4); 4.714 (0.7); 4.678 (8.4); 4.673 (8.4); 4.637 (0.7); 3.495 (3.4); 3.459 (4.4); 3.355 (0.9); 3.325 (468.6); 3.304 (0.8); 3.287 (0.4); 3.280 (0.4); 3.262 (0.4); 3.245 (4.4); 3.209 (3.5); 2.995 (0.7); 2.675 (1.4); 2.671 (2.0); 2.666 (1.4); 2.541 (99.0); 2.524 (5.4); 2.511 (119.5); 2.506 (240.7); 2.502 (314.6); 2.497 (222.8); 2.493 (104.1); 2.459 (0.6); 2.444 (0.5); 2.439 (0.4); 2.367 (0.5); 2.333 (1.5); 2.328 (2.0); 2.324 (1.4); 1.235 (0.6); 0.008 (0.5); 0.000 (12.8); - 0.008 (0.4) |
| Example I-03: ¹H-NMR (400.0 MHz, d₆-DMSO): |
| δ= 8.319 (8.0); 8.315 (8.1); 8.258 (15.8); 8.201 (5.1); 8.190 (5.2); 8.189 (5.2); 7.900 (16.0); 7.408 (0.9); 7.402 (5.7); 7.397 (2.2); 7.384 (8.5); 7.381 (8.4); 7.376 (1.4); 7.367 (2.6); 7.362 (7.2); 7.357 (1.1); 7.339 (10.0); 7.335 (3.6); 7.318 (11.1); 7.310 (1.4); 7.203 (3.2); 7.190 (3.9); 7.188 (4.0); 7.175 (3.1); 7.137 (3.3); 7.135 (2.2); 7.119 (5.6); 7.100 (2.7); 7.098 (1.6); 6.926 (7.6); 6.924 (9.5); 6.919 (2.8); 6.904 (8.3); 6.902 (7.4); 6.896 (1.0); 6.878 (1.4); 6.871 (12.0); 6.867 (3.9); 6.849 (11.1); 6.842 (1.2); 5.947 (0.4); 5.810 (14.1); 4.672 (16.0); 3.342 (34.2); 3.303 (4.7); 3.092 (4.6); 3.058 (3.6); 3.000 (0.4); 2.713 (0.5); 2.544 (105.7); 2.526 (0.5); 2.522 (0.6); 2.513 (7.8); 2.509 (15.9); 2.504 (21.1); 2.500 (15.3); 2.495 (7.4); 2.370 (0.5); 0.000 (7.2) |
| Example I-04: ¹H-NMR (400.0 MHz, d₆-DMSO): |
| δ= 8.303 (7.7); 8.299 (7.7); 8.257 (15.4); 8.179 (4.9); 8.167 (5.0); 7.889 (16.0); 7.665 (6.0); 7.662 (7.9); 7.657 (2.3); 7.644 (8.9); 7.570 (8.9); 7.549 (12.2); 7.460 (4.7); 7.456 (1.9); 7.442 (10.2); 7.439 (6.7); 7.432 (11.8); 7.427 (5.4); 7.422 (6.8); 7.411 (8.4); 7.365 (2.2); 7.362 (3.6); 7.359 (2.2); 7.348 (1.7); 7.343 (4.9); 7.339 (1.3); 7.328 (1.1); 7.325 (1.7); 7.322 (1.0); 7.220 (3.0); 7.207 (3.8); 7.205 (3.8); 7.192 (2.9); 5.853 (14.2); 4.681 (15.0); 3.396 (3.6); 3.361 (4.7); 3.333 (227.1); 3.131 (4.3); 3.096 (3.4); 2.996 (0.7); 2.711 (0.6); 2.676 (0.4); 2.671 (0.5); 2.667 (0.4); 2.567 (0.4); 2.562 (0.4); 2.558 (0.6); 2.542 (152.4); 2.525 (1.4); 2.511 (30.9); 2.507 (62.0); 2.502 (80.7); 2.498 (56.9); 2.493 (26.6); 2.368 (0.6); 2.333 (0.4); 2.329 (0.5); 2.324 (0.4); 0.008 (0.6); 0.000 (17.2); -0.009 (0.5) |
| Example I-05: ¹H-NMR (400.0 MHz, d₆-DMSO): |
| δ= 8.192 (15.6); 8.151 (6.9); 8.138 (7.0); 7.870 (16.0); 7.387 (0.6); 7.379 (5.1); 7.374 (2.2); 7.365 (5.8); 7.357 (6.1); 7.348 (2.5); 7.343 (5.5); 7.335 (0.6); 7.139 (8.5); 7.097 (0.7); 7.089 (6.0); 7.084 (1.9); 7.067 (10.9); 7.050 (1.8); 7.045 (5.1); 7.037 (0.5); 7.019 (4.7); 7.016 (4.5); 7.006 (4.6); 7.003 (4.4); 5.883 (15.3); 4.631 (3.4); 4.596 (7.1); 4.546 (7.0); 4.510 (3.4); 3.351 (29.4); 3.276 (3.8); 3.242 (5.8); 3.144 (5.6); 3.110 (3.6); 3.005 (0.4); 2.719 (0.4); 2.568 (0.4); 2.550 (93.6); 2.533 (0.5); 2.528 (0.4); 2.519 (6.0); 2.515 (12.4); 2.510 (16.6); 2.505 (11.8); 2.501 (5.5); 2.375 (0.4); 2.081 (0.4) |
| Example I-06: ¹H-NMR (400.0 MHz, CD3CN): |
| δ= 8.427 (11.6); 8.247 (7.2); 8.234 (7.3); 8.067 (12.1); 7.926 (1.6); 7.814 (11.5); 7.755 (0.4); 7.733 (0.4); 7.379 (1.1); 7.371 (11.1); 7.365 (3.6); 7.354 (4.0); 7.348 (12.2); 7.343 (2.0); 7.335 (11.2); 7.330 (3.6); 7.318 (4.1); 7.313 (12.1); 7.306 (1.3); 7.150 (6.3); 7.138 (6.2); 7.038 (0.4); 7.016 (0.4); 6.942 (1.4); 6.933 (12.3); 6.928 (3.8); 6.916 (3.8); 6.911 (11.2); 6.900 (12.1); 6.895 (3.7); 6.883 (3.7); 6.878 (10.5); 6.870 (1.1); 4.731 (4.8); 4.695 (8.3); 4.606 (8.4); 4.570 (4.9); 4.488 (10.3); 4.085 (0.6); 4.068 (1.7); 4.050 (1.7); 4.032 (0.6); 3.874 (1.8); 3.383 (3.8); 3.348 (7.9); 3.289 (8.3); 3.254 (4.0); 2.889 (16.0); 2.772 (14.0); 2.157 (27.3); 2.107 (0.3); 1.972 (7.8); 1.964 (1.7); 1.958 (4.0); 1.952 (19.9); 1.946 (35.5); 1.940 (47.2); 1.934 (32.3); 1.928 (16.4); 1.436 (0.9); 1.221 (2.0); 1.203 (3.9); 1.186 (1.9); 0.146 (0.7); 0.008 (6.2); 0.000 (135.8); -0.008 (5.6); -0.150 (0.7) |
| Example I-07: ¹H-NMR (400.0 MHz, d₆-DMSO): |
| δ= 8.771 (0.5); 8.366 (0.8); 8.316 (1.8); 8.298 (16.0); 8.005 (8.6); 7.992 (8.9); 7.775 (15.7); 7.576 (0.3); 7.525 (0.5); 7.513 (0.6); 7.491 (0.4); 7.475 (0.5); 7.441 (5.4); 7.423 (6.1); 7.421 (6.1); 7.404 (0.5); 7.394 (0.6); 7.383 (0.5); 7.372 (0.7); 7.350 (0.5); 7.334 (0.4); 7.304 (4.8); 7.288 (5.0); 7.284 (5.5); 7.257 (2.6); 7.253 (2.7); 7.238 (4.9); 7.234 (4.5); 7.219 (3.0); 7.215 (2.6); 7.130 (3.8); 7.110 (6.2); 7.095 (9.5); 7.082 (3.9); 6.143 (14.6); 5.137 (5.7); 5.101 (6.9); 4.831 (6.8); 4.795 (5.5); 4.648 (0.8); 3.833 (4.8); 3.798 (5.3); 3.480 (0.4); 3.445 (0.6); 3.439 (0.6); 3.333 (1808.3); 3.258 (5.7); 3.224 (4.9); 2.995 (0.9); 2.711 (1.7); 2.675 (3.3); 2.671 (4.5); 2.611 (0.3); 2.542 (382.5); 2.506 (543.5); 2.502 (706.6); 2.498 (533.1); 2.409 (0.5); 2.367 (1.9); 2.329 (4.5); 2.324 (3.5); 1.298 (0.4); 1.258 (0.6); 1.235 (2.5); 0.146 (0.5); 0.007 (4.6); 0.000 (112.9); - 0.150 (0.6) |
| Example I-08: ¹H-NMR (499.9 MHz, CDCl₃): |
| δ= 8.027 (10.0); 8.017 (10.3); 7.947 (15.0); 7.836 (16.0); 7.268 (2.3); 7.264 (1.7); 7.255 (2.6); 7.250 (4.4); 7.237 (4.3); 7.232 (2.9); 7.219 (2.3); 7.085 (4.7); 7.075 (9.0); 7.065 (4.7); 6.815 (2.3); 6.810 (2.6); 6.799 (2.5); 6.793 (3.2); 6.791 (3.2); 6.786 (2.8); 6.774 (2.4); 6.769 (2.6); 6.711 (2.2); 6.706 (2.2); 6.694 (3.8); 6.692 (3.6); 6.690 (3.7); 6.677 (2.1); 6.673 (2.0); 5.162 (0.7); 4.975 (6.5); 4.947 (7.2); 4.484 (7.6); 4.456 (6.8); 3.313 (3.4); 3.286 (8.3); 3.252 (9.3); 3.225 (3.7); 2.041 (0.6); 1.257 (0.6); 0.006 (0.4); 0.000 (8.8) |
| Example I-09: ¹H-NMR (400.0 MHz, d₆-DMSO): |
| δ= 8.308 (16.0); 8.047 (8.1); 8.035 (8.4); 7.764 (15.4); 7.654 (0.5); 7.633 (0.6); 7.597 (8.2); 7.592 (8.4); 7.536 (0.5); 7.532 (0.5); 7.461 (0.5); 7.456 (0.5); 7.349 (0.4); 7.341 (0.6); 7.336 (0.4); 7.320 (0.4); 7.313 (0.8); 7.292 (0.4); 7.260 (5.3); 7.238 (12.0); 7.225 (1.0); 7.206 (6.5); 7.201 (6.3); 7.185 (2.9); 7.180 (3.0); 7.143 (3.9); 7.130 (7.2); 7.118 (3.7); 7.101 (1.3); 6.262 (14.2); 5.751 (1.0); 5.189 (5.7); 5.153 (6.4); 4.757 (6.4); 4.721 (5.5); 3.811 (4.9); 3.777 (5.3); 3.339 (182.3); 3.310 (0.7); 3.294 (5.6); 3.259 (4.8); 2.997 (0.3); 2.713 (0.5); 2.673 (0.4); 2.543 (106.8); 2.508 (50.6); 2.504 (66.0); 2.370 (0.5); 2.331 (0.4); 1.234 (0.7); 0.000 (12.4) |
| Example I-10: ¹H-NMR (400.0 MHz, d₆-DMSO): |
| δ= 8.257 (15.9); 8.036 (8.3); 8.024 (8.5); 7.866 (15.6); 7.392 (0.3); 7.366 (8.1); 7.344 (15.5); 7.303 (16.0); 7.281 (8.2); 7.164 (3.9); 7.152 (7.2); 7.139 (3.8); 5.966 (14.4); 4.730 (1.8); 4.694 (9.8); 4.682 (9.5); 4.646 (1.8); 3.410 (4.7); 3.368 (56.6); 3.178 (5.5); 3.144 (4.2); 2.719 (0.6); 2.549 (97.0); 2.509 (14.0); 2.506 (10.9); 2.375 (0.6); 0.000 (2.4) |
| Example I-11: ¹H-NMR (400.0 MHz, d₆-DMSO): |
| δ= 8.420 (12.7); 8.279 (7.8); 8.266 (10.1); 8.262 (14.8); 7.878 (13.8); 7.353 (7.5); 7.348 (3.0); 7.336 (4.4); 7.331 (14.1); 7.326 (2.3); 7.283 (14.5); 7.278 (4.1); 7.266 (9.4); 7.262 (8.6); 7.254 (6.8); 5.927 (12.6); 4.671 (16.0); 3.463 (4.2); 3.427 (5.3); 3.328 (49.1); 3.208 (5.3); 3.172 (4.1); 2.542 (49.4); 2.512 (21.4); 2.507 (40.1); 2.503 (50.5); 2.498 (36.0); 2.494 (17.3); 2.330 (0.3); 0.000 (2.5) |
| Example I-12: ¹H-NMR (400.0 MHz, CD3CN): |
| δ= 12.244 (2.1); 8.457 (7.6); 8.228 (6.5); 8.216 (7.1); 8.120 (12.2); 8.090 (16.0); 7.829 (0.5); 7.807 (0.5); 7.718 (7.0); 7.697 (0.4); 7.438 (2.6); 7.434 (2.8); 7.418 (3.1); 7.414 (3.3); 7.409 (2.8); 7.406 (2.7); 7.389 (3.1); 7.386 (3.2); 7.250 (1.3); 7.246 (1.4); 7.231 (2.6); 7.227 (2.5); 7.212 (1.6); 7.208 (1.5); 7.151 (2.0); 7.148 (2.1); 7.140 (4.3); 7.128 (6.1); 7.113 (1.3); 7.110 (1.2); 5.466 (4.3); 5.430 (4.7); 4.858 (4.7); 4.593 (4.7); 4.557 (4.3); 3.729 (2.8); 3.694 (4.8); 3.600 (5.4); 3.564 (3.1); 3.430 (0.5); 3.413 (0.5); 2.894 (0.8); 2.778 (0.7); 2.590 (1.2); 2.560 (0.4); 2.492 (1.2); 2.487 (1.4); 2.482 (1.2); 2.245 (6.8); 2.122 (1.9); 2.115 (2.1); 2.109 (2.1); 2.103 (1.8); 2.097 (1.5); 2.088 (1.3); 1.966 (8.0); 1.960 (13.4); 1.954 (53.7); 1.948 (93.7); 1.942 (121.8); 1.936 (84.4); 1.930 (43.5); 1.782 (0.4); 1.776 (0.7); 1.770 (0.8); 1.764 (0.6); 1.758 (0.4); 1.270 (1.4); 1.141 (0.6); 1.124 (1.1); 1.106 (0.6); 0.882 (0.4); 0.858 (0.4); 0.007 (2.0); 0.000 (42.5); -0.008 (1.8) |
| Example I-13: ¹H-NMR (400.0 MHz, d₆-DMSO): |
| δ= 8.297 (8.0); 8.293 (8.1); 8.242 (15.9); 8.185 (5.0); 8.183 (5.0); 8.173 (5.1); 8.171 (5.2); 7.866 (16.0); 7.373 (0.6); 7.365 (5.0); 7.360 (2.2); 7.352 (5.7); 7.343 (6.0); 7.335 (2.5); 7.329 (5.4); 7.322 (0.7); 7.178 (3.2); 7.166 (3.9); 7.163 (4.0); 7.150 (3.1); 7.059 (0.7); 7.052 (6.1); 7.047 (2.0); 7.029 (11.1); 7.012 (1.9); 7.007 (5.3); 6.999 (0.5); 5.861 (15.0); 4.713 (1.0); 4.677 (10.2); 4.672 (9.8); 4.636 (0.9); 3.360 (4.8); 3.350 (37.4); 3.326 (4.9); 3.095 (4.7); 3.060 (3.7); 2.715 (0.4); 2.546 (90.8); 2.529 (0.4); 2.524 (0.4); 2.515 (5.2); 2.511 (10.9); 2.506 (14.5); 2.501 (10.4); 2.497 (5.0); 2.371 (0.4); 0.000 (4.7) |
| Example I-14: ¹H-NMR (400.0 MHz, d₆-DMSO): |
| δ= 8.804 (0.5); 8.316 (0.4); 8.169 (5.9); 8.133 (16.0); 7.367 (3.7); 7.350 (5.7); 7.325 (0.4); 7.289 (2.3); 7.272 (5.1); 7.253 (3.3); 7.234 (2.4); 7.216 (2.4); 7.199 (0.7); 6.916 (4.3); 5.965 (6.8); 4.605 (2.1); 4.569 (3.6); 4.487 (3.6); 4.451 (2.1); 3.444 (2.5); 3.409 (3.2); 3.383 (0.4); 3.331 (301.1); 3.182 (3.2); 3.147 (2.5); 2.671 (1.2); 2.542 (69.3); 2.506 (151.6); 2.502 (190.0); 2.498 (140.1); 2.435 (0.3); 2.368 (0.4); 2.329 (1.2); 1.236 (0.8); 0.000 (40.0) |
| Example I-15: ¹H-NMR (400.0 MHz, d₆-DMSO): |
| δ= 9.235 (1.0); 8.576 (1.4); 8.315 (0.8); 8.277 (4.6); 8.172 (1.2); 8.141 (14.4); 7.905 (5.3); 7.392 (5.8); 7.378 (6.6); 7.370 (7.2); 7.361 (3.2); 7.356 (6.4); 7.261 (0.5); 7.240 (0.9); 7.226 (0.7); 7.218 (0.3); 7.197 (1.0); 7.190 (1.4); 7.175 (1.0); 7.153 (0.4); 7.080 (6.8); 7.058 (12.6); 7.036 (6.4); 7.028 (9.5); 7.025 (9.1); 6.872 (0.7); 5.961 (16.0); 5.577 (0.6); 4.739 (2.2); 4.703 (6.0); 4.669 (6.1); 4.634 (2.2); 4.368 (0.6); 4.364 (0.6); 4.355 (0.6); 4.100 (0.4); 4.087 (0.4); 3.902 (8.8); 3.485 (4.9); 3.449 (6.1); 3.328 (327.4); 3.267 (0.7); 3.242 (0.4); 3.221 (6.3); 3.185 (5.0); 3.176 (2.5); 3.163 (2.1); 2.995 (0.8); 2.676 (1.6); 2.672 (2.2); 2.667 (1.6); 2.542 (14.5); 2.525 (6.0); 2.511 (138.9); 2.507 (288.6); 2.503 (402.7); 2.498 (306.0); 2.433 (0.4); 2.334 (1.7); 2.329 (2.4); 2.325 (1.7); 2.295 (0.4); 1.909 (0.4); 1.235 (0.3); 0.146 (0.4); 0.008 (2.7); 0.000 (85.2); -0.008 (3.4); -0.150 (0.4) |
| Example I-16: ¹H-NMR (400.0 MHz, CD3CN): |
| δ= 8.183 (4.8); 8.178 (4.9); 8.171 (5.0); 8.167 (4.8); 8.075 (14.6); 7.775 (14.5); 7.592 (5.2); 7.587 (6.2); 7.583 (5.9); 7.573 (6.2); 7.568 (8.2); 7.561 (16.0); 7.539 (15.3); 7.518 (5.3); 7.163 (5.5); 7.151 (5.4); 7.144 (5.1); 7.132 (4.9); 4.820 (7.8); 4.784 (10.8); 4.681 (12.4); 4.613 (10.5); 4.577 (7.5); 3.412 (4.4); 3.375 (11.6); 3.334 (11.7); 3.298 (4.5); 2.889 (2.9); 2.772 (2.5); 2.473 (0.7); 2.469 (1.5); 2.464 (2.1); 2.459 (1.4); 2.454 (0.7); 2.160 (614.5); 2.127 (1.6); 2.120 (2.0); 2.114 (2.8); 2.108 (3.5); 2.101 (2.5); 2.095 (1.4); 1.964 (31.6); 1.958 (52.4); 1.953 (234.4); 1.946 (413.7); 1.940 (542.3); 1.934 (373.5); 1.928 (193.3); 1.781 (1.3); 1.775 (2.3); 1.769 (3.2); 1.762 (2.2); 1.756 (1.1); 1.286 (0.4); 1.204 (0.6); 0.000 (1.7) |
| Example I-17: ¹H-NMR (400.0 MHz, CD3CN): |
| δ= 8.289 (0.4); 8.182 (2.9); 8.177 (3.1); 8.170 (3.1); 8.165 (3.2); 8.072 (9.6); 8.058 (0.3); 7.925 (1.7); 7.788 (9.2); 7.591 (2.9); 7.586 (3.0); 7.571 (3.2); 7.567 (3.3); 7.356 (4.9); 7.353 (6.0); 7.290 (0.8); 7.286 (0.6); 7.280 (1.3); 7.276 (2.2); 7.271 (1.9); 7.266 (4.4); 7.263 (4.4); 7.257 (1.4); 7.252 (1.6); 7.246 (7.0); 7.243 (5.2); 7.238 (4.0); 7.233 (4.9); 7.230 (4.8); 7.220 (0.6); 7.166 (3.4); 7.154 (3.3); 7.147 (3.3); 7.135 (3.1); 4.770 (4.9); 4.735 (7.1); 4.604 (7.6); 4.578 (7.2); 4.542 (5.0); 3.381 (3.3); 3.345 (7.4); 3.290 (7.5); 3.254 (3.4); 2.889 (16.0); 2.771 (14.3); 2.469 (0.4); 2.464 (0.6); 2.460 (0.5); 2.161 (134.4); 2.120 (0.5); 2.114 (0.7); 2.107 (0.9); 2.101 (0.6); 2.095 (0.4); 1.964 (8.4); 1.958 (12.8); 1.952 (59.7); 1.946 (106.3); 1.940 (139.7); 1.934 (95.9); 1.928 (49.6); 1.781 (0.4); 1.775 (0.6); 1.768 (0.8); 1.762 (0.6); 1.756 (0.4); 1.712 (0.4); 1.483 (0.3); 1.434 (0.7); 0.983 (2.3); 0.146 (0.4); 0.008 (3.0); 0.000 (92.3); -0.009 (3.2); -0.150 (0.4) |
| Example I-18: ¹H-NMR (400.0 MHz, d₆-DMSO): |
| δ= 8.410 (9.7); 8.272 (6.3); 8.259 (16.0); 7.885 (10.8); 7.377 (0.5); 7.369 (3.6); 7.364 (1.6); 7.356 (4.1); 7.347 (4.4); 7.339 (1.8); 7.333 (4.0); 7.326 (0.5); 7.255 (4.7); 7.242 (4.6); 7.067 (0.5); 7.059 (4.2); 7.054 (1.3); 7.037 (7.8); 7.020 (1.3); 7.015 (3.7); 7.007 (0.4); 5.882 (9.8); 4.711 (0.6); 4.676 (7.4); 4.671 (7.3); 4.635 (0.6); 3.449 (3.1); 3.414 (3.9); 3.333 (25.9); 3.201 (3.9); 3.165 (3.1); 2.543 (34.5); 2.526 (0.4); 2.513 (8.2); 2.508 (16.4); 2.504 (21.5); 2.499 (15.4); 2.495 (7.3); 0.000 (1.1) |
| Example I-19: ¹H-NMR (400.0 MHz, d₆-DMSO): |
| δ= 8.508 (0.7); 8.425 (11.2); 8.277 (7.0); 8.266 (16.0); 8.092 (0.5); 8.070 (0.5); 8.020 (0.6); 7.913 (12.7); 7.486 (1.5); 7.478 (1.5); 7.463 (1.7); 7.457 (2.5); 7.451 (1.5); 7.436 (1.4); 7.428 (1.4); 7.340 (1.1); 7.333 (8.1); 7.328 (2.7); 7.316 (3.1); 7.311 (8.9); 7.303 (1.0); 7.264 (5.2); 7.251 (5.0); 7.221 (1.2); 7.207 (1.2); 7.199 (2.7); 7.185 (2.7); 7.176 (2.1); 7.162 (2.0); 7.151 (1.6); 7.148 (1.5); 7.143 (1.4); 7.140 (1.3); 7.130 (1.7); 7.127 (2.2); 7.124 (2.2); 7.120 (1.9); 7.108 (0.9); 7.104 (1.1); 7.101 (0.9); 7.097 (0.7); 6.847 (1.2); 6.840 (8.7); 6.818 (8.0); 6.810 (0.8); 5.956 (1.0); 5.837 (10.6); 4.703 (1.7); 4.668 (5.4); 4.646 (5.4); 4.610 (1.7); 3.421 (3.1); 3.386 (3.9); 3.350 (17.0); 3.196 (3.9); 3.161 (3.0); 2.547 (48.4); 2.517 (4.4); 2.512 (8.9); 2.508 (11.7); 2.503 (8.3); 2.499 (3.9); 0.000 (0.6) |
| Example I-20: ¹H-NMR (400.0 MHz, d₆-DMSO): |
| δ= 8.507 (2.3); 8.308 (7.6); 8.304 (7.8); 8.241 (15.5); 8.187 (4.9); 8.186 (4.9); 8.175 (5.0); 8.173 (4.9); 8.090 (1.8); 8.085 (0.6); 8.073 (0.6); 8.068 (1.9); 8.020 (2.2); 7.891 (16.0); 7.559 (0.4); 7.500 (0.4); 7.487 (1.9); 7.480 (1.9); 7.465 (2.2); 7.459 (3.2); 7.453 (2.1); 7.438 (1.8); 7.431 (1.6); 7.321 (9.6); 7.316 (3.5); 7.304 (3.6); 7.299 (10.5); 7.292 (1.3); 7.224 (0.4); 7.222 (0.4); 7.217 (0.4); 7.214 (0.4); 7.209 (1.2); 7.201 (0.4); 7.195 (1.6); 7.186 (4.6); 7.172 (6.7); 7.165 (4.1); 7.156 (3.8); 7.152 (4.1); 7.150 (4.9); 7.146 (2.5); 7.143 (2.1); 7.133 (2.5); 7.130 (2.9); 7.125 (3.7); 7.107 (1.4); 7.103 (2.3); 6.835 (1.3); 6.828 (10.7); 6.805 (9.9); 6.798 (1.0); 5.954 (3.5); 5.811 (14.1); 4.651 (14.5); 3.357 (91.9); 3.326 (3.6); 3.291 (4.3); 3.084 (4.3); 3.049 (3.3); 3.002 (0.6); 2.716 (0.6); 2.546 (126.6); 2.530 (0.5); 2.516 (7.6); 2.512 (15.6); 2.507 (20.7); 2.502 (14.9); 2.498 (7.1); 2.373 (0.6); 2.372 (0.6); 0.000 (4.7) |
| Example I-21: ¹H-NMR (400.0 MHz, CD3CN): |
| δ= 12.004 (0.3); 11.991 (0.3); 8.420 (16.0); 8.330 (0.4); 8.266 (0.5); 8.244 (10.3); 8.231 (10.4); 8.186 (0.5); 8.113 (0.4); 8.067 (15.9); 8.016 (0.5); 8.004 (0.5); 7.977 (0.4); 7.929 (1.2); 7.800 (15.0); 7.368 (8.1); 7.366 (9.0); 7.362 (4.2); 7.298 (1.6); 7.294 (1.4); 7.288 (2.6); 7.285 (4.0); 7.279 (3.3); 7.275 (5.7); 7.270 (6.9); 7.265 (2.1); 7.260 (1.9); 7.251 (10.6); 7.250 (10.5); 7.248 (8.7); 7.243 (6.8); 7.238 (8.9); 7.235 (8.2); 7.229 (0.9); 7.225 (0.8); 7.165 (8.7); 7.152 (8.4); 5.448 (9.4); 4.746 (7.7); 4.710 (11.9); 4.613 (15.8); 4.590 (12.0); 4.554 (7.7); 3.409 (6.1); 3.373 (11.2); 3.295 (11.7); 3.260 (6.3); 2.890 (10.7); 2.774 (9.2); 2.171 (2.9); 1.965 (2.7); 1.959 (4.4); 1.953 (20.4); 1.947 (36.0); 1.940 (47.1); 1.934 (32.2); 1.928 (16.5); 0.008 (1.0); 0.000 (29.2); -0.009 (0.9) |
| Example I-22: ¹H-NMR (400.0 MHz, CD3CN): |
| δ= 8.425 (8.3); 8.238 (5.5); 8.226 (5.6); 8.051 (8.5); 7.806 (8.3); 7.357 (0.7); 7.352 (3.7); 7.346 (1.8); 7.339 (1.0); 7.333 (5.8); 7.330 (5.7); 7.317 (2.2); 7.311 (6.7); 7.298 (0.5); 7.292 (4.3); 7.290 (4.3); 7.272 (2.5); 7.271 (2.8); 7.188 (2.5); 7.185 (3.5); 7.184 (3.4); 7.182 (2.9); 7.169 (1.9); 7.166 (2.3); 7.164 (2.5); 7.162 (2.1); 7.143 (4.6); 7.129 (4.7); 7.124 (3.0); 7.122 (1.8); 7.109 (1.7); 7.106 (3.9); 7.090 (1.1); 7.087 (1.8); 7.085 (1.2); 6.889 (2.3); 6.885 (5.6); 6.883 (5.4); 6.881 (7.8); 6.878 (6.9); 6.861 (2.5); 6.859 (2.6); 6.855 (1.9); 6.853 (1.7); 6.801 (5.2); 6.798 (6.6); 6.793 (2.2); 6.781 (3.3); 6.779 (5.7); 6.776 (5.3); 6.770 (0.8); 4.739 (3.8); 4.703 (6.1); 4.594 (6.0); 4.558 (5.6); 4.551 (5.6); 4.085 (2.1); 4.068 (6.3); 4.050 (6.4); 4.032 (2.1); 3.396 (3.1); 3.361 (5.4); 3.275 (5.8); 3.240 (3.3); 2.888 (1.7); 2.772 (1.5); 2.770 (1.5); 2.181 (30.8); 1.972 (28.5); 1.965 (1.9); 1.958 (2.8); 1.952 (13.4); 1.946 (23.6); 1.940 (31.3); 1.934 (21.5); 1.928 (11.1); 1.436 (2.1); 1.221 (8.2); 1.203 (16.0); 1.185 (8.0); 0.146 (0.4); 0.017 (0.4); 0.016 (0.4); 0.008 (4.3); 0.000 (95.6); -0.009 (4.6); -0.150 (0.4) |
| Example I-23: ¹H-NMR (400.0 MHz, d₆-DMSO): |
| δ= 8.252 (12.7); 8.029 (6.5); 8.017 (6.6); 7.874 (12.5); 7.383 (4.3); 7.369 (5.1); 7.361 (5.5); 7.347 (4.8); 7.157 (3.2); 7.144 (5.9); 7.132 (3.0); 7.079 (4.8); 7.057 (8.8); 7.035 (4.2); 5.922 (10.7); 4.691 (16.0); 3.399 (3.9); 3.369 (39.9); 3.163 (4.5); 3.129 (3.4); 2.719 (0.5); 2.550 (88.1); 2.514 (8.5); 2.510 (11.1); 2.375 (0.5); 0.000 (2.4) |
| Example I-24: ¹H-NMR (400.0 MHz, d₆-DMSO): |
| δ= 8.516 (6.0); 8.221 (13.3); 8.195 (0.3); 8.180 (6.7); 8.176 (2.2); 8.170 (2.4); 8.166 (6.8); 8.108 (0.6); 8.101 (5.1); 8.096 (1.5); 8.084 (1.7); 8.079 (5.4); 8.071 (0.6); 8.027 (6.3); 7.910 (14.7); 7.519 (0.4); 7.514 (2.3); 7.509 (0.9); 7.495 (3.7); 7.493 (3.5); 7.487 (0.6); 7.479 (1.2); 7.474 (3.2); 7.468 (0.5); 7.409 (1.1); 7.403 (5.4); 7.398 (2.1); 7.391 (1.5); 7.385 (8.0); 7.382 (7.7); 7.376 (1.2); 7.368 (2.7); 7.363 (7.3); 7.354 (9.6); 7.349 (3.2); 7.337 (3.5); 7.332 (10.6); 7.325 (1.2); 7.305 (0.9); 7.302 (1.5); 7.300 (0.9); 7.284 (2.4); 7.268 (0.7); 7.265 (1.1); 7.262 (0.6); 7.199 (0.4); 7.178 (3.4); 7.176 (4.1); 7.170 (1.1); 7.159 (2.2); 7.157 (3.6); 7.154 (3.1); 7.145 (2.1); 7.143 (3.3); 7.141 (2.1); 7.136 (1.1); 7.128 (7.4); 7.125 (6.6); 7.111 (2.1); 7.106 (7.6); 7.099 (0.7); 7.076 (3.6); 7.073 (5.7); 7.066 (7.8); 7.063 (16.0); 7.051 (0.5); 7.039 (0.4); 6.951 (0.7); 6.942 (7.3); 6.939 (8.9); 6.934 (2.5); 6.923 (5.7); 6.920 (9.4); 6.917 (13.6); 6.911 (4.1); 6.899 (3.4); 6.894 (10.2); 6.887 (1.0); 5.954 (10.4); 5.831 (13.8); 4.637 (1.9); 4.602 (6.6); 4.583 (6.5); 4.547 (1.8); 3.348 (24.3); 3.261 (3.2); 3.228 (4.7); 3.121 (4.7); 3.087 (3.2); 3.006 (1.5); 3.002 (0.3); 2.719 (0.6); 2.570 (0.4); 2.550 (126.2); 2.532 (0.6); 2.519 (9.3); 2.514 (18.9); 2.510 (24.7); 2.505 (17.4); 2.501 (8.1); 2.375 (0.5); 2.082 (0.7); 1.467 (0.4) |
| Example I-25: ¹H-NMR (400.0 MHz, d₆-DMSO): |
| δ= 8.507 (9.8); 8.435 (1.5); 8.193 (9.6); 8.172 (2.8); 8.160 (7.0); 8.147 (4.5); 8.100 (14.6); 8.092 (8.3); 8.087 (2.6); 8.075 (3.1); 8.070 (8.6); 8.063 (0.9); 8.053 (0.6); 8.022 (10.2); 7.895 (10.4); 7.597 (1.3); 7.590 (1.4); 7.575 (1.5); 7.569 (2.1); 7.562 (1.5); 7.547 (1.4); 7.540 (1.4); 7.506 (2.0); 7.495 (1.8); 7.493 (1.8); 7.483 (3.7); 7.474 (3.0); 7.469 (4.1); 7.460 (1.8); 7.452 (1.7); 7.446 (2.8); 7.328 (7.1); 7.322 (5.4); 7.316 (2.0); 7.311 (2.8); 7.306 (8.4); 7.299 (5.3); 7.255 (1.0); 7.251 (1.1); 7.248 (1.0); 7.244 (1.0); 7.234 (1.9); 7.231 (1.8); 7.228 (1.8); 7.223 (1.6); 7.220 (1.8); 7.212 (2.3); 7.208 (1.4); 7.204 (1.9); 7.196 (3.1); 7.189 (1.3); 7.181 (2.4); 7.174 (2.6); 7.159 (1.9); 7.154 (2.3); 7.151 (2.1); 7.147 (2.1); 7.144 (2.2); 7.134 (3.1); 7.129 (9.7); 7.124 (4.9); 7.111 (3.3); 7.107 (7.8); 7.090 (5.7); 7.063 (3.6); 7.044 (3.2); 7.041 (3.0); 7.032 (3.1); 7.029 (2.9); 7.017 (2.0); 7.014 (1.9); 7.004 (1.9); 7.001 (1.8); 6.878 (4.5); 6.863 (7.9); 6.856 (5.4); 6.841 (6.7); 6.833 (0.7); 5.955 (16.0); 5.878 (5.8); 5.823 (9.5); 5.805 (1.0); 4.607 (1.5); 4.572 (4.4); 4.554 (2.1); 4.546 (4.5); 4.519 (2.2); 4.511 (1.7); 4.364 (2.1); 4.328 (1.5); 3.339 (99.6); 3.243 (2.5); 3.237 (1.8); 3.209 (3.7); 3.113 (3.3); 3.079 (2.2); 3.062 (1.9); 3.029 (1.4); 3.004 (2.1); 2.719 (0.8); 2.684 (0.4); 2.679 (0.5); 2.674 (0.4); 2.571 (0.4); 2.568 (0.7); 2.549 (216.2); 2.538 (1.1); 2.532 (1.5); 2.519 (30.5); 2.514 (60.8); 2.510 (79.1); 2.505 (55.8); 2.501 (25.9); 2.375 (0.8); 2.341 (0.4); 2.337 (0.5); 2.332 (0.4); 2.083 (0.7); 1.242 (0.3) |
| Example I-26: ¹H-NMR (400.0 MHz, d₆-DMSO): |
| δ= 8.813 (0.5); 8.725 (0.3); 8.651 (0.8); 8.535 (9.3); 8.353 (3.6); 8.349 (3.5); 8.295 (0.7); 8.272 (10.2); 8.198 (6.7); 8.153 (6.7); 8.132 (8.0); 8.100 (1.0); 8.085 (0.4); 8.063 (0.4); 8.038 (9.2); 7.963 (0.5); 7.941 (0.7); 7.926 (8.2); 7.903 (14.9); 7.874 (0.4); 7.852 (0.7); 7.843 (0.4); 7.828 (0.7); 7.822 (0.6); 7.809 (4.6); 7.805 (5.9); 7.787 (6.3); 7.762 (1.0); 7.751 (0.8); 7.744 (0.9); 7.707 (5.9); 7.701 (4.9); 7.686 (7.6); 7.682 (5.7); 7.669 (5.3); 7.666 (6.4); 7.648 (7.3); 7.629 (1.0); 7.607 (1.2); 7.593 (6.6); 7.572 (8.6); 7.551 (3.1); 7.533 (6.9); 7.513 (4.9); 7.494 (2.9); 7.476 (7.9); 7.468 (5.3); 7.457 (8.1); 7.447 (13.7); 7.429 (7.8); 7.426 (7.7); 7.400 (2.2); 7.381 (2.8); 7.369 (3.2); 7.350 (3.9); 7.332 (1.6); 7.295 (5.0); 7.275 (4.6); 7.254 (4.4); 7.242 (4.3); 7.077 (0.7); 7.057 (0.6); 6.963 (0.4); 6.950 (0.4); 6.823 (2.6); 6.810 (2.6); 6.723 (2.8); 6.031 (16.0); 5.915 (9.4); 5.589 (1.3); 5.575 (2.9); 5.562 (1.3); 5.513 (0.3); 4.729 (0.6); 4.693 (7.0); 4.689 (6.9); 4.653 (0.6); 4.369 (2.8); 4.364 (3.0); 4.355 (2.9); 4.351 (2.7); 3.433 (0.5); 3.422 (0.4); 3.393 (3.3); 3.342 (830.2); 3.299 (1.7); 3.290 (1.5); 3.280 (1.1); 3.268 (0.9); 3.257 (0.6); 3.247 (0.5); 3.239 (0.5); 3.229 (0.5); 3.219 (0.3); 3.194 (0.3); 3.127 (3.3); 3.092 (2.6); 3.003 (0.5); 2.996 (2.2); 2.712 (0.8); 2.676 (0.9); 2.672 (1.1); 2.668 (0.8); 2.617 (0.5); 2.542 (197.6); 2.508 (148.4); 2.503 (187.9); 2.499 (137.1); 2.437 (0.6); 2.410 (0.3); 2.392 (0.3); 2.368 (1.0); 2.335 (1.1); 2.330 (1.3); 2.075 (0.5); 1.234 (0.5); 0.000 (3.6) |
| Example I-27: ¹H-NMR (400.0 MHz, d₆-DMSO): |
| δ= 9.209 (1.5); 8.815 (0.5); 8.675 (2.5); 8.584 (1.9); 8.575 (0.4); 8.315 (1.2); 8.300 (2.2); 8.240 (5.3); 8.155 (7.1); 8.138 (3.1); 8.076 (1.6); 7.932 (2.4); 7.797 (0.5); 7.772 (0.5); 7.745 (0.5); 7.725 (0.4); 7.667 (7.5); 7.648 (10.7); 7.630 (3.4); 7.619 (4.8); 7.600 (8.8); 7.580 (6.3); 7.500 (0.5); 7.485 (2.3); 7.467 (13.1); 7.447 (14.0); 7.428 (4.3); 7.390 (1.7); 7.371 (3.8); 7.350 (3.3); 7.334 (1.0); 7.295 (1.6); 7.274 (3.1); 7.253 (0.5); 7.044 (4.9); 7.026 (4.4); 7.005 (3.9); 6.900 (1.2); 6.646 (3.4); 5.965 (6.2); 5.617 (0.5); 5.603 (0.8); 5.591 (0.4); 5.559 (1.1); 5.547 (2.3); 5.535 (1.1); 4.750 (1.0); 4.714 (2.7); 4.681 (2.8); 4.647 (1.0); 4.426 (1.1); 4.412 (1.0); 4.252 (0.6); 4.241 (0.7); 4.223 (1.4); 4.212 (1.2); 4.171 (1.3); 4.158 (1.3); 4.148 (0.4); 4.142 (0.8); 4.129 (0.7); 4.001 (1.1); 3.968 (2.4); 3.916 (2.6); 3.902 (16.0); 3.882 (1.3); 3.534 (2.1); 3.498 (2.8); 3.329 (381.8); 3.271 (3.6); 3.235 (2.5); 3.176 (1.3); 3.164 (1.2); 2.995 (0.4); 2.672 (2.5); 2.542 (24.3); 2.503 (465.1); 2.368 (0.4); 2.330 (2.7); 1.238 (0.5); 0.002 (50.9); 0.000 (61.7); -0.149 (0.4) |
| Example I-28: ¹H-NMR (400.0 MHz, d₆-DMSO): |
| δ= 8.316 (0.7); 8.185 (5.7); 8.159 (16.0); 7.374 (3.1); 7.353 (7.8); 7.326 (8.2); 7.304 (3.2); 6.990 (3.8); 6.986 (3.7); 6.096 (6.6); 4.646 (2.1); 4.611 (3.1); 4.486 (3.0); 4.451 (2.0); 3.451 (2.3); 3.416 (3.0); 3.395 (0.7); 3.332 (682.4); 3.290 (0.6); 3.278 (0.7); 3.176 (2.8); 3.141 (2.2); 2.995 (0.7); 2.671 (1.8); 2.667 (1.3); 2.565 (0.6); 2.542 (53.0); 2.507 (216.5); 2.502 (276.6); 2.498 (201.2); 2.455 (0.5); 2.333 (1.3); 2.329 (1.7); 2.324 (1.2); 1.258 (0.4); 1.235 (1.7); 0.000 (47.2) |
| Example I-29: ¹H-NMR (400.0 MHz, CD3CN): |
| δ= 8.196 (5.4); 8.191 (5.8); 8.184 (5.6); 8.179 (5.5); 8.114 (16.0); 7.835 (0.4); 7.726 (16.0); 7.625 (5.2); 7.621 (5.2); 7.606 (5.7); 7.602 (5.5); 7.209 (5.6); 7.203 (5.7); 7.192 (6.1); 7.180 (9.2); 7.174 (11.0); 7.162 (5.4); 7.145 (5.2); 7.124 (11.1); 7.102 (6.9); 7.002 (6.1); 6.997 (5.8); 6.981 (4.4); 6.976 (4.2); 5.448 (5.8); 5.014 (7.3); 4.978 (8.6); 4.711 (12.3); 4.563 (8.6); 4.527 (7.4); 4.068 (0.6); 4.050 (0.5); 3.463 (4.4); 3.427 (10.8); 3.382 (12.8); 3.346 (5.2); 3.043 (0.6); 2.818 (0.6); 2.469 (0.5); 2.465 (0.7); 2.460 (0.5); 2.160 (102.2); 2.132 (0.4); 2.126 (0.4); 2.120 (0.5); 2.114 (0.8); 2.107 (1.0); 2.101 (0.7); 2.095 (0.4); 1.972 (2.6); 1.964 (10.0); 1.958 (15.3); 1.952 (69.0); 1.946 (120.8); 1.940 (159.1); 1.934 (108.6); 1.928 (55.6); 1.781 (0.4); 1.775 (0.7); 1.768 (0.9); 1.762 (0.6); 1.756 (0.3); 1.371 (0.7); 1.340 (0.6); 1.322 (0.4); 1.285 (1.0); 1.276 (2.0); 1.270 (1.7); 1.221 (0.6); 1.204 (1.1); 1.186 (0.7); 0.983 (0.3); 0.882 (0.5); 0.146 (0.5); 0.008 (3.7); 0.000 (112.6); -0.009 (3.8); -0.150 (0.5) |
| Example I-30: ¹H-NMR (400.0 MHz, d₆-DMSO): |
| δ= 8.446 (10.0); 8.316 (16.0); 8.304 (6.5); 7.784 (11.3); 7.301 (4.9); 7.289 (4.8); 7.206 (1.3); 7.200 (1.3); 7.183 (1.4); 7.176 (2.8); 7.169 (1.5); 7.157 (1.6); 7.152 (3.2); 7.147 (1.6); 7.134 (2.2); 7.129 (1.4); 7.112 (1.2); 6.874 (1.2); 6.868 (1.2); 6.853 (2.2); 6.847 (2.0); 6.832 (1.1); 6.826 (1.0); 6.149 (11.0); 4.828 (3.2); 4.792 (4.2); 4.618 (3.9); 4.583 (2.8); 3.493 (2.7); 3.458 (3.5); 3.328 (74.6); 3.271 (4.0); 3.236 (3.0); 2.672 (0.3); 2.542 (38.0); 2.525 (0.9); 2.511 (20.2); 2.507 (40.2); 2.503 (52.4); 2.498 (37.1); 2.493 (17.3); 2.329 (0.3); 0.000 (2.4) |
| Example I-31: ¹H-NMR (400.0 MHz, d₆-DMSO): |
| δ= 8.510 (1.1); 8.308 (7.9); 8.304 (7.8); 8.248 (15.9); 8.191 (5.0); 8.189 (4.8); 8.179 (5.1); 8.177 (4.9); 8.080 (0.9); 8.075 (0.3); 8.058 (1.0); 8.029 (1.0); 7.858 (16.0); 7.696 (1.0); 7.675 (0.9); 7.347 (7.7); 7.343 (2.9); 7.331 (4.1); 7.326 (14.0); 7.320 (2.1); 7.280 (2.4); 7.275 (15.6); 7.270 (3.8); 7.258 (3.0); 7.253 (8.4); 7.247 (0.9); 7.189 (3.1); 7.176 (3.8); 7.173 (3.8); 7.161 (3.0); 5.998 (1.9); 5.903 (15.0); 4.721 (2.4); 4.686 (7.5); 4.661 (7.4); 4.625 (2.4); 3.373 (4.0); 3.341 (40.4); 3.102 (4.5); 3.067 (3.6); 3.000 (0.4); 2.714 (0.5); 2.544 (103.1); 2.527 (0.4); 2.514 (7.4); 2.509 (15.1); 2.505 (19.7); 2.500 (14.0); 2.495 (6.5); 2.370 (0.5); 0.008 (0.3); 0.000 (8.7) |
| Example I-32: ¹H-NMR (400.0 MHz, d₆-DMSO): |
| δ= 8.392 (0.7); 8.369 (0.3); 8.315 (0.4); 8.244 (13.2); 8.191 (8.8); 8.163 (0.4); 7.967 (0.6); 7.874 (13.0); 7.379 (4.3); 7.365 (5.5); 7.357 (6.0); 7.344 (5.1); 7.303 (0.4); 7.242 (5.9); 7.229 (5.7); 7.080 (5.1); 7.058 (9.4); 7.035 (4.5); 6.976 (0.3); 6.856 (0.4); 5.912 (13.1); 4.705 (0.4); 4.668 (16.0); 4.633 (0.4); 3.450 (0.5); 3.437 (0.6); 3.413 (0.8); 3.395 (1.4); 3.333 (1364.6); 3.285 (2.4); 3.267 (1.1); 3.257 (1.2); 3.246 (1.0); 3.225 (0.5); 3.210 (0.5); 3.204 (0.5); 3.194 (0.5); 3.138 (0.4); 3.083 (4.5); 3.049 (3.6); 2.995 (1.1); 2.712 (0.6); 2.671 (2.8); 2.667 (2.0); 2.541 (105.6); 2.506 (336.3); 2.502 (419.6); 2.498 (309.4); 2.367 (0.8); 2.329 (2.7); 1.259 (0.4); 1.234 (0.9); 0.000 (15.0) |
| Example I-33: ¹H-NMR (400.0 MHz, CD3CN): |
| δ= 8.441 (5.8); 8.340 (0.3); 8.319 (0.3); 8.277 (0.4); 8.266 (0.4); 8.235 (3.8); 8.223 (3.9); 8.208 (0.4); 8.195 (0.4); 8.158 (0.4); 8.130 (0.3); 8.111 (0.3); 8.095 (0.4); 8.083 (0.4); 8.054 (5.6); 8.039 (0.5); 8.007 (0.3); 7.996 (0.3); 7.960 (1.7); 7.822 (5.2); 7.399 (2.4); 7.395 (3.2); 7.390 (1.1); 7.378 (4.6); 7.375 (4.0); 7.369 (0.5); 7.321 (1.3); 7.316 (1.7); 7.311 (0.8); 7.300 (4.3); 7.296 (1.7); 7.281 (3.1); 7.274 (1.7); 7.270 (2.3); 7.267 (1.2); 7.260 (0.7); 7.253 (1.8); 7.244 (0.3); 7.238 (0.4); 7.235 (0.5); 7.163 (3.1); 7.151 (3.0); 4.761 (2.7); 4.725 (4.4); 4.610 (4.4); 4.595 (3.1); 4.574 (2.7); 3.432 (2.0); 3.396 (4.3); 3.342 (4.5); 3.306 (2.1); 2.920 (16.0); 2.805 (14.2); 2.250 (16.6); 2.002 (0.5); 1.995 (1.0); 1.988 (1.8); 1.983 (8.0); 1.976 (14.1); 1.970 (18.4); 1.964 (12.6); 1.958 (6.4) |
| Example I-34: ¹H-NMR (400.0 MHz, d₆-DMSO): |
| δ= 8.503 (9.2); 8.200 (2.8); 8.184 (7.8); 8.091 (7.2); 8.069 (7.8); 8.014 (9.0); 7.508 (3.1); 7.503 (1.5); 7.487 (5.8); 7.468 (4.5); 7.402 (1.2); 7.381 (2.2); 7.362 (4.0); 7.340 (2.7); 7.296 (2.1); 7.278 (3.4); 7.259 (1.4); 7.169 (6.2); 7.147 (5.7); 7.127 (2.3); 7.120 (7.8); 7.098 (7.6); 7.091 (1.2); 6.940 (4.0); 6.919 (4.4); 6.897 (2.6); 5.968 (3.4); 5.942 (16.0); 4.628 (0.9); 4.593 (1.4); 4.508 (1.4); 4.472 (0.9); 3.425 (1.0); 3.390 (1.4); 3.374 (0.4); 3.332 (222.2); 3.180 (1.3); 3.146 (1.0); 2.996 (0.4); 2.956 (0.5); 2.711 (0.4); 2.671 (0.7); 2.542 (81.3); 2.506 (80.9); 2.502 (107.3); 2.498 (81.1); 2.367 (0.4); 2.329 (0.7); 2.324 (0.5); 1.235 (0.4); 0.007 (1.1); 0.000 (24.6); -0.008 (1.2) |
| Example I-35: ¹H-NMR (400.0 MHz, CD3CN): |
| δ= 15.359 (0.5); 14.322 (0.5); 12.810 (0.6); 12.262 (0.6); 12.167 (1.1); 12.142 (1.1); 12.127 (1.2); 12.121 (1.0); 12.092 (1.0); 12.086 (1.0); 12.037 (1.0); 12.014 (1.0); 11.962 (1.6); 11.914 (1.0); 11.854 (0.9); 11.773 (1.1); 8.354 (0.9); 8.297 (6.1); 8.233 (1.0); 8.228 (0.9); 8.220 (0.9); 8.213 (0.7); 8.206 (0.9); 8.186 (5.0); 8.181 (4.8); 8.175 (4.7); 8.170 (4.6); 8.072 (13.8); 8.046 (0.6); 8.021 (0.7); 8.009 (0.8); 8.003 (0.8); 7.988 (0.9); 7.910 (5.8); 7.831 (0.6); 7.825 (0.6); 7.771 (12.7); 7.621 (10.3); 7.599 (14.7); 7.585 (0.6); 7.556 (4.5); 7.552 (4.1); 7.537 (5.0); 7.532 (4.6); 7.514 (16.0); 7.493 (9.7); 7.162 (4.5); 7.150 (4.8); 7.142 (4.3); 7.131 (4.0); 4.811 (6.4); 4.775 (9.3); 4.651 (14.6); 4.606 (9.1); 4.570 (6.4); 4.274 (0.5); 3.393 (3.4); 3.357 (10.5); 3.326 (10.8); 3.290 (3.3); 2.889 (1.2); 2.772 (0.9); 2.726 (0.6); 2.468 (0.9); 2.464 (1.1); 2.265 (0.5); 2.235 (0.6); 2.141 (465.3); 2.120 (3.5); 2.113 (4.8); 2.107 (5.8); 2.101 (3.8); 2.095 (2.1); 2.084 (0.7); 2.074 (0.6); 2.048 (0.7); 2.032 (0.7); 2.023 (0.9); 1.964 (63.8); 1.958 (116.2); 1.952 (405.2); 1.946 (687.0); 1.940 (860.7); 1.934 (587.0); 1.928 (296.9); 1.781 (2.2); 1.774 (3.9); 1.768 (4.9); 1.762 (3.4); 1.756 (1.6); 1.342 (0.6); 1.285 (0.8); 1.272 (1.0); 1.248 (0.5); 1.204 (0.9); 1.186 (0.6); 1.123 (0.6); 0.847 (0.6); 0.146 (2.0); 0.000 (454.6); - 0.008 (18.5); -0.149 (2.1); -0.312 (0.5) |
| Example I-36: ¹H-NMR (400.0 MHz, d₆-DMSO): |
| δ= 8.277 (7.4); 8.273 (7.2); 8.211 (14.6); 8.154 (4.6); 8.153 (4.6); 8.142 (4.7); 8.140 (4.6); 7.875 (14.6); 7.339 (4.6); 7.335 (6.1); 7.318 (8.2); 7.241 (2.3); 7.237 (3.3); 7.232 (1.4); 7.220 (8.4); 7.201 (5.9); 7.192 (2.6); 7.188 (4.3); 7.185 (2.5); 7.177 (1.4); 7.171 (4.0); 7.160 (3.2); 7.152 (1.6); 7.147 (3.8); 7.145 (3.7); 7.132 (2.9); 5.787 (15.1); 4.688 (0.3); 4.652 (16.0); 4.616 (0.3); 3.349 (4.1); 3.336 (39.0); 3.315 (4.7); 3.090 (4.6); 3.055 (3.6); 2.542 (56.3); 2.525 (0.4); 2.512 (8.5); 2.507 (17.1); 2.503 (22.2); 2.498 (15.7); 2.494 (7.3); 0.000 (7.4) |
| Example I-37: ¹H-NMR (400.0 MHz, d₆-DMSO): |
| δ= 8.161 (15.7); 8.128 (6.8); 8.115 (6.9); 7.882 (16.0); 7.354 (4.9); 7.350 (6.8); 7.345 (2.1); 7.333 (9.6); 7.278 (3.0); 7.275 (4.5); 7.270 (1.7); 7.257 (9.5); 7.253 (3.9); 7.238 (5.5); 7.217 (2.5); 7.214 (4.2); 7.211 (2.4); 7.202 (1.5); 7.197 (4.8); 7.190 (0.9); 7.182 (0.9); 7.179 (1.3); 7.176 (0.7); 7.109 (8.4); 7.017 (4.9); 7.014 (4.5); 7.005 (4.7); 7.001 (4.3); 5.809 (16.0); 4.610 (2.5); 4.575 (8.2); 4.551 (8.1); 4.516 (2.5); 3.341 (71.8); 3.273 (3.9); 3.239 (5.8); 3.127 (5.7); 3.093 (3.8); 3.004 (0.5); 2.719 (0.4); 2.549 (101.4); 2.537 (0.4); 2.532 (0.8); 2.519 (15.3); 2.514 (30.0); 2.510 (38.4); 2.505 (27.0); 2.501 (12.4); 2.375 (0.4); 2.082 (0.3) |
| Example I-38: ¹H-NMR (400.0 MHz, d₆-DMSO): |
| δ= 8.410 (1.7); 8.285 (1.9); 8.275 (1.0); 8.263 (1.0); 7.948 (1.2); 7.926 (1.4); 7.863 (1.9); 7.594 (1.3); 7.573 (1.2); 7.277 (0.8); 7.264 (0.8); 6.081 (1.6); 4.761 (0.8); 4.743 (0.8); 3.540 (0.5); 3.504 (0.6); 3.392 (0.3); 3.353 (90.5); 3.351 (89.6); 3.274 (0.6); 3.238 (0.5); 2.544 (20.0); 2.526 (0.4); 2.513 (8.0); 2.509 (16.1); 2.504 (21.2); 2.500 (15.5); 1.361 (16.0); 0.000 (1.4) |
| Example I-39: ¹H-NMR (400.0 MHz, d₆-DMSO): |
| δ= 8.547 (7.5); 8.220 (14.9); 8.163 (5.4); 8.155 (7.0); 8.141 (12.6); 8.049 (7.6); 7.932 (6.3); 7.927 (1.9); 7.914 (2.0); 7.910 (5.4); 7.901 (16.0); 7.814 (3.1); 7.811 (4.3); 7.806 (1.1); 7.793 (4.5); 7.785 (0.6); 7.734 (0.3); 7.713 (0.3); 7.676 (5.9); 7.673 (7.7); 7.668 (2.2); 7.655 (8.8); 7.600 (8.9); 7.579 (11.7); 7.558 (1.5); 7.556 (2.2); 7.551 (0.9); 7.538 (5.0); 7.534 (2.5); 7.519 (3.3); 7.484 (1.5); 7.481 (2.5); 7.478 (2.6); 7.473 (4.8); 7.469 (2.5); 7.463 (3.6); 7.455 (11.4); 7.450 (13.2); 7.445 (4.9); 7.435 (7.2); 7.428 (8.8); 7.377 (2.2); 7.374 (3.6); 7.371 (2.1); 7.360 (1.7); 7.355 (4.8); 7.351 (1.3); 7.340 (1.2); 7.337 (1.8); 7.334 (1.0); 7.177 (8.1); 7.082 (4.5); 7.079 (4.2); 7.069 (4.4); 7.066 (4.1); 6.042 (12.3); 5.882 (13.7); 4.657 (2.8); 4.621 (6.1); 4.578 (6.1); 4.542 (2.8); 3.347 (35.7); 3.316 (3.3); 3.282 (4.8); 3.173 (4.7); 3.139 (3.2); 3.006 (1.7); 2.719 (0.7); 2.569 (0.6); 2.549 (169.9); 2.532 (0.8); 2.519 (12.3); 2.514 (25.4); 2.510 (33.4); 2.505 (23.4); 2.501 (10.9); 2.375 (0.7); 2.082 (1.5) |
| Example I-40: ¹H-NMR (400.0 MHz, d₆-DMSO): |
| δ= 8.314 (0.7); 8.288 (0.5); 8.264 (3.5); 8.200 (10.8); 7.876 (4.0); 7.385 (0.4); 7.363 (7.2); 7.341 (14.2); 7.301 (16.0); 7.279 (8.2); 7.271 (7.3); 7.258 (6.7); 5.959 (13.8); 4.711 (0.6); 4.673 (9.1); 4.635 (0.6); 4.099 (0.5); 4.085 (0.5); 3.902 (11.8); 3.373 (4.6); 3.327 (198.3); 3.267 (0.6); 3.219 (0.4); 3.175 (2.7); 3.163 (2.6); 3.093 (4.6); 3.059 (3.7); 2.676 (1.3); 2.672 (1.7); 2.667 (1.3); 2.542 (3.1); 2.524 (4.9); 2.507 (223.0); 2.502 (309.8); 2.498 (236.5); 2.334 (1.3); 2.329 (1.7); 2.325 (1.3); 1.234 (0.4); 0.008 (2.0); 0.000 (59.1); -0.008 (2.3) |
| Example I-41: ¹H-NMR (300.2 MHz, d₆-DMSO): |
| δ= 8.510 (0.6); 8.343 (15.7); 8.213 (10.6); 8.043 (0.4); 7.818 (16.0); 7.257 (2.0); 7.249 (2.4); 7.217 (5.5); 7.191 (4.0); 7.186 (3.9); 7.162 (2.0); 7.121 (8.0); 7.115 (7.9); 6.938 (1.9); 6.929 (2.0); 6.910 (3.4); 6.902 (3.2); 6.882 (1.7); 6.874 (1.6); 6.246 (15.9); 5.842 (0.5); 5.832 (0.5); 4.852 (4.3); 4.805 (6.3); 4.654 (5.9); 4.606 (3.9); 4.088 (0.7); 4.064 (2.0); 4.040 (2.0); 4.016 (0.7); 3.551 (3.8); 3.504 (5.4); 3.409 (0.5); 3.342 (106.6); 3.287 (4.4); 2.530 (40.0); 2.524 (53.9); 2.518 (39.5); 2.513 (19.1); 2.012 (8.9); 1.932 (0.5); 1.270 (1.3); 1.221 (2.4); 1.198 (4.7); 1.174 (2.4); 0.896 (0.4); 0.881 (1.0); 0.860 (0.5); 0.034 (2.8); 0.023 (61.9); 0.012 (2.7) |
| Example I-42: ¹H-NMR (400.0 MHz, d₆-DMSO): |
| δ= 8.487 (13.2); 8.305 (16.0); 8.300 (9.0); 8.288 (8.6); 7.761 (15.2); 7.561 (8.8); 7.555 (8.9); 7.365 (6.5); 7.344 (9.3); 7.252 (6.0); 7.246 (5.9); 7.241 (6.7); 7.228 (7.6); 7.206 (0.4); 6.214 (13.5); 6.205 (0.3); 5.325 (0.6); 5.187 (5.0); 5.151 (5.7); 4.669 (5.3); 4.633 (4.9); 3.898 (0.3); 3.749 (4.5); 3.714 (5.4); 3.682 (0.3); 3.657 (0.3); 3.638 (0.3); 3.629 (0.4); 3.617 (0.5); 3.606 (0.4); 3.567 (0.4); 3.547 (0.5); 3.532 (0.5); 3.509 (0.5); 3.503 (0.5); 3.498 (0.6); 3.479 (0.6); 3.461 (5.9); 3.445 (1.0); 3.425 (5.1); 3.402 (1.9); 3.395 (2.3); 3.389 (2.3); 3.380 (4.4); 3.336 (2969.6); 3.299 (4.1); 3.287 (2.6); 3.259 (1.4); 3.233 (0.8); 3.222 (0.6); 3.210 (0.4); 3.197 (0.5); 3.186 (0.5); 3.150 (0.4); 3.092 (0.3); 2.995 (1.3); 2.711 (0.6); 2.676 (3.4); 2.671 (4.6); 2.667 (3.3); 2.662 (1.6); 2.541 (109.2); 2.525 (12.0); 2.511 (277.1); 2.507 (556.6); 2.502 (723.9); 2.498 (511.4); 2.493 (237.9); 2.464 (1.2); 2.429 (0.5); 2.424 (0.5); 2.367 (0.4); 2.333 (3.3); 2.329 (4.4); 2.324 (3.3); 2.320 (1.6); 2.282 (0.5); 2.026 (0.5); 2.008 (1.0); 1.989 (1.0); 1.977 (0.5); 1.958 (0.4); 1.495 (0.3); 1.475 (0.4); 1.454 (0.4); 1.437 (0.3); 1.313 (0.5); 1.298 (0.9); 1.235 (10.8); 1.209 (2.8); 1.193 (2.7); 0.870 (0.7); 0.854 (2.0); 0.837 (0.8); 0.008 (0.5); 0.000 (11.7) |
| Example I-43: ¹H-NMR (400.0 MHz, d₆-DMSO): |
| δ= 8.489 (0.3); 8.312 (16.0); 8.212 (3.5); 8.200 (3.6); 8.199 (3.5); 8.021 (0.4); 7.776 (11.1); 7.226 (1.3); 7.219 (1.3); 7.204 (3.2); 7.192 (3.5); 7.189 (4.1); 7.176 (2.5); 7.166 (1.3); 7.089 (1.0); 7.071 (1.3); 7.066 (2.1); 7.049 (2.1); 7.044 (1.4); 7.026 (1.1); 6.823 (1.2); 6.817 (1.2); 6.802 (2.1); 6.796 (2.0); 6.780 (1.0); 6.774 (1.0); 6.123 (11.2); 5.810 (0.3); 4.811 (3.0); 4.775 (4.2); 4.644 (4.1); 4.608 (2.8); 3.449 (2.6); 3.414 (3.1); 3.381 (0.3); 3.362 (1.2); 3.332 (587.9); 3.298 (0.6); 3.286 (0.5); 3.101 (3.2); 3.067 (2.7); 3.054 (0.6); 2.995 (0.8); 2.711 (0.4); 2.676 (0.7); 2.671 (1.0); 2.667 (0.7); 2.663 (0.4); 2.541 (120.5); 2.525 (2.6); 2.511 (60.5); 2.507 (122.6); 2.502 (160.6); 2.498 (115.0); 2.493 (54.7); 2.368 (0.5); 2.333 (0.8); 2.329 (1.0); 2.324 (0.7); 0.008 (0.6); 0.000 (16.2); -0.009 (0.5) |
| Example I-44: ¹H-NMR (400.0 MHz, d₆-DMSO): |
| δ= 8.311 (15.2); 8.156 (7.2); 8.143 (7.4); 7.804 (16.0); 7.251 (1.7); 7.244 (1.7); 7.228 (1.9); 7.221 (3.0); 7.214 (1.8); 7.197 (1.7); 7.191 (1.6); 7.146 (9.1); 7.125 (1.8); 7.119 (3.0); 7.102 (3.1); 7.097 (2.0); 7.079 (1.6); 7.017 (4.8); 7.014 (4.6); 7.004 (4.7); 7.001 (4.5); 6.874 (1.8); 6.867 (1.8); 6.852 (3.2); 6.846 (3.0); 6.831 (1.6); 6.825 (1.5); 6.163 (14.1); 4.745 (4.3); 4.710 (6.2); 4.587 (5.9); 4.551 (4.0); 3.358 (13.1); 3.333 (2.9); 3.297 (3.8); 3.131 (5.2); 3.097 (3.8); 3.006 (0.4); 2.719 (0.5); 2.550 (88.2); 2.528 (0.4); 2.519 (3.9); 2.514 (8.0); 2.510 (10.6); 2.505 (7.6); 2.501 (3.6); 2.375 (0.5); 2.081 (0.5) |
| Example I-45: ¹H-NMR (400.0 MHz, d₆-DMSO): |
| δ= 8.322 (16.0); 8.218 (9.8); 8.216 (9.8); 7.791 (15.6); 7.307 (6.7); 7.294 (6.7); 7.254 (1.8); 7.248 (1.8); 7.232 (2.1); 7.224 (3.2); 7.218 (2.0); 7.201 (1.9); 7.195 (1.8); 7.126 (1.5); 7.109 (2.0); 7.104 (3.2); 7.086 (3.2); 7.081 (2.2); 7.064 (1.7); 6.869 (1.9); 6.863 (1.9); 6.848 (3.4); 6.842 (3.1); 6.826 (1.7); 6.820 (1.5); 6.187 (15.6); 4.797 (4.2); 4.761 (6.3); 4.644 (6.1); 4.608 (4.0); 3.447 (4.1); 3.413 (4.8); 3.363 (0.9); 3.332 (273.8); 3.289 (0.4); 3.279 (0.4); 3.104 (4.9); 3.069 (4.0); 2.996 (1.1); 2.712 (0.3); 2.676 (0.5); 2.672 (0.7); 2.667 (0.5); 2.542 (83.4); 2.511 (44.9); 2.507 (84.3); 2.503 (106.7); 2.498 (77.4); 2.368 (0.4); 2.334 (0.5); 2.329 (0.7); 2.325 (0.5); 0.000 (16.9); -0.008 (0.8) |
| Example I-46: ¹H-NMR (400.0 MHz, d₆-DMSO): |
| δ= 8.286 (11.6); 8.059 (6.0); 8.046 (6.2); 7.919 (12.0); 7.394 (4.2); 7.373 (8.0); 7.354 (12.4); 7.332 (8.9); 7.227 (2.9); 7.215 (5.4); 7.202 (2.8); 7.132 (2.6); 7.114 (4.4); 7.095 (2.0); 6.948 (0.4); 6.921 (8.7); 6.902 (16.0); 6.880 (8.8); 5.879 (10.1); 4.761 (1.7); 4.725 (6.4); 4.706 (6.4); 4.671 (1.7); 3.381 (4.4); 3.365 (30.0); 3.348 (4.5); 3.125 (4.0); 3.091 (3.2); 2.718 (0.5); 2.549 (88.2); 2.513 (8.4); 2.509 (11.4); 2.505 (9.2); 2.374 (0.5); 0.000 (2.5) |
| Example I-47: ¹H-NMR (400.0 MHz, d₆-DMSO): |
| δ= 8.201 (14.9); 8.159 (6.6); 8.146 (6.8); 7.862 (15.3); 7.362 (6.2); 7.357 (2.4); 7.346 (3.6); 7.341 (15.1); 7.336 (2.6); 7.311 (15.6); 7.305 (3.4); 7.294 (2.3); 7.289 (6.4); 7.166 (8.0); 7.031 (4.4); 7.028 (4.2); 7.018 (4.2); 7.015 (4.0); 5.928 (16.0); 4.643 (3.6); 4.608 (6.2); 4.531 (6.1); 4.496 (3.6); 3.345 (29.6); 3.283 (3.5); 3.249 (5.3); 3.155 (5.2); 3.121 (3.3); 3.005 (0.5); 2.719 (0.5); 2.550 (95.6); 2.533 (0.4); 2.527 (0.6); 2.519 (7.5); 2.514 (15.5); 2.510 (20.5); 2.505 (14.6); 2.501 (6.9); 2.375 (0.5); 2.082 (0.4) |
| Example I-48: ¹H-NMR (400.0 MHz, d₆-DMSO): |
| δ= 8.224 (16.0); 8.171 (11.4); 7.888 (15.4); 7.351 (7.9); 7.333 (11.5); 7.266 (4.5); 7.249 (10.7); 7.230 (7.4); 7.215 (5.4); 7.194 (9.2); 7.181 (8.3); 5.846 (15.6); 4.702 (2.7); 4.666 (9.5); 4.644 (9.5); 4.609 (2.6); 3.389 (0.4); 3.351 (7.3); 3.332 (228.1); 3.317 (9.3); 3.085 (6.0); 3.051 (4.7); 2.995 (0.6); 2.712 (0.5); 2.671 (0.7); 2.542 (114.5); 2.506 (82.9); 2.502 (103.6); 2.498 (78.6); 2.368 (0.6); 2.329 (0.7); 0.000 (4.6) |
| Example I-49: ¹H-NMR (400.0 MHz, d₆-DMSO): |
| δ= 8.773 (0.4); 8.226 (15.7); 8.118 (0.4); 7.999 (9.0); 7.987 (9.2); 7.886 (16.0); 7.521 (0.4); 7.500 (0.4); 7.347 (7.6); 7.329 (10.4); 7.260 (4.2); 7.243 (10.3); 7.224 (7.4); 7.212 (5.4); 7.202 (1.8); 7.195 (4.5); 7.187 (1.0); 7.177 (1.3); 7.142 (4.0); 7.130 (7.4); 7.117 (3.8); 5.851 (13.6); 4.715 (2.2); 4.679 (9.5); 4.663 (9.5); 4.627 (2.2); 3.389 (4.6); 3.350 (53.7); 3.149 (5.6); 3.115 (4.3); 2.714 (0.5); 2.568 (0.4); 2.561 (1.0); 2.545 (102.8); 2.531 (1.6); 2.522 (1.2); 2.510 (16.9); 2.505 (23.0); 2.501 (18.1); 2.371 (0.5); 0.000 (6.8) |
| Example I-50: ¹H-NMR (400.0 MHz, d₆-DMSO): |
| δ= 8.310 (16.0); 8.200 (1.2); 8.187 (1.2); 8.152 (7.0); 8.140 (7.2); 7.788 (16.0); 7.754 (1.3); 7.749 (1.3); 7.664 (0.7); 7.643 (0.9); 7.584 (8.2); 7.578 (8.3); 7.543 (0.7); 7.538 (0.7); 7.528 (0.9); 7.523 (0.8); 7.507 (0.9); 7.502 (0.9); 7.468 (0.7); 7.463 (0.7); 7.356 (0.6); 7.351 (0.7); 7.349 (0.6); 7.343 (0.5); 7.333 (1.7); 7.328 (0.5); 7.321 (1.1); 7.312 (1.4); 7.300 (0.6); 7.283 (6.7); 7.261 (11.4); 7.253 (1.2); 7.249 (0.9); 7.242 (0.8); 7.237 (0.8); 7.232 (0.9); 7.206 (6.5); 7.200 (6.1); 7.184 (3.9); 7.178 (4.2); 7.169 (8.9); 7.110 (1.6); 7.107 (1.4); 7.069 (0.5); 7.064 (0.4); 7.048 (0.4); 7.043 (0.4); 7.028 (4.9); 7.025 (4.7); 7.016 (4.8); 7.012 (4.6); 6.994 (1.4); 6.839 (1.3); 6.820 (0.8); 6.816 (0.8); 6.806 (0.8); 6.803 (0.8); 6.259 (12.4); 5.761 (1.6); 5.081 (5.2); 5.045 (6.0); 4.705 (6.0); 4.669 (5.1); 4.193 (1.1); 3.776 (4.9); 3.741 (5.3); 3.344 (19.3); 3.155 (5.0); 3.120 (4.5); 3.005 (0.6); 2.990 (0.5); 2.977 (0.5); 2.719 (0.7); 2.550 (155.4); 2.533 (0.9); 2.519 (12.2); 2.515 (24.9); 2.510 (32.9); 2.505 (23.7); 2.501 (11.4); 2.375 (0.7); 2.082 (0.5) |
| Example I-51: ¹H-NMR (400.0 MHz, CD3CN): |
| δ= 8.164 (4.3); 8.159 (4.4); 8.152 (4.5); 8.147 (4.3); 8.017 (13.3); 7.925 (0.8); 7.780 (13.2); 7.546 (4.4); 7.541 (4.3); 7.527 (4.8); 7.522 (4.6); 7.359 (5.8); 7.355 (7.9); 7.350 (2.7); 7.343 (5.5); 7.338 (11.2); 7.335 (10.3); 7.329 (1.4); 7.287 (3.2); 7.282 (4.3); 7.277 (2.0); 7.265 (11.4); 7.262 (4.1); 7.250 (3.8); 7.247 (7.0); 7.239 (4.0); 7.235 (6.2); 7.232 (3.3); 7.225 (1.8); 7.218 (4.6); 7.210 (0.8); 7.204 (0.8); 7.200 (1.2); 7.197 (0.6); 7.129 (5.2); 7.117 (5.1); 7.110 (4.8); 7.098 (4.6); 5.447 (3.3); 4.761 (7.2); 4.726 (10.2); 4.559 (10.2); 4.523 (7.3); 4.460 (16.0); 3.454 (0.3); 3.369 (3.2); 3.333 (11.3); 3.307 (11.6); 3.271 (3.3); 2.888 (10.6); 2.772 (8.6); 2.771 (8.8); 2.139 (49.1); 2.119 (0.4); 2.113 (0.6); 2.107 (0.8); 2.101 (0.6); 1.971 (1.2); 1.964 (8.6); 1.958 (13.3); 1.952 (54.2); 1.946 (94.0); 1.939 (121.2); 1.933 (82.6); 1.927 (41.8); 1.914 (0.4); 1.774 (0.5); 1.768 (0.7); 1.762 (0.5); 1.285 (0.4); 1.270 (0.4); 0.000 (4.8) |
| Example I-52: ¹H-NMR (400.0 MHz, CD3CN): |
| δ= 8.295 (1.0); 8.179 (2.8); 8.174 (2.9); 8.168 (2.9); 8.163 (2.8); 8.134 (0.3); 8.060 (8.6); 7.926 (1.6); 7.910 (1.0); 7.784 (8.4); 7.572 (2.8); 7.567 (2.7); 7.553 (3.0); 7.548 (2.9); 7.333 (0.9); 7.327 (5.9); 7.321 (2.5); 7.310 (3.3); 7.305 (13.2); 7.299 (1.9); 7.274 (2.0); 7.268 (12.8); 7.263 (2.9); 7.252 (2.2); 7.246 (5.6); 7.240 (0.7); 7.158 (3.5); 7.146 (3.3); 7.139 (3.1); 7.127 (2.9); 4.757 (4.5); 4.721 (6.4); 4.571 (6.4); 4.553 (6.8); 4.535 (4.4); 3.365 (2.5); 3.329 (6.7); 3.289 (6.8); 3.253 (2.5); 2.889 (16.0); 2.772 (12.8); 2.771 (12.9); 2.473 (0.4); 2.468 (0.8); 2.464 (1.1); 2.459 (0.8); 2.454 (0.4); 2.153 (191.7); 2.120 (1.2); 2.113 (1.6); 2.107 (2.0); 2.101 (1.4); 2.095 (0.7); 1.972 (2.1); 1.964 (21.1); 1.958 (31.7); 1.952 (145.5); 1.946 (255.5); 1.940 (333.2); 1.934 (227.8); 1.928 (116.3); 1.915 (1.4); 1.781 (0.7); 1.774 (1.4); 1.768 (1.9); 1.762 (1.3); 1.756 (0.7); 1.713 (1.9); 1.479 (0.4); 1.461 (0.4); 1.277 (0.5); 1.204 (0.5); 0.146 (1.0); 0.008 (7.9); 0.000 (246.3); -0.009 (7.4); - 0.150 (0.9) |
| Example I-53: ¹H-NMR (400.0 MHz, d₆-DMSO): |
| δ= 8.347 (7.8); 8.343 (7.8); 8.306 (16.0); 8.201 (4.9); 8.199 (4.7); 8.188 (5.0); 8.186 (4.9); 7.757 (15.8); 7.567 (9.1); 7.561 (9.2); 7.255 (6.1); 7.233 (10.4); 7.179 (6.5); 7.173 (6.2); 7.165 (3.3); 7.157 (4.4); 7.152 (7.4); 7.136 (3.1); 6.201 (14.8); 5.209 (5.1); 5.173 (5.7); 4.727 (5.5); 4.691 (4.8); 3.792 (4.2); 3.757 (4.6); 3.332 (54.9); 3.226 (4.5); 3.192 (4.0); 2.543 (53.5); 2.526 (0.6); 2.521 (0.9); 2.512 (13.8); 2.508 (28.2); 2.503 (37.1); 2.499 (26.4); 2.494 (12.5); 0.008 (0.5); 0.000 (13.8); -0.009 (0.4) |
| Example I-54: ¹H-NMR (400.0 MHz, d₆-DMSO): |
| δ= 8.621 (3.1); 8.314 (1.1); 8.282 (2.8); 8.231 (6.6); 8.151 (9.3); 8.103 (3.6); 7.927 (3.2); 7.508 (1.0); 7.499 (1.2); 7.490 (1.7); 7.482 (2.4); 7.468 (2.1); 7.461 (3.0); 7.439 (1.6); 7.432 (1.6); 7.346 (7.1); 7.324 (7.9); 7.284 (0.8); 7.270 (1.0); 7.261 (1.8); 7.247 (1.9); 7.238 (1.3); 7.224 (2.0); 7.210 (1.3); 7.201 (2.6); 7.187 (2.6); 7.178 (2.1); 7.164 (2.2); 7.150 (2.3); 7.139 (2.7); 7.129 (3.0); 7.119 (2.6); 7.107 (1.3); 7.045 (0.4); 7.023 (0.4); 6.989 (6.0); 6.925 (2.3); 6.902 (8.0); 6.884 (8.2); 6.862 (2.9); 6.851 (8.0); 6.829 (7.5); 6.644 (3.9); 5.898 (9.3); 5.519 (1.4); 5.507 (3.1); 5.495 (1.4); 4.728 (1.6); 4.693 (3.2); 4.637 (3.3); 4.602 (1.6); 4.184 (0.7); 4.173 (0.9); 4.155 (1.7); 4.144 (1.6); 4.111 (1.6); 4.098 (1.9); 4.082 (1.0); 4.069 (0.8); 3.919 (1.1); 3.902 (16.0); 3.886 (3.4); 3.861 (3.3); 3.827 (1.2); 3.508 (0.4); 3.447 (3.0); 3.411 (4.1); 3.328 (519.6); 3.257 (0.8); 3.211 (3.8); 3.176 (4.5); 3.163 (1.9); 2.995 (0.4); 2.672 (3.1); 2.542 (27.4); 2.507 (426.9); 2.503 (579.3); 2.499 (465.0); 2.398 (0.5); 2.392 (0.5); 2.384 (0.4); 2.368 (0.4); 2.330 (3.3); 1.908 (0.4); 1.260 (0.3); 1.235 (0.5); 0.146 (0.4); 0.000 (83.7); -0.149 (0.4) |
| Example I-55: ¹H-NMR (499.9 MHz, CDCl₃): |
| δ= 8.024 (6.4); 8.015 (6.7); 7.945 (9.4); 7.835 (10.0); 7.269 (4.9); 7.254 (1.7); 7.249 (2.8); 7.236 (2.8); 7.230 (1.9); 7.217 (1.7); 7.082 (3.0); 7.073 (5.7); 7.063 (3.1); 6.818 (1.5); 6.813 (1.6); 6.802 (1.6); 6.796 (2.0); 6.794 (2.0); 6.789 (1.8); 6.777 (1.6); 6.772 (1.7); 6.711 (1.4); 6.707 (1.4); 6.695 (2.5); 6.693 (2.3); 6.690 (2.3); 6.678 (1.5); 6.674 (1.4); 5.156 (1.3); 4.973 (4.0); 4.945 (4.5); 4.488 (4.7); 4.460 (4.3); 3.318 (2.1); 3.291 (5.0); 3.253 (5.7); 3.226 (2.6); 2.003 (16.0); 1.993 (0.6); 1.256 (0.5); 0.000 (4.1) |
| Example I-56: ¹H-NMR (499.9 MHz, CDCl₃): |
| δ= 8.054 (0.9); 8.027 (6.0); 8.017 (6.2); 7.960 (9.2); 7.843 (9.6); 7.268 (5.2); 7.253 (1.7); 7.247 (2.7); 7.234 (2.7); 7.229 (1.8); 7.216 (1.5); 7.082 (2.8); 7.072 (5.4); 7.063 (2.9); 6.820 (1.4); 6.815 (1.5); 6.803 (1.6); 6.798 (2.0); 6.796 (1.9); 6.791 (1.7); 6.779 (1.5); 6.774 (1.6); 6.713 (1.4); 6.708 (1.4); 6.696 (2.4); 6.694 (2.3); 6.692 (2.3); 6.680 (1.4); 6.675 (1.3); 5.136 (0.3); 5.116 (0.3); 4.975 (4.0); 4.947 (4.5); 4.491 (4.6); 4.464 (4.2); 3.320 (2.2); 3.293 (5.0); 3.254 (5.6); 3.227 (2.6); 2.003 (16.0); 1.256 (0.4); 0.000 (4.3) |
| Example I-57: ¹H-NMR (300.2 MHz, CDCl₃): |
| δ= 8.023 (3.3); 8.006 (3.5); 7.870 (5.2); 7.794 (5.1); 7.646 (0.8); 7.367 (0.5); 7.300 (124.9); 7.233 (0.4); 7.167 (0.8); 7.145 (0.8); 7.136 (1.5); 7.115 (1.5); 7.107 (1.0); 7.085 (0.9); 7.058 (1.5); 7.043 (2.7); 7.026 (1.5); 6.949 (0.7); 6.929 (0.9); 6.920 (0.8); 6.901 (0.9); 6.892 (0.9); 6.888 (1.0); 6.879 (0.8); 6.859 (0.8); 6.851 (0.8); 6.783 (0.8); 6.776 (0.7); 6.756 (1.1); 6.751 (1.1); 6.727 (0.6); 6.719 (0.5); 4.965 (1.5); 4.915 (3.6); 4.848 (3.7); 4.797 (1.7); 3.636 (16.0); 3.507 (0.4); 3.461 (3.7); 3.450 (3.7); 3.403 (0.4); 2.997 (1.1); 2.925 (0.9); 1.654 (0.6); 1.587 (106.3); 1.522 (0.5); 1.293 (1.1); 0.893 (0.4); 0.872 (0.3); 0.233 (0.5); 0.108 (2.8); 0.049 (4.4); 0.039 (126.0); 0.028 (4.7); -0.028 (0.5); -0.159 (0.5) |
| |
| Example XII-1: ¹H-NMR (400.0 MHz, CD3CN): |
| δ= 8.488 (0.4); 8.318 (0.3); 8.247 (5.8); 8.242 (5.9); 8.235 (6.3); 8.230 (6.2); 8.223 (0.8); 8.218 (0.8); 8.215 (0.5); 8.210 (0.4); 8.203 (0.8); 8.198 (0.6); 8.191 (0.6); 8.186 (0.6); 7.757 (0.6); 7.752 (0.5); 7.738 (0.7); 7.733 (0.5); 7.674 (5.9); 7.669 (5.7); 7.655 (6.5); 7.650 (6.1); 7.640 (0.5); 7.588 (0.4); 7.357 (0.6); 7.353 (0.4); 7.346 (0.7); 7.339 (0.5); 7.326 (0.9); 7.313 (0.5); 7.294 (0.3); 7.282 (0.8); 7.270 (0.8); 7.263 (7.8); 7.257 (1.1); 7.251 (7.7); 7.244 (7.0); 7.239 (1.0); 7.232 (6.5); 7.227 (0.9); 7.221 (5.4); 7.214 (1.2); 7.208 (7.0); 7.202 (11.5); 7.201 (12.4); 7.194 (1.0); 7.189 (1.3); 7.181 (11.4); 7.178 (8.8); 7.141 (8.8); 7.136 (7.7); 7.126 (1.5); 7.121 (4.5); 7.119 (4.2); 7.115 (4.3); 7.111 (2.0); 7.105 (1.1); 7.093 (0.8); 7.086 (0.8); 7.074 (1.1); 7.069 (0.8); 7.053 (0.5); 7.048 (0.4); 6.106 (0.5); 4.430 (0.5); 4.410 (0.7); 4.391 (0.5); 4.001 (0.5); 3.983 (0.5); 3.577 (0.4); 3.553 (2.0); 3.517 (0.5); 3.494 (7.1); 3.481 (0.7); 3.458 (10.6); 3.315 (10.8); 3.294 (0.5); 3.278 (7.2); 3.225 (1.2); 3.214 (1.2); 3.207 (0.7); 3.196 (0.5); 3.193 (0.8); 3.186 (0.4); 3.183 (0.5); 3.172 (1.1); 3.136 (0.6); 2.938 (12.9); 2.926 (16.0); 2.831 (12.8); 2.819 (10.3); 2.740 (0.9); 2.728 (0.9); 2.710 (0.4); 2.704 (0.4); 2.697 (0.5); 2.690 (0.3); 2.464 (0.4); 2.380 (1.0); 2.157 (69.5); 2.120 (0.4); 2.114 (0.6); 2.108 (0.7); 2.102 (0.5); 1.972 (0.9); 1.965 (7.7); 1.959 (11.7); 1.953 (50.3); 1.947 (87.6); 1.941 (113.1); 1.934 (77.0); 1.928 (39.4); 1.915 (0.6); 1.775 (0.5); 1.769 (0.7); 1.763 (0.5); 1.621 (1.3); 1.584 (0.3); 1.454 (0.9); 1.441 (1.0); 1.436 (1.5); 1.273 (2.5); 1.255 (2.4); 1.249 (0.8); 1.230 (0.5); 1.227 (0.4); 1.045 (1.0); 0.985 (2.2); 0.973 (2.3); 0.008 (2.0); 0.000 (56.0); -0.009 (1.7) |
| Example XII-23: ¹H-NMR (400.0 MHz, CD3CN): |
| δ= 8.242 (3.5); 8.237 (3.7); 8.230 (3.7); 8.225 (3.7); 7.711 (3.7); 7.706 (3.6); 7.692 (4.0); 7.687 (3.9); 7.406 (3.2); 7.402 (5.5); 7.399 (8.4); 7.395 (3.6); 7.358 (0.3); 7.346 (0.4); 7.340 (1.3); 7.335 (3.6); 7.333 (3.4); 7.331 (3.5); 7.324 (8.0); 7.320 (16.0); 7.314 (8.3); 7.309 (6.0); 7.304 (2.8); 7.301 (3.2); 7.297 (2.2); 7.287 (0.4); 7.283 (0.4); 7.251 (4.8); 7.239 (4.7); 7.232 (4.5); 7.220 (4.4); 3.644 (5.8); 3.606 (7.7); 3.373 (7.5); 3.335 (5.6); 3.196 (0.4); 3.012 (0.4); 2.999 (0.4); 2.835 (8.3); 2.823 (11.1); 2.756 (9.8); 2.744 (7.3); 2.464 (0.3); 2.165 (60.0); 2.114 (0.4); 2.108 (0.5); 2.102 (0.4); 1.972 (0.7); 1.965 (4.6); 1.959 (7.0); 1.953 (31.2); 1.947 (54.8); 1.940 (71.9); 1.934 (49.4); 1.928 (25.4); 1.775 (0.3); 1.769 (0.4); 1.621 (1.7); 1.531 (0.5); 1.517 (0.5); 1.503 (0.8); 1.486 (0.8); 1.436 (1.2); 1.239 (0.8); 1.229 (0.5); 1.222 (0.9); 0.927 (0.9); 0.914 (0.8); 0.008 (1.6); 0.000 (44.7); -0.009 (1.6) |
| Example XII-24: ¹H-NMR (400.0 MHz, CD3CN): |
| δ= 8.318 (0.3); 8.239 (2.5); 8.234 (2.6); 8.227 (2.7); 8.222 (2.7); 7.698 (2.6); 7.693 (2.5); 7.679 (2.9); 7.674 (2.8); 7.379 (2.5); 7.377 (2.0); 7.372 (1.4); 7.363 (2.4); 7.356 (15.9); 7.346 (16.0); 7.340 (2.6); 7.331 (1.7); 7.326 (3.0); 7.324 (2.9); 7.306 (0.6); 7.291 (0.7); 7.269 (0.4); 7.266 (0.3); 7.257 (0.7); 7.250 (1.2); 7.244 (3.3); 7.232 (3.2); 7.225 (3.0); 7.213 (2.8); 3.621 (4.0); 3.582 (5.4); 3.371 (5.2); 3.333 (3.9); 3.011 (0.4); 2.999 (0.5); 2.834 (5.7); 2.822 (7.4); 2.815 (0.5); 2.746 (6.6); 2.734 (5.1); 2.143 (26.6); 2.120 (0.3); 2.114 (0.4); 2.107 (0.5); 2.101 (0.3); 1.964 (3.9); 1.958 (6.5); 1.952 (28.4); 1.946 (49.8); 1.940 (65.0); 1.934 (44.5); 1.928 (22.7); 1.768 (0.4); 1.621 (2.1); 1.527 (0.5); 1.514 (0.5); 1.437 (1.4); 1.241 (0.4); 1.223 (0.4); 0.914 (1.0); 0.901 (1.0); 0.008 (2.7); 0.000 (67.0); -0.009 (2.3) |
| Example XII-25: ¹H-NMR (400.0 MHz, CD3CN): |
| δ= 8.323 (0.5); 8.318 (0.6); 8.311 (0.5); 8.306 (0.5); 8.244 (5.5); 8.240 (5.8); 8.233 (5.8); 8.228 (5.7); 7.778 (0.6); 7.716 (5.7); 7.711 (5.7); 7.697 (6.3); 7.692 (6.2); 7.663 (9.7); 7.642 (16.0); 7.611 (0.5); 7.606 (0.5); 7.582 (15.5); 7.561 (9.4); 7.507 (0.5); 7.487 (0.4); 7.355 (0.6); 7.343 (0.6); 7.336 (0.5); 7.324 (0.5); 7.249 (6.9); 7.237 (6.9); 7.230 (6.5); 7.218 (6.2); 5.447 (5.7); 4.140 (0.4); 4.133 (0.5); 4.130 (0.5); 4.123 (0.4); 3.690 (9.1); 3.651 (11.9); 3.601 (0.6); 3.413 (11.6); 3.375 (8.9); 3.196 (0.6); 3.185 (0.7); 3.182 (0.7); 3.172 (0.7); 2.874 (12.6); 2.862 (15.6); 2.771 (14.6); 2.759 (11.6); 2.709 (0.7); 2.703 (0.7); 2.696 (0.7); 2.689 (0.6); 2.138 (11.1); 1.965 (2.6); 1.959 (4.0); 1.953 (16.9); 1.947 (29.4); 1.941 (38.1); 1.934 (26.0); 1.928 (13.4); 1.621 (1.4); 1.551 (0.4); 1.537 (0.4); 1.522 (0.6); 1.504 (0.6); 1.454 (0.6); 1.441 (0.6); 1.271 (0.4); 1.259 (0.7); 1.241 (0.7); 0.910 (0.9); 0.897 (0.9); 0.000 (0.6) |
| Example XII-26: ¹H-NMR (400.0 MHz, CD3CN): |
| δ= 8.246 (3.9); 8.241 (4.2); 8.234 (4.1); 8.229 (4.1); 7.703 (10.9); 7.699 (8.7); 7.694 (5.4); 7.687 (5.8); 7.682 (16.0); 7.555 (14.6); 7.538 (4.7); 7.534 (11.0); 7.248 (4.5); 7.237 (4.5); 7.230 (4.3); 7.218 (4.0); 3.675 (6.2); 3.636 (8.1); 3.403 (8.0); 3.364 (6.1); 2.874 (8.5); 2.862 (10.4); 2.760 (9.6); 2.748 (7.9); 2.464 (0.3); 2.153 (107.8); 2.120 (0.4); 2.114 (0.6); 2.108 (0.7); 2.101 (0.5); 1.964 (5.2); 1.958 (10.4); 1.952 (44.0); 1.946 (77.4); 1.940 (101.9); 1.934 (71.1); 1.928 (37.1); 1.775 (0.5); 1.769 (0.6); 1.762 (0.4); 1.437 (0.6); 1.428 (0.6); 1.218 (0.5); 1.203 (0.5); 0.146 (0.5); 0.008 (5.5); 0.000 (120.2); -0.009 (6.0); -0.150 (0.5) |
| Example XII-47: ¹H-NMR (300.2 MHz, CDCl₃): |
| δ= 8.116 (14.3); 8.100 (14.8); 7.300 (14.3); 7.256 (3.2); 7.235 (3.6); 7.229 (5.7); 7.221 (1.1); 7.208 (6.0); 7.199 (3.8); 7.185 (1.2); 7.178 (2.8); 7.150 (6.7); 7.134 (12.8); 7.118 (6.4); 6.886 (0.3); 6.877 (2.7); 6.869 (4.9); 6.864 (2.4); 6.858 (4.5); 6.849 (3.5); 6.841 (7.8); 6.832 (10.7); 6.823 (3.6); 6.813 (3.5); 6.805 (9.2); 6.796 (2.0); 3.841 (0.4); 3.830 (0.3); 3.690 (0.4); 3.663 (0.3); 3.613 (7.3); 3.565 (9.0); 3.166 (9.0); 3.118 (7.3); 3.021 (12.5); 3.004 (15.6); 2.878 (15.4); 2.862 (12.5); 2.771 (0.6); 2.758 (0.6); 1.608 (16.0); 1.304 (1.3); 0.941 (0.5); 0.918 (1.5); 0.895 (0.6); 0.048 (0.5); 0.037 (15.1); 0.026 (0.6) |

### Use Examples

**Example A: in vivo preventive test on Alternaria brassicae (leaf spot on radish)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of radish are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Alternaria brassicae* spores. The contaminated radish plants are incubated for 6 days at 20°C and at 100% relative humidity.

The test is evaluated 6 days after the inoculation. 0% means an efficacy which corresponds to that of the control plants while an efficacy of 100% means that no disease is observed.

In this test the following compounds according to the invention showed efficacy between 70% and 79% at a concentration of 500 ppm of active ingredient: I-07.

In this test the following compounds according to the invention showed efficacy between 90% and 100% at a concentration of 500 ppm of active ingredient: 1-29.

**Example B: in vivo preventive test on Botrvtis cinerea (grey mould)**

| | | |
|---|---|---|
| Solvent: | 5% | by volume of Dimethyl sulfoxide |
| | 10% | by volume of Acetone |
| Emulsifier: | 1 µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of gherkin are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Botrytis cinerea* spores. The contaminated gherkin plants are incubated for 4 to 5 days at 17°C and at 90% relative humidity.

The test is evaluated 4 to 5 days after the inoculation. 0% means an efficacy which corresponds to that of the control plants while an efficacy of 100% means that no disease is observed.

In this test the following compounds according to the invention showed efficacy between 70% and 79% at a concentration of 500 ppm of active ingredient: I-22.

In this test the following compounds according to the invention showed efficacy between 80% and 89% at a concentration of 500 ppm of active ingredient: I-17; I-51.

In this test the following compounds according to the invention showed efficacy between 90% and 100% at a concentration of 500 ppm of active ingredient: I-14; I-15; I-16; I-28; I-29; I-41; I-52; I-56.

In this test the following compounds according to the invention showed efficacy between 70% and 79% at a concentration of 100 ppm of active ingredient: I-11; I-36.

In this test the following compounds according to the invention showed efficacy between 80% and 89% at a concentration of 100 ppm of active ingredient: I-12; I-31; I-42.

In this test the following compounds according to the invention showed efficacy between 90% and 100% at a concentration of 100 ppm of active ingredient: I-03; I-04; I-53.

**Example C: in vivo preventive test on Puccinia recondita (brown rust on wheat)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of wheat are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Puccinia recondita* spores. The contaminated wheat plants are incubated for 24 hours at 20°C and at 100% relative humidity and then for 10 days at 20°C and at 70-80% relative humidity.

The test is evaluated 11 days after the inoculation. 0% means an efficacy which corresponds to that of the control plants while an efficacy of 100% means that no disease is observed.

In this test the following compounds according to the invention showed efficacy between 80% and 89% at a concentration of 500 ppm of active ingredient: I-22; I-25; I-26; I-34; I-38; I-47; I-54; I-55.

In this test the following compounds according to the invention showed efficacy between 90% and 100% at a concentration of 500 ppm of active ingredient: I-07; I-08; I-09; I-10; I-14; I-15; I-16; I-17; I-23; I-24; I-27; I-28; I-29; I-32; I-35; I-39; I-40; I-41; I-44; I-45; 1-46; I-49; I-50; I-51; I-56; I-57.

In this test the following compounds according to the invention showed efficacy between 70% and 79% at a concentration of 100 ppm of active ingredient: I-02; I-04; I-11; I-18; I-19; I-20; I-30; I-31; I-42; I-43; I-53.

In this test the following compounds according to the invention showed efficacy between 80% and 89% at a concentration of 100 ppm of active ingredient: I-06; I-12; I-13; I-52.

In this test the following compounds according to the invention showed efficacy between 90% and 100% at a concentration of 100 ppm of active ingredient: I-21; I-33.

**Example D: in vivo preventive test on Pyrenophora teres (net blotch on barley)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of barley are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Pyrenophora teres* spores. The contaminated barley plants are incubated for 48 hours at 20°C and at 100% relative humidity and then for 12 days at 20°C and at 70-80% relative humidity.

The test is evaluated 14 days after the inoculation. 0% means an efficacy which corresponds to that of the control plants while an efficacy of 100% means that no disease is observed.

In this test the following compounds according to the invention showed efficacy between 70% and 79% at a concentration of 500 ppm of active ingredient: I-09; I-10; I-14; I-15; I-23; I-24; I-32; I-41; I-46; I-49; I-50.

In this test the following compounds according to the invention showed efficacy between 80% and 89% at a concentration of 500 ppm of active ingredient: I-07; I-08; I-29; I-44.

In this test the following compounds according to the invention showed efficacy between 90% and 100% at a concentration of 500 ppm of active ingredient: I-45.

In this test the following compounds according to the invention showed efficacy between 70% and 79% at a concentration of 100 ppm of active ingredient: I-12; I-30; I-53.

**Example E: in vivo preventive test on Septoria tritici (leaf spot on wheat)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of wheat are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Septoria tritici* spores. The contaminated wheat plants are incubated for 72 hours at 18°C and at 100% relative humidity and then for 21 days at 20°C and at 90% relative humidity.

The test is evaluated 24 days after the inoculation. 0% means an efficacy which corresponds to that of the control plants while an efficacy of 100% means that no disease is observed.

In this test the following compounds according to the invention showed efficacy between 70% and 79% at a concentration of 500 ppm of active ingredient: I-34.

In this test the following compounds according to the invention showed efficacy between 80% and 89% at a concentration of 500 ppm of active ingredient: I-23; I-55.

In this test the following compounds according to the invention showed efficacy between 90% and 100% at a concentration of 500 ppm of active ingredient: I-07; I-08; I-09; I-10; I-14; I-15; I-16; I-17; I-22; I-24; I-25; I-26; I-27; I-28; I-29; I-32; I-35; I-38; I-39; I-40; I-41; I-44; I-45; I-46; I-47; I-49; I-50; I-51; I-52; I-54; I-56; I-57.

In this test the following compounds according to the invention showed efficacy between 80% and 89% at a concentration of 100 ppm of active ingredient: I-01; I-21; I-33.

In this test the following compounds according to the invention showed efficacy between 90% and 100% at a concentration of 100 ppm of active ingredient: I-02; I-04; I-06; I-11; I-12; I-13; I-18; I-19; I-20; I-30; I-31; I-42; I-43; I-53.

**Example F: in vivo preventive test on Sphaerotheca fuliginea (powdery mildew on cucurbits)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of gherkin are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Sphaerotheca fuliginea* spores. The contaminated gherkin plants are incubated for 72 hours at 18°C and at 100% relative humidity and then for 12 days at 20°C and at 70-80% relative humidity.

The test is evaluated 15 days after the inoculation. 0% means an efficacy which corresponds to that of the control plants while an efficacy of 100% means that no disease is observed.

In this test the following compounds according to the invention showed efficacy between 90% and 100% at a concentration of 500 ppm of active ingredient: I-07; I-08; I-09; I-10; I-14; I-15; I-16; I-17; 1-22; I-23; I-24; I-25; I-26; I-27; I-28; I-29; I-32; I-34; I-35; I-38; I-39; I-40; I-41; I-44; I-45; I-46; I-47; I-49; I-50; I-51; I-52; I-54; I-55; I-56; I-57.

In this test the following compounds according to the invention showed efficacy between 90% and 100% at a concentration of 100 ppm of active ingredient: I-01; I-02; I-03; I-04; I-05; I-06; I-11; I-13; I-18; I-19; I-20; I-21; I-30; I-31; I-33; I-36; I-37; I-42; I-43; I-48; I-53.

**Example G: in vivo preventive test on Uromyces appendiculatus (bean rust)**

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of bean are treated by spraying the active ingredient prepared as described above. Control plants are treated only with an aqueous solution of Acetone/Dimethyl sulfoxide/ Tween® 80.

After 24 hours, the plants are contaminated by spraying the leaves with an aqueous suspension of *Uromyces appendiculatus* spores. The contaminated bean plants are incubated for 24 hours at 20°C and at 100% relative humidity and then for 10 days at 20°C and at 70-80% relative humidity.

The test is evaluated 11 days after the inoculation. 0% means an efficacy which corresponds to that of the control plants while an efficacy of 100% means that no disease is observed.

In this test the following compounds according to the invention showed efficacy between 80% and 89% at a concentration of 500 ppm of active ingredient: I-22.

In this test the following compounds according to the invention showed efficacy between 90% and 100% at a concentration of 500 ppm of active ingredient: I-07; I-08; I-09; I-10; I-14; I-15; I-16; I-17; I-23; I-24; I-25; I-26; I-27; I-28; I-29; I-32; I-34; I-35; I-38; I-39; I-40; I-41; I-44; I-45; I-46; I-47; I-49; I-50; I-51; I-52; I-54; I-55; I-56; I-57.

In this test the following compounds according to the invention showed efficacy between 70% and 79% at a concentration of 100 ppm of active ingredient: I-21.

In this test the following compounds according to the invention showed efficacy between 80% and 89% at a concentration of 100 ppm of active ingredient: I-05.

In this test the following compounds according to the invention showed efficacy between 90% and 100% at a concentration of 100 ppm of active ingredient: I-01; I-02; I-03; I-04; I-11; I-12; I-13; I-18; I-19; I-20; I-30; I-31; I-33; I-36; I-42; I-43; I-53.

**Example H: in vivo preventive test on Alternaria test (tomatoes)**

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous spore suspension of ***Alternaria solani.*** The plants are then placed in an incubation cabinet at approximately 20°C and a relative atmospheric humidity of 100%.

The test is evaluated 3 days after the inoculation. 0% means an efficacy which corresponds to that of the untreated control while an efficacy of 100% means that no disease is observed.

In this test the following compounds according to the invention showed efficacy between 80% and 89% at a concentration of 100 ppm of active ingredient: I-29.

In this test the following compounds according to the invention showed efficacy between 90% and 100% at a concentration of 100 ppm of active ingredient: I-56.

**Example I: in vivo preventive test on Phakopsora test (soybeans)**

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous spore suspension of the causal agent of soybean rust **(*Phakopsora pachyrhizi*)** and stay for 24h without light in an incubation cabinet at approximately 24°C and a relative atmospheric humidity of 95 %.

The plants remain in the incubation cabinet at approximately 24°C and a relative atmospheric humidity of approximately 80 % and a day / night interval of 12h.

The test is evaluated 7 days after the inoculation. 0% means an efficacy which corresponds to that of the untreated control, while an efficacy of 100% means that no disease is observed.

In this test the following compounds according to the invention showed efficacy between 90% and 100% at a concentration of 100 ppm of active ingredient: I-29; I-56.

**Example J: in vivo preventive test on Venturia test (apples)**

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous conidia suspension of the causal agent of apple scab ***(Venturia inaequalis)*** and then remain for 1 day in an incubation cabinet at approximately 20°C and a relative atmospheric humidity of 100%.

The plants are then placed in a greenhouse at approximately 21°C and a relative atmospheric humidity of approximately 90%.

The test is evaluated 10 days after the inoculation. 0% means an efficacy which corresponds to that of the untreated control, while an efficacy of 100% means that no disease is observed.

In this test the following compounds according to the invention showed efficacy between 90% and 100% at a concentration of 100 ppm of active ingredient: I-29.

**Example K: in vivo preventive Blumeria test (barley)**

| | | |
|---|---|---|
| Solvent: | 49 | parts by weight of N,N-dimethylacetamide |
| Emulsifier: | 1 | part by weight of alkylaryl polyglycol ether |

To produce a suitable preparation of active compound, 1 part by weight of active compound or active compound combination is mixed with the stated amounts of solvent and emulsifier, and the concentrate is diluted with water to the desired concentration.

To test for preventive activity, young plants are sprayed with the preparation of active compound or active compound combination at the stated rate of application.

After the spray coating has been dried, the plants are dusted with spores of ***Blumeria graminis f.sp. hordei.***

The plants are placed in the greenhouse at a temperature of approximately 18°C and a relative atmospheric humidity of approximately 80% to promote the development of mildew pustules.

The test is evaluated 7 days after the inoculation. 0% means an efficacy which corresponds to that of the untreated control, while an efficacy of 100% means that no disease is observed.

In this test the following compounds according to the invention showed efficacy between 70% and 79% at a concentration of 500 ppm of active ingredient: I-35.

In this test the following compounds according to the invention showed efficacy between 80% and 89% at a concentration of 500 ppm of active ingredient: I-24.

In this test the following compounds according to the invention showed efficacy between 90% and 100% at a concentration of 500 ppm of active ingredient: I-01; I-04; I-06; I-08; I-10; I-12; I-13; I-14; I-15; I-16; I-17; I-18; I-19; I-20; I-21; I-22; I-23; I-28; I-29; I-30; I-31; I-33; I-40; I-41; I-42; I-44; I-45; I-47; I-50; I-51; I-52; I-53; I-56; I-57.

**Example L: in vivo preventive Leptosphaeria nodorum test (wheat)**

| | | |
|---|---|---|
| Solvent: | 49 | parts by weight of N,N-dimethylacetamide |
| Emulsifier: | 1 | part by weight of alkylaryl polyglycol ether |

To produce a suitable preparation of active compound, 1 part by weight of active compound or active compound combination is mixed with the stated amounts of solvent and emulsifier, and the concentrate is diluted with water to the desired concentration.

To test for preventive activity, young plants are sprayed with the preparation of active compound or active compound combination at the stated rate of application.

After the spray coating has been dried, the plants are sprayed with a spore suspension of ***Leptosphaeria nodorum.*** The plants remain for 48 hours in an incubation cabinet at approximately 20°C and a relative atmospheric humidity of approximately 100%.

The plants are placed in the greenhouse at a temperature of approximately 25°C and a relative atmospheric humidity of approximately 80%.

The test is evaluated 8 days after the inoculation. 0% means an efficacy which corresponds to that of the untreated control, while an efficacy of 100% means that no disease is observed.

In this test the following compounds according to the invention showed efficacy between 70% and 79% at a concentration of 500 ppm of active ingredient: I-12; I-14; I-15; I-21; I-40; I-42; I-50.

In this test the following compounds according to the invention showed efficacy between 80% and 89% at a concentration of 500 ppm of active ingredient: I-04; I-18; I-20; I-29; I-41; I-51.

In this test the following compounds according to the invention showed efficacy between 90% and 100% at a concentration of 500 ppm of active ingredient: I-06; I-08; I-30; I-31; I-33; I-44; I-45; I-53; I-56.

In this test the following compounds according to the invention showed efficacy between 70% and 79% at a concentration of 250 ppm of active ingredient: I-03; I-39.

In this test the following compounds according to the invention showed efficacy between 80% and 89% at a concentration of 250 ppm of active ingredient: I-28.

**Example M: in vivo preventive Pyricularia oryzae test (rice)**

| | | |
|---|---|---|
| Solvent: | 49 | parts by weight of N,N-dimethylacetamide |
| Emulsifier: | 1 | part by weight of alkylaryl polyglycol ether |

To produce a suitable preparation of active compound, 1 part by weight of active compound or active compound combination is mixed with the stated amounts of solvent and emulsifier, and the concentrate is diluted with water to the desired concentration.

To test for preventive activity, young plants are sprayed with the preparation of active compound or active compound combination at the stated rate of application.

After the spray coating has been dried, the plants are sprayed with a spore suspension of ***Pyricularia oryzae.*** The plants remain for 48 hours in an incubation cabinet at approximately 25°C and a relative atmospheric humidity of approximately 100%.

The plants are placed in the greenhouse under a translucent incubations cabinet at a temperature of approximately 25°C and a relative atmospheric humidity of approximately 100%.

The test is evaluated 8 days after the inoculation. 0% means an efficacy which corresponds to that of the control, while an efficacy of 100% means that no disease is observed.

In this test the following compounds according to the invention showed efficacy between 70% and 79% at a concentration of 500 ppm of active ingredient: I-12; I-19; I-46; I-50; I-56.

In this test the following compounds according to the invention showed efficacy between 80% and 89% at a concentration of 500 ppm of active ingredient: I-14; I-15; I-41; I-45.

In this test the following compounds according to the invention showed efficacy between 90% and 100% at a concentration of 500 ppm of active ingredient: I-01; I-06; I-18; I-29; I-42; I-53.

In this test the following compounds according to the invention showed efficacy between 80% and 89% at a concentration of 250 ppm of active ingredient: I-03.

## Claims

1. Triazole derivatives of the formula (I) 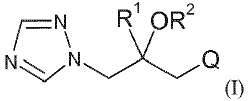 wherein
R¹ represents phenyl or naphthyl; wherein the phenyl or naphthyl may be non-substituted or substituted by one or more group(s) selected from halogen; hydroxyl; cyano; amino; sulfanyl; pentafluoro-λ⁶-sulfanyl; carboxaldehyde, hydroxycarbonyl, C₁-C₈-alkyl; C₁-C₈-haloalkyl; C₁-C₈-cyanoalkyl; C₁-C₈-alkyloxy; C₁-C₈-halogenalkyloxy; tri(C₁-C₈-alkyl)silyl; tri(C₁-C₈-alkyl)silyl-C₁-C₈-alkyl; C₃-C₇-cycloalkyl; C₃-C₇-halogencycloalkyl; C₃-C₇-cycloalkenyl; C₃-C₇-halogencycloalkenyl; C₄-C₁₀-cycloalkylalkyl; C₄-C₁₀-halocycloalkylalkyl; C₆-C₁₂-cycloalkylcycloalkyl; C₁-C₈-alkyl-C₃-C₇-cycloalkyl; C₁-C₈-alkoxy-C₃-C₇-cycloalkyl; tri(C₁-C₈-alkyl)silyl-C₃-C₇-cycloalkyl; C₂-C₈-alkenyl; C₂-C₈-alkynyl; C₂-C₈-alkenyloxy; C₂-C₈-halogenalkenyloxy; C₃-C₈-alkynyloxy; C₃-C₈-halogenoalkynyloxy; C₁-C₈-alkylamino; C₁-C₈-halogenalkylamino; C₁-C₈-cyanoalkoxy; C₄-C₈-cycloalkylalkoxy; C₃-C₆-cycloalkoxy; C₁-C₈-alkylsulfanyl; C₁-C₈-halogenoalkylsulfanyl; C₁-C₈-alkylcarbonyl; C₁-C₈-halogenoalkylcarbonyl; arylcarbonyl; C₃-C₈-cycloalkylcarbonyl; C₃-C₈-halogenocycloalkylcarbonyl; C₁-C₈-alkylcarbamoyl; di-C₁-C₈-alkylcarbamoyl; N-C₁-C₈-alkyloxycarbamoyl; C₁-C₈-alkoxycarbamoyl; N-C₁-C₈-alkyl-C₁-C₈-alkoxycarbamoyl; C₁-C₈-alkoxycarbonyl; C₁-C₈-halogenoalkoxycarbonyl; C₃-C₈-cycloalkoxycarbonyl; C₂-C₈-alkoxyalkylcarbonyl; C₂-C₈-halogenoalkoxyalkylcarbonyl; C₃-C₁₀-cycloalkoxyalkylcarbonyl; C₁-C₈-alkylaminocarbonyl; di-C₁-C₈-alkylaminocarbonyl; C₃-C₈-cycloalkylaminocarbonyl; C₁-C₈-alkylcarbonyloxy; C₁-C₈-halogenoalkylcarbonyloxy; C₃-C₈-cycloalkylcarbonyloxy; C₁-C₈-alkylcarbonylamino; C₁-C₈-halogenoalkylcarbonylamino; C₁-C₈-alkylaminocarbonyloxy; di-C₁-C₈-alkylaminocarbonyloxy; C₁-C₈-alkyloxycarbonyloxy; C₁-C₈-alkylsulfinyl; C₁-C₈-halogenoalkylsulfinyl; C₁-C₈-alkylsulfonyl; C₁-C₈-halogenoalkylsulfonyl; C₁-C₈-alkylsulfonyloxy; C₁-C₈-halogenoalkylsulfonyloxy; C₁-C₈-alkylaminosulfamoyl; di-C₁-C₈-alkylaminosulfamoyl; (C₁-C₈-alkoxyimino)-C₁-C₈-alkyl; (C₃-C₇-cycloalkoxyimino)-C₁-C₈-alkyl; hydroxyimino-C₁-C₈-alkyl; (C₁-C₈-alkoxyimino)-C₃-C₇-cycloalkyl; hydroxyimino-C₃-C₇-cycloalkyl; (C₁-C₈-alkylimino)-oxy; (C₁-C₈-alkylimino)-oxy-C₁-C₈-alkyl; (C₃-C₇-cycloalkylimino)-oxy-C₁-C₈-alkyl; (C₁-C₆-alkylimino)-oxy-C₃-C₇-cycloalkyl; (C₁-C₈-alkenyloxyimino)-C₁-C₈-alkyl; (C₁-C₈-alkynyloxyimino)-C₁-C₈-alkyl; (benzyloxyimino)-C₁-Cs-alkyl; C₁-C₈-alkoxyalkyl; C₁-C₈-alkylthioalkyl; C₁-C₈-alkoxyalkoxyalkyl; C₁-C₈-halogenoalkoxyalkyl; benzyl; phenyl; 5-membered heteroaryl; 6-membered heteroaryl; benzyloxy; phenyloxy; benzylsulfanyl; benzylamino; phenylsulfanyl; or phenylamino;
R² represents H, C₁-C₈-alkyl, -Si(R^{3a})(R^{3b})(R^{3c}), -P(O)(OH)₂, -CH₂-O-P(O)(OH)₂, C(O)-C₁-C₈-alkyl, -C(O)-C₃-C₇-cycloalkyl, -C(O)NH-C₁-C₈-alkyl; -C(O)N-di-C₁-C₈-alkyl; -C(O)O-C₁-C₈-alkyl; wherein the -C(O)-C₁-C₈-alkyl, -C(O)-C₃-C₇-cycloalkyl, C(O)NH-C₁-C₈-alkyl; -C(O)N-di-C₁-C₈-alkyl or -C(O)O-C₁-C₈-alkyl may be non-substituted or substituted by one or more group(s) selected from halogen or C₁-C₈-alkoxy;
wherein
R^{3a}, R^{3b}, R^{3c} represent independent from each other a phenyl or C₁-C₈-alkyl;
Q represents a substituted 6-membered aromatic heterocycle of formula (Q-I) containing one nitrogen atom 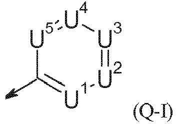
wherein
U¹ represents CX¹ or N;
wherein X¹ represents hydrogen, halogen, C₁-C₈-alkyl; C₁-C₈-haloalkyl; C₁-C₈-alkoxy; or C₁-C₈-halogenalkyloxy;
U² represents CX² or N;
wherein X² represents hydrogen, halogen, C₁-C₈-alkyl; C₁-C₈-haloalkyl; C₁-C₈-alkoxy; or C₁-C₈-halogenalkyloxy;
U³ represents CX³ or N;
wherein X³ represents hydrogen, halogen, C₁-C₈-alkyl; C₁-C₈-haloalkyl; C₁-C₈-alkoxy; or C₁-C₈-halogenalkyloxy;
U⁴ represents CX⁴ or N;
wherein X⁴ represents hydrogen, halogen, C₁-C₈-alkyl; C₁-C₈-haloalkyl; C₁-C₈-alkoxy; or C₁-C₈-halogenalkyloxy;
U⁵ represents CX⁵ or N; wherein X⁵ represents hydrogen, halogen, C₁-C₈-alkyl; C₁-C₈-haloalkyl; C₁-C₈-alkoxy; or C₁-C₈-halogenalkyloxy;
and wherein one of U¹, U², U³, U⁴ or U⁵ represents N and wherein at least one of X¹, X², X³, X⁴ or X⁵ is different from hydrogen;
and its salts or N-oxides.

2. Triazole derivatives of the formula (I) according to claim 1, wherein
R¹ represents non-substituted phenyl or phenyl which is substituted by one or more group(s) selected from halogen; hydroxyl; cyano; amino; sulfanyl; pentafluoro-λ⁶-sulfanyl; carboxaldehyde, hydroxycarbonyl, C₁-C₈-alkyl; C₁-C₈-haloalkyl; C₁-C₈-cyanoalkyl; C₁-C₈-alkyloxy; C₁-C₈-halogenalkyloxy; tri(C₁-C₈-alkyl)silyl; tri(C₁-C₈-alkyl)silyl-C₁-C₈-alkyl; C₃-C₇-cycloalkyl; C₃-C₇-halogencycloalkyl; C₃-C₇-cycloalkenyl; C₃-C₇-halogencycloalkenyl; C₄-C₁₀-cycloalkylalkyl; C₄-C₁₀-halocycloalkylalkyl; C₆-C₁₂-cycloalkylcycloalkyl; C₁-C₈-alkyl-C₃-C₇-cycloalkyl; C₁-C₈-alkoxy-C₃-C₇-cycloalkyl; tri(C₁-C₈-alkyl)silyl-C₃-C₇-cycloalkyl; C₂-C₈-alkenyl; C₂-C₈-alkynyl; C₂-C₈-alkenyloxy; C₂-C₈-halogenalkenyloxy; C₃-C₈-alkynyloxy; C₃-C₈-halogenoalkynyloxy; C₁-C₈-alkylamino; C₁-C₈-halogenalkylamino; C₁-C₈-cyanoalkoxy; C₄-C₈-cycloalkylalkoxy; C₃-C₆-cycloalkoxy; C₁-C₈-alkylsulfanyl; C₁-Cs-halogenoalkylsulfanyl; C₁-C₈-alkylcarbonyl; C₁-C₈-halogenoalkylcarbonyl; arylcarbonyl; C₃-C₈-cycloalkylcarbonyl; C₃-C₈-halogenocycloalkylcarbonyl; C₁-C₈-alkylcarbamoyl; di-C₁-Cs-alkylcarbamoyl; N-C₁-C₈-alkyloxycarbamoyl; C₁-C₈-alkoxycarbamoyl; N-C₁-C₈-alkyl-C₁-C₈-alkoxycarbamoyl; C₁-C₈-alkoxycarbonyl; C₁-C₈-halogenoalkoxycarbonyl; C₃-C₈-cycloalkoxycarbonyl; C₂-C₈-alkoxyalkylcarbonyl; C₂-C₈-halogenoalkoxyalkylcarbonyl; C₃-C₁₀-cycloalkoxyalkylcarbonyl; C₁-C₈-alkylaminocarbonyl; di-C₁-C₈-alkylaminocarbonyl; C₃-C₈-cycloalkylaminocarbonyl; C₁-C₈-alkylcarbonyloxy; C₁-C₈-halogenoalkylcarbonyloxy; C₃-C₈-cycloalkylcarbonyloxy; C₁-C₈-alkylcarbonylamino; C₁-C₈-halogenoalkylcarbonylamino; C₁-C₈-alkylaminocarbonyloxy; di-C₁-C₈-alkylaminocarbonyloxy; C₁-C₈-alkyloxycarbonyloxy; C₁-C₈-alkylsulfinyl; C₁-C₈-halogenoalkylsulfinyl; C₁-C₈-alkylsulfonyl; C₁-C₈-halogenoalkylsulfonyl; C₁-C₈-alkylsulfonyloxy; C₁-C₈-halogenoalkylsulfonyloxy; Ci-Cs-alkylaminosulfamoyl; di-C₁-C₈-alkylaminosulfamoyl; (C₁-C₈-alkoxyimino)-C₁-C₈-alkyl; (C₃-C₇-cycloalkoxyimino)-C₁-C₈-alkyl; hydroxyimino-C₁-C₈-alkyl; (C₁-C₈-alkoxyimino)-C₃-C₇-cycloalkyl; hydroxyimino-C₃-C₇-cycloalkyl; (C₁-C₈-alkylimino)-oxy; (C₁-C₈-alkylimino)-oxy-C₁-C₈-alkyl; (C₃-C₇-cycloalkylimino)-oxy-C₁-C₈-alkyl; (C₁-C₆-alkylimino)-oxy-C₃-C₇-cycloalkyl; (C₁-C₈-alkenyloxyimino)-C₁-C₈-alkyl; (C₁-C₈-alkynyloxyimino)-C₁-C₈-alkyl; (benzyloxyimino)-C₁-C₈-alkyl; C₁-C₈-alkoxyalkyl; C₁-C₈-alkylthioalkyl; C₁-C₈-alkoxyalkoxyalkyl; C₁-C₈-halogenoalkoxyalkyl; benzyl; phenyl; 5-membered heteroaryl; 6-membered heteroaryl; benzyloxy; phenyloxy; benzylsulfanyl; benzylamino; phenylsulfanyl; or phenylamino;
R² represents H, C₁-C₈-alkyl, halogen- or C₁-C₈-alkoxy-substituted or non-substituted -C(O)-C₁-C₈-alkyl;
and wherein Q has the same meaning as given in claim 1.
and its salts or N-oxides.

3. Triazole derivatives of the formula (I) according to claim 1 or 2, wherein
R¹ represents non-substituted phenyl or phenyl which is substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino; wherein the benzyl, phenyl, 5-membered heteroaryl, 6-membered heteroaryl, benzyloxy or phenyloxy may be optionally substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino.
R² represents H, C₁-C₈-alkyl, halogen- or C₁-C₈-alkoxy-substituted or non-substituted -C(O)-C₁-C₈-alkyl;
Q represents a substituted 6-membered aromatic heterocycle containing one nitrogen atom of formula (Q-I-1) to (Q-I-3) 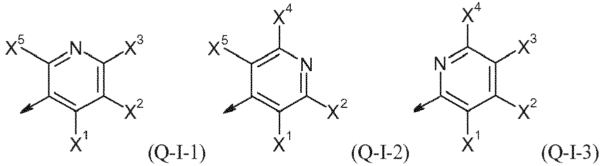 wherein X¹, X², X³, X⁴ or X⁵ independent from each other represent hydrogen, halogen, C₁-C₈-alkyl; C₁-C₈-haloalkyl; Ci-Cs-alkoxy; or C₁-C₈-halogenalkyloxy; preferably represent hydrogen or halogen;
and wherein at least one of X¹, X², X³, X⁴ or X⁵ is different from hydrogen; and its salts or N-oxides.

4. Triazole derivatives of the formula (I) according to claim 1, 2 or 3, wherein
R¹ represents non-substituted phenyl or phenyl which is substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino; wherein the benzyl, phenyl, 5-membered heteroaryl, 6-membered heteroaryl, benzyloxy or phenyloxy may be optionally substituted by one or more group(s) selected from halogen, C₁-C₈-alkyl; C₁-C₈-haloalkyl; C₁-C₈-halogenalkyloxy; C₃-C₇-cycloalkyl; C₂-C₈-alkenyl; C₂-C₈-alkynyl; C₂-C₈-alkenyloxy; C₃-C₈-alkynyloxy; C₃-C₈-halogenoalkynyloxy; C₁-C₈-alkoxy; phenyl; 5-membered heteroaryl; 6-membered heteroaryl; benzyloxy; phenyloxy; benzylsulfanyl; benzylamino; phenylsulfanyl; or phenylamino.
R² represents H, C₁-C₈-alkyl, halogen- or C₁-C₈-alkoxy-substituted or non-substituted -C(O)-C₁-C₈-alkyl;
Q represents a substituted 6-membered aromatic heterocycle containing one nitrogen atom of formula (Q-I-1) to (Q-I-2) 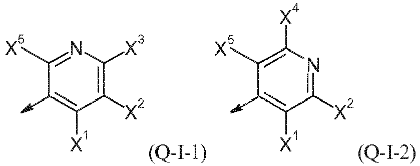 wherein X¹, X², X³, X⁴ or X⁵ independent from each other represent hydrogen, halogen, C₁-C₈-alkyl; C₁-C₈-haloalkyl; C₁-C₈-alkoxy; or C₁-C₈-halogenalkyloxy; preferably represent hydrogen or halogen;
and wherein at least one of X¹, X², X³, X⁴ or X⁵ is different from hydrogen;
and its salts or N-oxides.

5. Method for controlling harmful microorganisms in crop protection and in the protection of materials, **characterized in that** compounds of the formula (I) according to Claim 1, 2, 3 or 4 are applied to the harmful microorganisms and/or their habitat.

6. Method for controlling phytopathogenic harmful fungi, **characterized in that** compounds of the formula (I) according to Claim 1, 2, 3 or 4 are applied to the phytopathogenic harmful fungi and/or their habitat.

7. Composition for controlling harmful microorganisms in crop protection and in the protection of materials, preferably for controlling phytopathogenic harmful fungi, **characterized by** a content of at least one compound of the formula (I) according to Claim 1, 2, 3 or 4, in addition to extenders and/or surfactants.

8. Composition according to Claim 7 comprising at least one further active ingredient selected from the group of the insecticides, attractants, sterilants, bactericides, acaricides, nematicides, fungicides, growth regulators, herbicides, fertilizers, safeners and semiochemicals.

9. Use of compounds of the formula (I) according to Claim 1, 2, 3 or 4 for control of harmful microorganisms, preferably phytopathogenic harmful fungi, in crop protection and in the protection of materials.

10. Process for producing compositions for controlling harmful microorganisms, preferably for controlling phytopathogenic harmful fungi, **characterized in that** compounds of the formula (I) according to Claim 1, 2, 3 or 4 are mixed with extenders and/or surfactants.

11. Use of compounds of the formula (I) according to Claim 1, 2, 3 or 4 for treatment of transgenic plants.

12. Use of compounds of the formula (I) according to Claim 1, 2, 3 or 4 for treatment of seed and of seed of transgenic plants.

13. Epoxides of formula (XII) 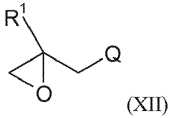 wherein
Q represents a substituted 6-membered aromatic heterocycle of formula (Q-I) containing one nitrogen atom 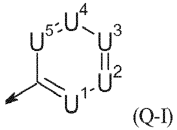
wherein
U¹ represents CX¹ or N;
wherein X¹ represents hydrogen, halogen, C₁-C₈-alkyl; C₁-C₈-haloalkyl; C₁-C₈-alkoxy; or C₁-C₈-halogenalkyloxy;
U² represents CX² or N;
wherein X² represents hydrogen, halogen, C₁-C₈-alkyl; C₁-C₈-haloalkyl; C₁-C₈-alkoxy; or C₁-C₈-halogenalkyloxy;
U³ represents CX³ or N;
wherein X³ represents hydrogen, halogen, C₁-C₈-alkyl; C₁-C₈-haloalkyl; C₁-C₈-alkoxy; or C₁-C₈-halogenalkyloxy;
U⁴ represents CX⁴ or N;
wherein X⁴ represents hydrogen, halogen, C₁-C₈-alkyl; C₁-C₈-haloalkyl; C₁-C₈-alkoxy; or C₁-C₈-halogenalkyloxy;
U⁵ represents CX⁵ or N;
wherein X⁵ represents hydrogen, halogen, C₁-C₈-alkyl; C₁-C₈-haloalkyl; C₁-C₈-alkoxy; or C₁-C₈-halogenalkyloxy;
and wherein one of U¹, U², U³, U⁴ or U⁵ represents N and wherein at least one of X¹, X², X³, X⁴ or X⁵ is different from hydrogen;;
and
R¹ represents phenyl or naphthyl; wherein the phenyl or naphthyl may be non-substituted or substituted by one or more group(s) selected from halogen; hydroxyl; cyano; amino; sulfanyl; pentafluoro-λ⁶-sulfanyl; carboxaldehyde, hydroxycarbonyl, C₁-C₈-alkyl; C₁-C₈-haloalkyl; C₁-C₈-cyanoalkyl; C₁-C₈-alkyloxy; C₁-C₈-halogenalkyloxy; tri(C₁-C₈-alkyl)silyl; tri(C₁-C₈-alkyl)silyl-C₁-C₈-alkyl; C₃-C₇-cycloalkyl; C₃-C₇-halogencycloalkyl; C₃-C₇-cycloalkenyl; C₃-C₇-halogencycloalkenyl; C₄-C₁₀-cycloalkylalkyl; C₄-C₁₀-halocycloalkylalkyl; C₆-C₁₂-cycloalkylcycloalkyl; C₁-C₈-alkyl-C₃-C₇-cycloalkyl; C₁-C₈-alkoxy-C₃-C₇-cycloalkyl; tri(C₁-C₈-alkyl)silyl-C₃-C₇-cycloalkyl; C₂-C₈-alkenyl; C₂-C₈-alkynyl; C₂-C₈-alkenyloxy; C₂-C₈-halogenalkenyloxy; C₃-C₈-alkynyloxy; C₃-C₈-halogenoalkynyloxy; C₁-C₈-alkylamino; C₁-C₈-halogenalkylamino; C₁-C₈-cyanoalkoxy; C₄-C₈-cycloalkylalkoxy; C₃-C₆-cycloalkoxy; C₁-C₈-alkylsulfanyl; C₁-C₈-halogenoalkylsulfanyl; C₁-C₈-alkylcarbonyl; C₁-C₈-halogenoalkylcarbonyl; arylcarbonyl; C₃-C₈-cycloalkylcarbonyl; C₃-C₈-halogenocycloalkylcarbonyl; C₁-C₈-alkylcarbamoyl; di-C₁-C₈-alkylcarbamoyl; N-C₁-C₈-alkyloxycarbamoyl; C₁-C₈-alkoxycarbamoyl; N-C₁-C₈-alkyl-C₁-C₈-alkoxycarbamoyl; C₁-C₈-alkoxycarbonyl; C₁-C₈-halogenoalkoxycarbonyl; C₃-C₈-cycloalkoxycarbonyl; C₂-C₈-alkoxyalkylcarbonyl; C₂-C₈-halogenoalkoxyalkylcarbonyl; C₃-C₁₀-cycloalkoxyalkylcarbonyl; C₁-C₈-alkylaminocarbonyl; di-C₁-C₈-alkylaminocarbonyl; C₃-C₈-cycloalkylaminocarbonyl; C₁-C₈-alkylcarbonyloxy; C₁-C₈-halogenoalkylcarbonyloxy; C₃-C₈-cycloalkylcarbonyloxy; C₁-C₈-alkylcarbonylamino; C₁-C₈-halogenoalkylcarbonylamino; C₁-C₈-alkylaminocarbonyloxy; di-C₁-C₈-alkylaminocarbonyloxy; C₁-C₈-alkyloxycarbonyloxy; C₁-C₈-alkylsulfinyl; C₁-C₈-halogenoalkylsulfinyl; C₁-C₈-alkylsulfonyl; C₁-C₈-halogenoalkylsulfonyl; C₁-C₈-alkylsulfonyloxy; C₁-C₈-halogenoalkylsulfonyloxy; C₁-C₈-alkylaminosulfamoyl; di-C₁-C₈-alkylaminosulfamoyl; (C₁-C₈-alkoxyimino)-C₁-C₈-alkyl; (C₃-C₇-cycloalkoxyimino)-C₁-C₈-alkyl; hydroxyimino-C₁-C₈-alkyl; (C₁-C₈-alkoxyimino)-C₃-C₇-cycloalkyl; hydroxyimino-C₃-C₇-cycloalkyl; (C₁-C₈-alkylimino)-oxy; (C₁-C₈-alkylimino)-oxy-C₁-C₈-alkyl; (C₃-C₇-cycloalkylimino)-oxy-C₁-C₈-alkyl; (C₁-C₆-alkylimino)-oxy-C₃-C₇-cycloalkyl; (C₁-C₈-alkenyloxyimino)-C₁-C₈-alkyl; (C₁-C₈-alkynyloxyimino)-C₁-C₈-alkyl; (benzyloxyimino)-C₁-C₈-alkyl; C₁-C₈-alkoxyalkyl; C₁-C₈-alkylthioalkyl; C₁-C₈-alkoxyalkoxyalkyl; C₁-C₈-halogenoalkoxyalkyl; benzyl; phenyl; 5-membered heteroaryl; 6-membered heteroaryl; benzyloxy; phenyloxy; benzylsulfanyl; benzylamino; phenylsulfanyl; or phenylamino;
and its salts or N-oxides.

## Patentansprüche

1. Triazolderivate der Formel (I) 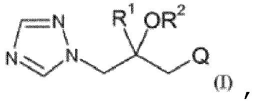 wobei
R¹ für Phenyl oder Naphthyl steht, wobei Phenyl bzw. Naphthyl unsubstituiert oder durch eine oder mehrere Gruppen ausgewählt aus Halogen, Hydroxyl, Cyano, Amino, Sulfanyl, Pentafluor-λ⁶-sulfanyl, Carboxaldehyd, Hydroxycarbonyl, C₁-C₈-Alkyl, C₁-C₈-Halogenalkyl, C₁-C₈-Cyanoalkyl, C₁-C₈-Alkyloxy, C₁-C₈-Halogenalkyloxy, Tri(C₁-C₈-alkyl) silyl, Tri(C₁-C₈-alkyl)silyl-C₁-C₈-alkyl, C₃-C₇-Cycloalkyl, C₃-C₇-Halogencycloalkyl, C₃-C₇-Cycloalkenyl, C₃-C₇-Halogencycloalkenyl, C₄-C₁₀-Cycloalkylalkyl, C₄-C₁₀-Halogencycloalkylalkyl, C₆-C₁₂-Cycloalkylcycloalkyl, C₁-C₈-Alkyl-C₃-C₇-cycloalkyl, C₁-C₈-Alkoxy-C₃-C₇-cycloalkyl, Tri (C₁-C₈-alkyl) silyl-C₃-C₇-cycloalkyl, C₂-C₈-Alkenyl, C₂-C₈-Alkinyl, C₂-C₈-Alkenyloxy, C₂-C₈-Halogenalkenyloxy, C₃-C₈-Alkinyloxy, C₃-C₈-Halogenalkinyloxy, C₁-C₈-Alkylamino, C₁-C₈-Halogenalkylamino, C₁-C₈-Cyanoalkoxy, C₄-C₈-Cycloalkylalkoxy, C₃-C₆-Cycloalkoxy, C₁-C₈-Alkylsulfanyl, C₁-C₈-Halogenalkylsulfanyl, C₁-C₈-Alkylcarbonyl, C₁-C₈-Halogenalkylcarbonyl, Arylcarbonyl, C₃-C₈-Cycloalkylcarbonyl, C₃-C₈-Halogencycloalkylcarbonyl, C₁-C₈-Alkylcarbamoyl, Di-C₁-C₈-alkylcarbamoyl, N-C₁-C₈-Alkyloxy-carbamoyl, C₁-C₈-Alkoxycarbamoyl, N-C₁-C₈-Alkyl-C₁-C₈-alkoxycarbamoyl, C₁-C₈-Alkoxycarbonyl, C₁-C₈-Halogenalkoxycarbonyl, C₃-C₈-Cycloalkoxycarbonyl, C₂-C₈-Alkoxyalkylcarbonyl, C₂-C₈-Halogenalkoxyalkylcarbonyl, C₃-C₁₀-Cycloalkoxyalkylcarbonyl, C₁-C₈-Alkylaminocarbonyl, Di-C₁-C₈-alkylaminocarbonyl, C₃-C₈-Cycloalkylaminocarbonyl, C₁-C₈-Alkylcarbonyloxy, C₁-C₈-Halogenalkylcarbonyloxy, C₃-C₈-Cycloalkylcarbonyloxy, C₁-C₈-Alkylcarbonylamino, C₁-C₈-Halogenalkylcarbonylamino, C₁-C₈-Alkylaminocarbonyloxy, Di-C₁-C₈-alkylaminocarbonyloxy, C₁-C₈-Alkyloxycarbonyloxy, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyloxy, C₁-C₈-Halogenalkylsulfonyloxy, C₁-C₈-Alkylaminosulfamoyl, Di-C₁-C₈-alkylaminosulfamoyl, (C₁-C₈-Alkoxyimino)-C₁-C₈-alkyl, (C₃-C₇-Cycloalkoxyimino)-C₁-C₈-alkyl, Hydroxyimino-C₁-C₈-alkyl, (C₁-C₈-Alkoxyimino)-C₃-C₇-cycloalkyl, Hydroxyimino-C₃-C₇-cycloalkyl, (C₁-C₈-Alkylimino)oxy, (C₁-C₈-Alkylimino)oxy-C₁-C₈-alkyl, (C₃-C₇-Cycloalkylimino)oxy-C₁-C₈-alkyl, (C₁-C₆-Alkylimino)-oxy-C₃-C₇-cycloalkyl, (C₁-C₈-Alkenyloxyimino)-C₁-C₈-alkyl, (C₁-C₈-Alkinyloxyimino)-C₁-C₈-alkyl, (Benzyloxyimino)-C₁-C₈-alkyl, C₁-C₈-Alkoxyalkyl, C₁-C₈-Alkylthioalkyl, C₁-C₈-Alkoxyalkoxyalkyl, C₁-C₈-Halogenalkoxyalkyl, Benzyl, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert sein kann,
R² für H, C₁-C₈-Alkyl, -Si(R^{3a})(R^{3b})(R^{3c}), -P(O)(OH)₂, -CH₂-O-P(O)(OH)₂, -C(O)-C₁-C₈-Alkyl, -C(O)-C₃-C₇-Cycloalkyl, -C(O)NH-C₁-C₈-Alkyl, -C(O)N-Di-C₁-C₈-alkyl, -C(O)O-C₁-C₈-Alkyl steht, wobei -C(O)-C₁-C₈-Alkyl, -C(O)-C₃-C₇-Cycloalkyl, -C(O)NH-C₁-C₈-Alkyl, -C(O)N-Di-C₁-C₈-alkyl oder -C(O)O-C₁-C₈-Alkyl unsubstituiert oder durch eine oder mehrere aus Halogen und C₁-C₈-Alkoxy ausgewählte Gruppen substituiert sein können,
wobei R^{3a}, R^{3b}, R^{3c} unabhängig voneinander für Phenyl oder C₁-C₈-Alkyl stehen,
Q für einen substituierten 6-gliedrigen aromatischen Heterocyclus der Formel (Q-I) mit einem Stickstoffatom steht 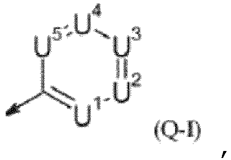
wobei
U¹ für CX¹ oder N steht, wobei X¹ für Wasserstoff, Halogen, C₁-C₈-Alkyl, C₁-C₈-Halogenalkyl, C₁-C₈-Alkoxy oder C₁-C₈-Halogenalkyloxy steht,
U² für CX² oder N steht,
wobei X² für Wasserstoff, Halogen, C₁-C₈-Alkyl, C₁-C₈-Halogenalkyl, C₁-C₈-Alkoxy oder C₁-C₈-Halogenalkyloxy steht,
U³ für CX³ oder N steht,
wobei X³ für Wasserstoff, Halogen, C₁-C₈-Alkyl, C₁-C₈-Halogenalkyl, C₁-C₈-Alkoxy oder C₁-C₈-Halogenalkyloxy steht,
U⁴ für CX⁴ oder N steht,
wobei X⁴ für Wasserstoff, Halogen, C₁-C₈-Alkyl, C₁-C₈-Halogenalkyl, C₁-C₈-Alkoxy oder C₁-C₈-Halogenalkyloxy steht,
U⁵ für CX⁵ oder N steht,
wobei X⁵ für Wasserstoff, Halogen, C₁-C₈-Alkyl, C₁-C₈-Halogenalkyl, C₁-C₈-Alkoxy oder C₁-C₈-Halogenalkyloxy steht,
und wobei einer der Reste U¹, U², U³, U⁴ oder U⁵ für N steht und wobei mindestens einer der Reste X¹, X², X³, X⁴ oder X⁵ von Wasserstoff verschieden ist,
und ihre Salze und N-Oxide.

2. Triazolderivate der Formel (I) nach Anspruch 1, wobei
R¹ für unsubstituiertes Phenyl oder Phenyl, welches durch eine oder mehrere Gruppen ausgewählt aus Halogen, Hydroxyl, Cyano, Amino, Sulfanyl, Pentafluor-λ⁶-sulfanyl, Carboxaldehyd, Hydroxycarbonyl, C₁-C₈-Alkyl, C₁-C₈-Halogenalkyl, C₁-C₈-Cyanoalkyl, C₁-C₈-Alkyloxy, C₁-C₈-Halogenalkyloxy, Tri(C₁-C₈-alkyl)silyl, Tri(C₁-C₈-alkyl)silyl-C₁-C₈-alkyl, C₃-C₇-Cycloalkyl, C₃-C₇-Halogencycloalkyl, C₃-C₇-Cycloalkenyl, C₃-C₇-Halogencycloalkenyl, C₄-C₁₀-Cycloalkylalkyl, C₄-C₁₀-Halogencycloalkylalkyl, C₆-C₁₂-Cycloalkylcycloalkyl, C₁-C₈-Alkyl-C₃-C₇-cycloalkyl, C₁-C₈-Alkoxy-C₃-C₇-cycloalkyl, Tri(C₁-C₈-alkyl)silyl-C₃-C₇-cycloalkyl, C₂-C₈-Alkenyl, C₂-C₈-Alkinyl, C₂-C₈-Alkenyloxy, C₂-C₈-Halogenalkenyloxy, C₃-C₈-Alkinyloxy, C₃-C₈-Halogenalkinyloxy, C₁-C₈-Alkylamino, C₁-C₈-Halogenalkylamino, C₁-C₈-Cyanoalkoxy, C₄-C₈-Cycloalkylalkoxy, C₃-C₆-Cycloalkoxy, C₁-C₈-Alkylsulfanyl, C₁-C₈-Halogenalkylsulfanyl, C₁-C₈-Alkylcarbonyl, C₁-C₈-Halogenalkylcarbonyl, Arylcarbonyl, C₃-C₈-Cycloalkylcarbonyl, C₃-C₈-Halogencycloalkylcarbonyl, C₁-C₈-Alkylcarbamoyl, Di-C₁-C₈-alkylcarbamoyl, N-C₁-C₈-Alkyloxycarbamoyl, C₁-C₈-Alkoxycarbamoyl, N-C₁-C₈-Alkyl-C₁-C₈-alkoxycarbamoyl, C₁-C₈-Alkoxycarbonyl, C₁-C₈-Halogenalkoxycarbonyl, C₃-C₈-Cycloalkoxycarbonyl, C₂-C₈-Alkoxyalkylcarbonyl, C₂-C₈-Halogenalkoxyalkylcarbonyl, C₃-C₁₀-Cycloalkoxyalkylcarbonyl, C₁-C₈-Alkylaminocarbonyl, Di-C₁-C₈-alkylaminocarbonyl, C₃-C₈-Cycloalkylaminocarbonyl, C₁-C₈-Alkylcarbonyloxy, C₁-C₈-Halogenalkylcarbonyloxy, C₃-C₈-Cycloalkylcarbonyloxy, C₁-C₈-Alkylcarbonylamino, C₁-C₈-Halogenalkylcarbonylamino, C₁-C₈-Alkylaminocarbonyloxy, Di-C₁-C₈-alkylaminocarbonyloxy, C₁-C₈-Alkyloxycarbonyloxy, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyloxy, C₁-C₈-Halogenalkylsulfonyloxy, C₁-C₈-Alkylaminosulfamoyl, Di-C₁-C₈-alkylaminosulfamoyl, (C₁-C₈-Alkoxyimino)-C₁-C₈-alkyl, (C₃-C₇-Cycloalkoxyimino) -C₁-C₈-alkyl, Hydroxyimino-C₁-C₈-alkyl, (C₁-C₈-Alkoxyimino)-C₃-C₇-cycloalkyl, Hydroxyimino-C₃-C₇-cycloalkyl, (C₁-C₈-Alkylimino)oxy, (C₁-C₈-Alkylimino) oxy-C₁-C₈-alkyl, (C₃-C₇-Cycloalkylimino) oxy-C₁-C₈-alkyl, (C₁-C₆-Alkylimino) oxy-C₃-C₇-cycloalkyl, (C₁-C₈-Alkenyloxyimino)-C₁-C₈-alkyl, (C₁-C₈-Alkinyloxyimino)-C₁-C₈-alkyl, (Benzyloxyimino)-C₁-C₈-alkyl, C₁-C₈-Alkoxyalkyl, C₁-C₈-Alkylthioalkyl, C₁-C₈-Alkoxyalkoxyalkyl, C₁-C₈-Halogenalkoxyalkyl, Benzyl, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert ist, steht,
R² für H, C₁-C₈-Alkyl, Halogen- oder C₁-C₈-Alkoxy-substituiertes oder unsubstituiertes-C(O)-C₁-C₈-Alkyl steht,
und wobei Q die gleiche Bedeutung wie nach Anspruch 1 hat,
und ihre Salze und N-Oxide.

3. Triazolderivate der Formel (I) nach Anspruch 1 oder 2, wobei
R¹ für unsubstituiertes Phenyl oder Phenyl, das durch eine oder mehrere Gruppen ausgewählt aus Halogen, C₁-C₈-Alkyl, C₁-C₈-Halogenalkyl,C₁-C₈-Halogenalkyloxy, C₃-C₇-Cycloalkyl, C₂-C₈-Alkenyl, C₂-C₈-Alkinyl, C₂-C₈-Alkenyloxy, C₃-C₈-Alkinyloxy, C₃-C₈-Halogenalkinyloxy, C₁-C₈-Alkoxy, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert ist, steht, wobei Benzyl, Phenyl, 5-gliedriges Heteroaryl, 6-gliedriges Heteroaryl, Benzyloxy und Phenyloxy gegebenenfalls durch eine oder mehrere Gruppen ausgewählt aus Halogen, C₁-C₈-Alkyl, C₁-C₈-Halogenalkyl,C₁-C₈-Halogenalkyloxy, C₃-C₇-Cycloalkyl, C₂-C₈-Alkenyl, C₂-C₈-Alkinyl, C₂-C₈-Alkenyloxy, C₃-C₈-Alkinyloxy, C₃-C₈-Halogenalkinyloxy, C₁-C₈-Alkoxy, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert sein können,
R² für H, C₁-C₈-Alkyl, Halogen- oder C₁-C₈-Alkoxy-substituiertes oder unsubstituiertes-C(O)-C₁-C₈-Alkyl steht,
Q für einen substituierten 6-gliedrigen aromatischen Heterocyclus mit einem Stickstoffatom der Formel (Q-I-1) bis (Q-I-3) steht 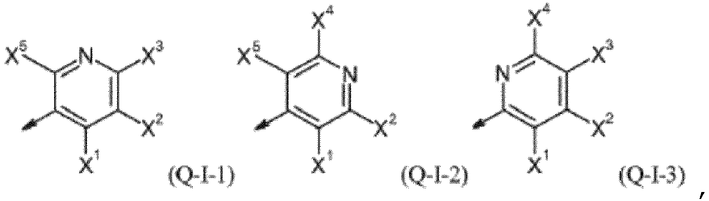 wobei X¹, X², X³, X⁴ und X⁵ unabhängig voneinander jeweils für Wasserstoff, Halogen, C₁-C₈-Alkyl, C₁-C₈-Halogenalkyl, C₁-C₈-Alkoxy oder C₁-C₈-Halogenalkyloxy stehen und vorzugsweise für Wasserstoff oder Halogen stehen,
und wobei mindestens einer der Reste X¹, X², X³, X⁴ und X⁵ von Wasserstoff verschieden ist,
und deren Salze und N-Oxide.

4. Triazolderivate der Formel (I) nach Anspruch 1, 2 oder 3, wobei
R¹ für unsubstituiertes Phenyl oder Phenyl, das durch eine oder mehrere Gruppen ausgewählt aus Halogen, C₁-C₈-Alkyl, C₁-C₈-Halogenalkyl, C₁-C₈-Halogenalkyloxy, C₃-C₇-Cycloalkyl, C₂-C₈-Alkenyl, C₂-C₈-Alkinyl, C₂-C₈-Alkenyloxy, C₃-C₈-Alkinyloxy, C₃-C₈-Halogenalkinyloxy, C₁-C₈-Alkoxy, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert ist, steht, wobei Benzyl, Phenyl, 5-gliedriges Heteroaryl, 6-gliedriges Heteroaryl, Benzyloxy und Phenyloxy gegebenenfalls durch eine oder mehrere Gruppen ausgewählt aus Halogen, C₁-C₈-Alkyl, C₁-C₈-Halogenalkyl, C₁-C₈-Halogenalkyloxy, C₃-C₇-Cycloalkyl, C₂-C₈-Alkenyl, C₂-C₈-Alkinyl, C₂-C₈-Alkenyloxy, C₃-C₈-Alkinyloxy, C₃-C₈-Halogenalkinyloxy, C₁-C₈-Alkoxy, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert sein können,
R² für H, C₁-C₈-Alkyl, Halogen- oder C₁-C₈-Alkoxy-substituiertes oder unsubstituiertes-C(O)-C₁-C₈-Alkyl steht,
Q für einen substituierten 6-gliedrigen aromatischen Heterocyclus mit einem Stickstoffatom der Formel (Q-I-1) bis (Q-I-2) steht 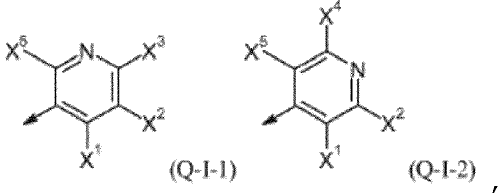 wobei X¹, X², X³, X⁴ und X⁵ unabhängig voneinander jeweils für Wasserstoff, Halogen, C₁-C₈-Alkyl, C₁-C₈-Halogenalkyl,C₁-C₈-Alkoxy oder C₁-C₈-Halogenalkyloxy stehen und vorzugsweise für Wasserstoff oder Halogen stehen,
und wobei mindestens einer der Reste X¹, X², X³, X⁴ und X⁵ von Wasserstoff verschieden ist,
und deren Salze und N-Oxide.

5. Verfahren zur Bekämpfung von schädlichen Mikroorganismen im Pflanzenschutz und im Materialschutz, **dadurch gekennzeichnet, dass** man Verbindungen der Formel (I) nach Anspruch 1, 2, 3 oder 4, auf die schädlichen Mikroorganismen und/oder ihren Lebensraum ausbringt.

6. Verfahren zur Bekämpfung von phytopathogenen Schadpilzen, **dadurch gekennzeichnet, dass** man Verbindungen der Formel (I) nach Anspruch 1, 2, 3 oder 4 auf die phytopathogenen Schadpilze und/oder ihren Lebensraum ausbringt.

7. Zusammensetzung zur Bekämpfung von schädlichen Mikroorganismen im Pflanzenschutz und im Materialschutz, vorzugsweise zur Bekämpfung phytopathogener Schadpilze, **gekennzeichnet durch** einen Gehalt an mindestens einer Verbindung der Formel (I) nach Anspruch 1, 2, 3 oder 4, zusätzlich zu Streckmitteln und/oder Tensiden.

8. Zusammensetzung nach Anspruch 7, umfassend mindestens einen weiteren Wirkstoff ausgewählt aus der Gruppe der Insektizide, Lockstoffe, Sterilantien, Bakterizide, Akarizide, Nematizide, Fungizide, Wachstumsregulatoren, Herbizide, Düngemittel, Safener und Semiochemicals.

9. Verwendung von Verbindungen der Formel (I) nach Anspruch 1, 2, 3 oder 4 zur Bekämpfung von schädlichen Mikroorganismen, vorzugsweise phytopathogenen Schadpilzen, im Pflanzenschutz und im Materialschutz.

10. Verfahren zur Herstellung von Zusammensetzungen zur Bekämpfung von schädlichen Mikroorganismen, vorzugsweise zur Bekämpfung von phytopathogenen Schadpilzen, **dadurch gekennzeichnet, dass** man Verbindungen der Formel (I) nach Anspruch 1, 2, 3 oder 4 mit Streckmitteln und/oder Tensiden mischt.

11. Verwendung von Verbindungen der Formel (I) nach Anspruch 1, 2, 3 oder 4 zur Behandlung transgener Pflanzen.

12. Verwendung von Verbindungen der Formel (I) nach Anspruch 1, 2, 3 oder 4 zur Behandlung von Saatgut und von Saatgut transgener Pflanzen.

13. Epoxide der Formel (XII) 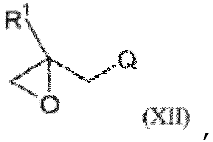 wobei
Q für einen substituierten 6-gliedrigen aromatischen Heterocyclus der Formel (Q-I) mit einem Stickstoffatom steht 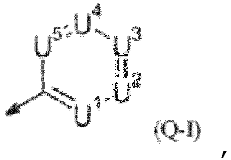 wobei
U¹ für CX¹ oder N steht,
wobei X¹ für Wasserstoff, Halogen, C₁-C₈-Alkyl, C₁-C₈-Halogenalkyl, C₁-C₈-Alkoxy oder C₁-C₈-Halogenalkyloxy steht,
U² für CX² oder N steht,
wobei X² für Wasserstoff, Halogen, C₁-C₈-Alkyl, C₁-C₈-Halogenalkyl, C₁-C₈-Alkoxy oder C₁-C₈-Halogenalkyloxy steht,
U³ für CX³ oder N steht,
wobei X³ für Wasserstoff, Halogen, C₁-C₈-Alkyl, C₁-C₈-Halogenalkyl, C₁-C₈-Alkoxy oder C₁-C₈-Halogenalkyloxy steht,
U⁴ für CX⁴ oder N steht,
wobei X⁴ für Wasserstoff, Halogen, C₁-C₈-Alkyl, C₁-C₈-Halogenalkyl, C₁-C₈-Alkoxy oder C₁-C₈-Halogenalkyloxy steht,
U⁵ für CX⁵ oder N steht,
wobei X⁵ für Wasserstoff, Halogen, C₁-C₈-Alkyl, C₁-C₈-Halogenalkyl, C₁-C₈-Alkoxy oder C₁-C₈-Halogenalkyloxy steht,
und wobei einer der Reste U¹, U², U³, U⁴ oder U⁵ für N steht und wobei mindestens einer der Reste X¹, X², X³, X⁴ oder X⁵ von Wasserstoff verschieden ist, und
R¹ für Phenyl oder Naphthyl steht, wobei Phenyl bzw. Naphthyl unsubstituiert oder durch eine oder mehrere Gruppen ausgewählt aus Halogen, Hydroxyl, Cyano, Amino, Sulfanyl, Pentafluor-λ⁶-sulfanyl, Carboxaldehyd, Hydroxycarbonyl, C₁-C₈-Alkyl, C₁-C₈-Halogenalkyl, C₁-C₈-Cyanoalkyl, C₁-C₈-Alkyloxy, C₁-C₈-Halogenalkyloxy, Tri(C₁-C₈-alkyl)silyl, Tri(C₁-C₈-alkyl)silyl-C₁-C₈-alkyl, C₃-C₇-Cycloalkyl, C₃-C₇-Halogencycloalkyl, C₃-C₇-Cycloalkenyl, C₃-C₇-Halogencycloalkenyl, C₄-C₁₀-Cycloalkylalkyl, C₄-C₁₀-Halogencycloalkylalkyl, C₆-C₁₂-Cycloalkylcycloalkyl, C₁-C₈-Alkyl-C₃-C₇-cycloalkyl, C₁-C₈-Alkoxy-C₃-C₇-cycloalkyl, Tri (C₁-C₈-alkyl) silyl-C₃-C₇-cycloalkyl, C₂-C₈-Alkenyl, C₂-C₈-Alkinyl, C₂-C₈-Alkenyloxy, C₂-C₈-Halogenalkenyloxy, C₃-C₈-Alkinyloxy, C₃-C₈-Halogenalkinyloxy, C₁-C₈-Alkylamino, C₁-C₈-Halogenalkylamino, C₁-C₈-Cyanoalkoxy, C₄-C₈-Cycloalkylalkoxy, C₃-C₆-Cycloalkoxy, C₁-C₈-Alkylsulfanyl, C₁-C₈-Halogenalkylsulfanyl, C₁-C₈-Alkylcarbonyl, C₁-C₈-Halogenalkylcarbonyl, Arylcarbonyl, C₃-C₈-Cycloalkylcarbonyl, C₃-C₈-Halogencycloalkylcarbonyl, C₁-C₈-Alkylcarbamoyl, Di-C₁-C₈-alkylcarbamoyl, N-C₁-C₈-Alkyloxycarbamoyl, C₁-C₈-Alkoxycarbamoyl, N-C₁-C₈-Alkyl-C₁-C₈-alkoxycarbamoyl, C₁-C₈-Alkoxycarbonyl, C₁-C₈-Halogenalkoxycarbonyl, C₃-C₈-Cycloalkoxycarbonyl, C₂-C₈-Alkoxyalkylcarbonyl, C₂-C₈-Halogenalkoxyalkylcarbonyl, C₃-C₁₀-cycloalkoxyalkylcarbonyl, C₁-C₈-Alkylaminocarbonyl, Di-C₁-C₈-alkylaminocarbonyl, C₃-C₈-Cycloalkylaminocarbonyl, C₁-C₈-Alkylcarbonyloxy, C₁-C₈-Halogenalkylcarbonyloxy, C₃-C₈-Cycloalkylcarbonyloxy, C₁-C₈-Alkylcarbonylamino, C₁-C₈-Halogenalkylcarbonylamino, C₁-C₈-Alkylaminocarbonyloxy, Di-C₁-C₈-alkylaminocarbonyloxy, C₁-C₈-Alkyloxycarbonyloxy, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyloxy, C₁-C₈-Halogenalkylsulfonyloxy, C₁-C₈-Alkylaminosulfamoyl, Di-C₁-C₈-alkylaminosulfamoyl, (C₁-C₈-Alkoxyimino)-C₁-C₈-alkyl, (C₃-C₇-Cycloalkoxyimino)-C₁-C₈-alkyl, Hydroxyimino-C₁-C₈-alkyl, (C₁-C₈-Alkoxyimino)-C₃-C₇-cycloalkyl, Hydroxyimino-C₃-C₇-cycloalkyl, (C₁-C₈-Alkylimino) oxy, (C₁-C₈-Alkylimino) oxy-C₁-C₈-alkyl, (C₃-C₇-Cycloalkylimino) oxy-C₁-C₈-alkyl, (C₁-C₆-Alkylimino)-oxy-C₃-C₇-cycloalkyl, (C₁-C₈-Alkenyloxyimino)-C₁-C₈-alkyl, (C₁-C₈-Alkinyloxyimino) -C₁-C₈-alkyl, (Benzyloxyimino) -C₁-C₈-alkyl, C₁-C₈-Alkoxyalkyl, C₁-C₈-Alkylthioalkyl, C₁-C₈-Alkoxyalkoxyalkyl, C₁-C₈-Halogenalkoxyalkyl, Benzyl, Phenyl, 5-gliedrigem Heteroaryl, 6-gliedrigem Heteroaryl, Benzyloxy, Phenyloxy, Benzylsulfanyl, Benzylamino, Phenylsulfanyl und Phenylamino substituiert sein kann,
und deren Salze und N-Oxide.

## Revendications

1. Dérivés de triazole de formule (I) 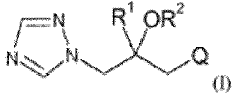 dans lesquels
R¹ représente phényle ou naphtyle ; où phényle ou naphtyle peut être non substitué ou substitué par un ou plusieurs groupements choisis parmi halogène ; hydroxyle ; cyano ; amino ; sulfanyle ; pentafluoro-λ⁶-sulfanyle ; carboxaldéhyde, hydroxycarbonyle, C₁-C₈-alkyle ; C₁-C₈-halogénoalkyle ; C₁-C₈-cyanoalkyle ; C₁-C₈-alkyloxy ; C₁-C₈-halogénoalkyloxy ; tri (C₁-C₈-alkyl)silyle ; tri (C₁-C₈-alkyl) silyl-C₁-C₈-alkyle ; C₃-C₇-cycloalkyle ; C₃-C₇-halogénocycloalkyle ; C₃-C₇-cycloalcényle ; C₃-C₇-halogénocycloalcényle ; C₄-C₁₀-cycloalkylalkyle ; C₄-C₁₀-halogénocycloalkylalkyle ; C₆-C₁₂-cycloalkylcycloalkyle ; C₁-C₈-alkyl-C₃-C₇-cycloalkyle ; C₁-C₈-alcoxy-C₃-C₇-cycloalkyle ; tri(C₁-C₈-alkyl) silyl-C₃-C₇-cycloalkyle ; C₂-C₈-alcényle ; C₂-C₈-alcynyle ; C₂-C₈-alcényloxy ; C₂-C₈-halogénoalcényloxy ; C₃-C₈-alcynyloxy ; C₃-C₈-halogénoalcynyloxy ; C₁-C₈-alkylamino ; C₁-C₈-halogénoalkylamino ; C₁-C₈-cyanoalcoxy ; C₄-C₈-cycloalkylalcoxy ; C₃-C₆-cycloalcoxy ; C₁-C₈-alkylsulfanyle ; C₁-C₈-halogénoalkylsulfanyle ; C₁-C₈-alkylcarbonyle ; C₁-C₈-halogénoalkylcarbonyle ; arylcarbonyle ; C₃-C₈-cycloalkylcarbonyle ; C₃-C₈-halogénocycloalkylcarbonyle ; C₁-C₈-alkylcarbamoyle ; di-C₁-C₈-alkylcarbamoyle ; N-C₁-C₈-alkyloxycarbamoyle ; C₁-C₈-alcoxycarbamoyle ; N-C₁-C₈-alkyl-C₁-C₈-alcoxycarbamoyle ; C₁-C₈-alcoxycarbonyle ; C₁-C₈-halogénoalcoxycarbonyle ; C₃-C₈-cycloalcoxycarbonyle ; C₂-C₈-alcoxyalkylcarbonyle ; C₂-C₈-halogénoalcoxyalkylcarbonyle ; C₃-C₁₀-cycloalcoxyalkylcarbonyle ; C₁-C₈-alkylaminocarbonyle ; di-C₁-C₈-alkylaminocarbonyle ; C₃-C₈-cycloalkylaminocarbonyle ; C₁-C₈-alkylcarbonyloxy ; C₁-C₈-halogénoalkylcarbonyloxy ; C₃-C₈-cycloalkylcarbonyloxy ; C₁-C₈-alkylcarbonylamino ; C₁-C₈-halogénoalkylcarbonylamino ; C₁-C₈-alkylaminocarbonyloxy ; di-C₁-C₈-alkylaminocarbonyloxy ; C₁-C₈-alkyloxycarbonyloxy ; C₁-C₈-alkylsulfinyle ; C₁-C₈-halogénoalkylsulfinyle ; C₁-C₈-alkylsulfonyle ; C₁-C₈-halogénoalkylsulfonyle ; C₁-C₈-alkylsulfonyloxy ; C₁-C₈-halogénoalkylsulfonyloxy ; C₁-C₈-alkylaminosulfamoyle ; di-C₁-C₈-alkylaminosulfamoyle ; (C₁-C₈-alcoxyimino)-C₁-C₈-alkyle ; (C₃-C₇-cycloalcoxyimino)-C₁-C₈-alkyle ; hydroxyimino-C₁-C₈-alkyle ; (C₁-C₈-alcoxyimino) -C₃-C₇-cycloalkyle ; hydroxyimino-C₃-C₇-cycloalkyle ; (C₁-C₈-alkylimino)-oxy ; (C₁-C₈-alkylimino)-oxy-C₁-C₈-alkyle ; (C₃-C₇-cycloalkylimino)-oxy-C₁-C₈-alkyle ; (C₁-C₆-alkylimino)-oxy-C₃-C₇-cycloalkyle ; (C₁-C₈-alcényloxyimino)-C₁-C₈-alkyle ; (C₁-C₈-alcynyloxyimino)-C₁-C₈-alkyle ; (benzyloxyimino)-C₁-C₈-alkyle ; C₁-C₈-alcoxyalkyle ; C₁-C₈-alkylthioalkyle ; C₁-C₈-alcoxyalcoxyalkyle ; C₁-C₈-halogénoalcoxyalkyle ; benzyle ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ;
R² représente H, C₁-C₈-alkyle, -Si(R^{3a})(R^{3b})(R^{3c}),-P(O)(OH)₂, -CH₂-O-P(O)(OH)₂, -C(O)-C₁-C₈-alkyle, -C(O)-C₃-C₇-cycloalkyle, -C(O)NH-C₁-C₈-alkyle ; -C(O)N-di-C₁-C₈-alkyle ; -C(O)O-C₁-C₈-alkyle ; où -C(O)-C₁-C₈-alkyle, -C(O)-C₃-C₇-cycloalkyle, -C(O)NH-C₁-C₈-alkyle ; -C(O)N-di-C₁-C₈-alkyle ou -C(O)O-C₁-C₈-alkyle peuvent être non substitués ou substitués par un ou plusieurs groupements choisis parmi halogène ou C₁-C₈-alcoxy ;
où R^{3a}, R^{3b}, R^{3c} représentent, indépendamment les uns des autres, phényle ou C₁-C₈-alkyle ;
Q représente un hétérocycle aromatique de 6 chaînons substitué de formule (Q-I) contenant un atome d'azote 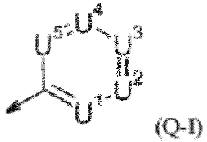
où
U¹ représente CX¹ ou N ;
où X¹ représente hydrogène, halogène, C₁-C₈-alkyle ; C₁-C₈-halogénoalkyle ; C₁-C₈-alcoxy ; ou C₁-C₈-halogénoalkyloxy ;
U² représente CX² ou N ;
où X² représente hydrogène, halogène, C₁-C₈-alkyle ; C₁-C₈-halogénoalkyle ; C₁-C₈-alcoxy ; ou C₁-C₈-halogénoalkyloxy ;
U³ représente CX³ ou N ;
où X³ représente hydrogène, halogène, C₁-C₈-alkyle ; C₁-C₈-halogénoalkyle ; C₁-C₈-alcoxy ; ou C₁-C₈-halogénoalkyloxy ;
U⁴ représente CX⁴ ou N ;
où X⁴ représente hydrogène, halogène, C₁-C₈-alkyle ; C₁-C₈-halogénoalkyle ; C₁-C₈-alcoxy ; ou C₁-C₈-halogénoalkyloxy ;
U⁵ représente CX⁵ ou N ;
où X⁵ représente hydrogène, halogène, C₁-C₈-alkyle ; C₁-C₈-halogénoalkyle ; C₁-C₈-alcoxy ; ou C₁-C₈-halogénoalkyloxy ; et
où l'un parmi U¹, U², U³, U⁴ et U⁵ représente N et où au moins l'un parmi X¹, X², X³, X⁴ et X⁵ est autre qu'hydrogène ;
ainsi que leurs sels ou N-oxydes.

2. Dérivés de triazole de formule (I) selon la revendication 1, dans lesquels
R¹ représente phényle non substitué ou phényle qui est substitué par un ou plusieurs groupements choisis parmi halogène ; hydroxyle ; cyano ; amino ; sulfanyle ; pentafluoro-λ⁶-sulfanyle ; carboxaldéhyde, hydroxycarbonyle, C₁-C₈-alkyle ; C₁-C₈-halogénoalkyle ; C₁-C₈-cyanoalkyle ; C₁-C₈-alkyloxy ; C₁-C₈-halogénoalkyloxy ; tri (C₁-C₈-alkyl) silyle ; tri (C₁-C₈-alkyl) silyl-C₁-C₈-alkyle ; C₃-C₇-cycloalkyle ; C₃-C₇-halogénocycloalkyle ; C₃-C₇-cycloalcényle ; C₃-C₇-halogénocycloalcényle ; C₄-C₁₀-cycloalkylalkyle ; C₄-C₁₀-halogénocycloalkylalkyle ; C₆-C₁₂-cycloalkylcycloalkyle; C₁-C₈-alkyl-C₃-C₇-cycloalkyle ; C₁-C₈-alcoxy-C₃-C₇-cycloalkyle ; tri (C₁-C₈-alkyl) silyl-C₃-C₇-cycloalkyle ; C₂-C₈-alcényle ; C₂-C₈-alcynyle ; C₂-C₈-alcényloxy ; C₂-C₈-halogénoalcényloxy ; C₃-C₈-alcynyloxy ; C₃-C₈-halogénoalcynyloxy ; C₁-C₈-alkylamino ; C₁-C₈-halogénoalkylamino ; C₁-C₈-cyanoalcoxy ; C₄-C₈-cycloalkylalcoxy ; C₃-C₆-cycloalcoxy ; C₁-C₈-alkylsulfanyle ; C₁-C₈-halogénoalkylsulfanyle ; C₁-C₈-alkylcarbonyle ; C₁-C₈-halogénoalkylcarbonyle ; arylcarbonyle ; C₃-C₈-cycloalkylcarbonyle ; C₃-C₈-halogénocycloalkylcarbonyle ; C₁-C₈-alkylcarbamoyle ; di-C₁-C₈-alkylcarbamoyle ; N-C₁-C₈-alkyloxycarbamoyle ; C₁-C₈-alcoxycarbamoyle ; N-C₁-C₈-alkyl-C₁-C₈-alcoxycarbamoyle ; C₁-C₈-alcoxycarbonyle ; C₁-C₈-halogénoalcoxycarbonyle ; C₃-C₈-cycloalcoxycarbonyle ; C₂-C₈-alcoxyalkylcarbonyle ; C₂-C₈-halogénoalcoxyalkylcarbonyle ; C₃-C₁₀-cycloalcoxyalkylcarbonyle ; C₁-C₈-alkylaminocarbonyle ; di-C₁-C₈-alkylaminocarbonyle ; C₃-C₈-cycloalkylaminocarbonyle ; C₁-C₈-alkylcarbonyloxy ; C₁-C₈-halogénoalkylcarbonyloxy ; C₃-C₈-cycloalkylcarbonyloxy ; C₁-C₈-alkylcarbonylamino ; C₁-C₈-halogénoalkylcarbonylamino ; C₁-C₈-alkylaminocarbonyloxy ; di-C₁-C₈-alkylaminocarbonyloxy ; C₁-C₈-alkyloxycarbonyloxy ; C₁-C₈-alkylsulfinyle ; C₁-C₈-halogénoalkylsulfinyle ; C₁-C₈-alkylsulfonyle ; C₁-C₈-halogénoalkylsulfonyle ; C₁-C₈-alkylsulfonyloxy ; C₁-C₈-halogénoalkylsulfonyloxy ; C₁-C₈-alkylaminosulfamoyle ; di-C₁-C₈-alkylaminosulfamoyle ; (C₁-C₈-alcoxyimino)-C₁-C₈-alkyle ; (C₃-C₇-cycloalcoxyimino)-C₁-C₈-alkyle ; hydroxyimino-C₁-C₈-alkyle ; (C₁-C₈-alcoxyimino)-C₃-C₇-cycloalkyle ; hydroxyimino-C₃-C₇-cycloalkyle ; (C₁-C₈-alkylimino)-oxy ; (C₁-C₈-alkylimino)-oxy-C₁-C₈-alkyle ; (C₃-C₇-cycloalkylimino)-oxy-C₁-C₈-alkyle ; (C₁-C₆-alkylimino)-oxy-C₃-C₇-cycloalkyle ; (C₁-C₈-alcényloxyimino)-C₁-C₈-alkyle ; (C₁-C₈-alcynyloxyimino)-C₁-C₈-alkyle ; (benzyloxyimino)-C₁-C₈-alkyle ; C₁-C₈-alcoxyalkyle ; C₁-C₈-alkylthioalkyle ; C₁-C₈-alcoxyalcoxyalkyle ; C₁-C₈-halogénoalcoxyalkyle ; benzyle ; phényle ; hétéroaryle de 5 chaînons ; hétéroaryle de 6 chaînons ; benzyloxy ; phényloxy ;
benzylsulfanyle ; benzylamino ; phénylsulfanyle ; ou phénylamino ;
R² représente H, C₁-C₈-alkyle ; -C(O)-C₁-C₈-alkyle substitué par halogène ou C₁-C₈-alcoxy ou non substitué ;
et où Q revêt la même signification que celle donnée selon la revendication 1 ;
ainsi que leurs sels ou N-oxydes.

3. Dérivés de triazole de formule (I) selon la revendication 1 ou 2, dans lesquels
R¹ représente phényle non substitué ou phényle qui est substitué par un ou plusieurs groupements choisis parmi halogène, C₁-C₈-alkyle ; C₁-C₈-halogénoalkyle ; C₁-C₈-halogénoalkyloxy ; C₃-C₇-cycloalkyle ; C₂-C₈-alcényle ; C₂-C₈-alcynyle ; C₂-C₈-alcényloxy ; C₃-C₈-alcynyloxy ; C₃-C₈-halogénoalcynyloxy ; C₁-C₈-alcoxy ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ; où benzyle, phényle, hétéroaryle de 5 chaînons, hétéroaryle de 6 chaînons, benzyloxy ou phényloxy peuvent être éventuellement substitués par un ou plusieurs groupements choisis parmi halogène, C₁-C₈-alkyle ; C₁-C₈-halogénoalkyle ; C₁-C₈-halogénoalkyloxy ; C₃-C₇-cycloalkyle ; C₂-C₈-alcényle ; C₂-C₈-alcynyle ; C₂-C₈-alcényloxy ; C₃-C₈-alcynyloxy ; C₃-C₈-halogénoalcynyloxy ; C₁-C₈-alcoxy ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ;
R² représente H, C₁-C₈-alkyle ; -C(O)-C₁-C₈-alkyle substitué par halogène ou C₁-C₈-alcoxy ou non substitué ;
Q représente un hétérocycle aromatique de 6 chaînons substitué contenant un atome d'azote de formule (Q-I-1) à (Q-I-3) 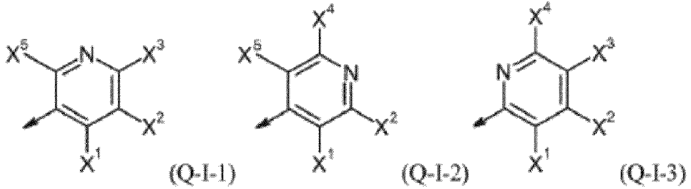 où X¹, X², X³, X⁴ ou X⁵ représentent, indépendamment les uns des autres, hydrogène, halogène, C₁-C₈-alkyle ; C₁-C₈-halogénoalkyle ; C₁-C₈-alcoxy ; ou C₁-C₈-halogénoalkyloxy ; représentent de préférence hydrogène ou halogène ;
et où au moins l'un parmi X¹, X², X³, X⁴ et X⁵ est autre qu'hydrogène ;
ainsi que leurs sels ou N-oxydes.

4. Dérivés de triazole de formule (I) selon la revendication 1, 2 ou 3, dans lesquels
R¹ représente phényle non substitué ou phényle qui est substitué par un ou plusieurs groupements choisis parmi halogène, C₁-C₈-alkyle ; C₁-C₈-halogénoalkyle ; C₁-C₈-halogénoalkyloxy ; C₃-C₇-cycloalkyle ; C₂-C₈-alcényle ;
C₂-C₈-alcynyle ; C₂-C₈-alcényloxy ; C₃-C₈-alcynyloxy ; C₃-C₈-halogénoalcynyloxy ; C₁-C₈-alcoxy ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ; où benzyle, phényle, hétéroaryle de 5 chaînons, hétéroaryle de 6 chaînons, benzyloxy ou phényloxy peuvent être éventuellement substitués par un ou plusieurs groupements choisis parmi halogène, C₁-C₈-alkyle ; C₁-C₈-halogénoalkyle ; C₁-C₈-halogénoalkyloxy ; C₃-C₇-cycloalkyle ; C₂-C₈-alcényle ; C₂-C₈-alcynyle ; C₂-C₈-alcényloxy ; C₃-C₈-alcynyloxy ; C₃-C₈-halogénoalcynyloxy ; C₁-C₈-alcoxy ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ;
R² représente H, C₁-C₈-alkyle ; -C(O)-C₁-C₈-alkyle substitué par halogène ou C₁-C₈-alcoxy ou non substitué ;
Q représente un hétérocycle aromatique de 6 chaînons substitué contenant un atome d'azote de formule (Q-I-1) à (Q-I-2) 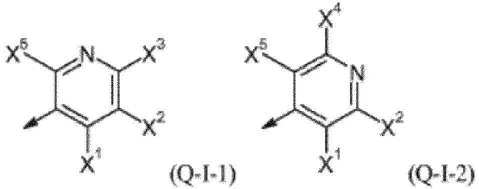 où X¹, X², X³, X⁴ ou X⁵ représentent, indépendamment les uns des autres, hydrogène, halogène, C₁-C₈-alkyle ; C₁-C₈-halogénoalkyle ; C₁-C₈-alcoxy ; ou C₁-C₈-halogénoalkyloxy ; représentent de préférence hydrogène ou halogène ;
et où au moins l'un parmi X¹, X², X³, X⁴ et X⁵ est autre qu'hydrogène ;
ainsi que leurs sels ou N-oxydes.

5. Méthode de contrôle de microorganismes néfastes dans le cadre de la protection des cultures et de la protection de matériaux, **caractérisée en ce que** des composés de formule (I) selon la revendication 1, 2, 3 ou 4, sont appliqués aux microorganismes néfastes et/ou à leur habitat.

6. Méthode de contrôle de champignons phytopathogènes néfastes, **caractérisée en ce que** des composés de formule (I) selon la revendication 1, 2, 3 ou 4, sont appliqués aux champignons phytopathogènes néfastes et/ou à leur habitat.

7. Composition destinée au contrôle de microorganismes néfastes dans le cadre de la protection des cultures et de la protection de matériaux, préférablement au contrôle de champignons phytopathogènes néfastes, **caractérisée par** une teneur en au moins un composé de formule (I) selon la revendication 1, 2, 3 ou 4, outre des matières de charge et/ou des agents tensioactifs.

8. Composition selon la revendication 7, comprenant au moins un autre ingrédient actif choisi dans le groupe constitué par les insecticides, les attractifs, les stérilisants, les bactéricides, les acaricides, les nématicides, les fongicides, les substances de croissance, les herbicides, les fertilisants, les phytoprotecteurs et les composés sémiochimiques.

9. Utilisation de composés de formule (I) selon la revendication 1, 2, 3 ou 4, pour le contrôle de microorganismes néfastes, préférablement de champignons phytopathogènes néfastes, dans le cadre de la protection des cultures et de la protection de matériaux.

10. Procédé de production de compositions destinées au contrôle de microorganismes néfastes, préférablement au contrôle de champignons phytopathogènes néfastes, **caractérisé en ce que** des composés de formule (I) selon la revendication 1, 2, 3 ou 4, sont mélangés avec des matières de charge et/ou des agents tensioactifs.

11. Utilisation de composés de formule (I) selon la revendication 1, 2, 3 ou 4, pour le traitement de plantes transgéniques.

12. Utilisation de composés de formule (I) selon la revendication 1, 2, 3 ou 4, pour le traitement de semences et de semences de plantes transgéniques.

13. Epoxydes de formule (XII) 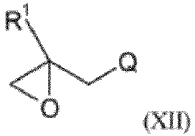 dans lesquels Q représente un hétérocycle aromatique de 6 chaînons substitué de formule (Q-I) contenant un atome d'azote 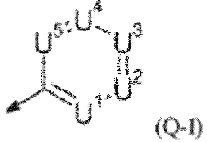 où
U¹ représente CX¹ ou N ;
où X¹ représente hydrogène, halogène, C₁-C₈-alkyle ; C₁-C₈-halogénoalkyle ; C₁-C₈-alcoxy ; ou C₁-C₈-halogénoalkyloxy ;
U² représente CX² ou N ;
où X² représente hydrogène, halogène, C₁-C₈-alkyle ; C₁-C₈-halogénoalkyle ; C₁-C₈-alcoxy ; ou C₁-C₈-halogénoalkyloxy ;
U³ représente CX³ ou N ;
où X³ représente hydrogène, halogène, C₁-C₈-alkyle ; C₁-C₈-halogénoalkyle ; C₁-C₈-alcoxy ; ou C₁-C₈-halogénoalkyloxy ;
U⁴ représente CX⁴ ou N ;
où X⁴ représente hydrogène, halogène, C₁-C₈-alkyle ; C₁-C₈-halogénoalkyle ; C₁-C₈-alcoxy ; ou C₁-C₈-halogénoalkyloxy ;
U⁵ représente CX⁵ ou N ;
où X⁵ représente hydrogène, halogène, C₁-C₈-alkyle ; C₁-C₈-halogénoalkyle ; C₁-C₈-alcoxy ; ou C₁-C₈-halogénoalkyloxy ;
et où l'un parmi U¹, U², U³, U⁴ et U⁵ représente N et où au moins l'un parmi X¹, X², X³, X⁴ et X⁵ est autre qu'hydrogène ;
et
R¹ représente phényle ou naphtyle ; où phényle ou naphtyle peut être non substitué ou substitué par un ou plusieurs groupements choisis parmi halogène ; hydroxyle ; cyano ; amino ; sulfanyle ; pentafluoro-λ⁶-sulfanyle ; carboxaldéhyde, hydroxycarbonyle, C₁-C₈-alkyle ; C₁-C₈-halogénoalkyle ; C₁-C₈-cyanoalkyle ; C₁-C₈-alkyloxy ; C₁-C₈-halogénoalkyloxy ; tri(C₁-C₈-alkyl)silyle ; tri (C₁-C₈-alkyl) silyl-C₁-C₈-alkyle ; C₃-C₇-cycloalkyle ; C₃-C₇-halogénocycloalkyle ; C₃-C₇-cycloalcényle ; C₃-C₇-halogénocycloalcényle ; C₄-C₁₀-cycloalkylalkyle ; C₄-C₁₀-halogénocycloalkylalkyle ; C₆-C₁₂-cycloalkylcycloalkyle ; C₁-C₈-alkyl-C₃-C₇-cycloalkyle ; C₁-C₈-alcoxy-C₃-C₇-cycloalkyle ; tri (C₁-C₈-alkyl) silyl-C₃-C₇-cycloalkyle ; C₂-C₈-alcényle ; C₂-C₈-alcynyle ; C₂-C₈-alcényloxy ; C₂-C₈-halogénoalcényloxy ; C₃-C₈-alcynyloxy ; C₃-C₈-halogénoalcynyloxy ; C₁-C₈-alkylamino ; C₁-C₈-halogénoalkylamino ; C₁-C₈-cyanoalcoxy ; C₄-C₈-cycloalkylalcoxy ; C₃-C₆-cycloalcoxy ; C₁-C₈-alkylsulfanyle ; C₁-C₈-halogénoalkylsulfanyle ; C₁-C₈-alkylcarbonyle ; C₁-C₈-halogénoalkylcarbonyle ; arylcarbonyle ; C₃-C₈-cycloalkylcarbonyle ; C₃-C₈-halogénocycloalkylcarbonyle ; C₁-C₈-alkylcarbamoyle ; di-C₁-C₈-alkylcarbamoyle ; N-C₁-C₈-alkyloxycarbamoyle ; C₁-C₈-alcoxycarbamoyle ; N-C₁-C₈-alkyl-C₁-C₈-alcoxycarbamoyle ; C₁-C₈-alcoxycarbonyle ; C₁-C₈-halogénoalcoxycarbonyle ; C₃-C₈-cycloalcoxycarbonyle ; C₂-C₈-alcoxyalkylcarbonyle ; C₂-C₈-halogénoalcoxyalkylcarbonyle ; C₃-C₁₀-cycloalcoxyalkylcarbonyle ; C₁-C₈-alkylaminocarbonyle ; di-C₁-C₈-alkylaminocarbonyle ; C₃-C₈-cycloalkylaminocarbonyle ; C₁-C₈-alkylcarbonyloxy ; C₁-C₈-halogénoalkylcarbonyloxy ; C₃-C₈-cycloalkylcarbonyloxy ; C₁-C₈-alkylcarbonylamino ; C₁-C₈-halogénoalkylcarbonylamino ; C₁-C₈-alkylaminocarbonyloxy ; di-C₁-C₈-alkylaminocarbonyloxy ; C₁-C₈-alkyloxycarbonyloxy ; C₁-C₈-alkylsulfinyle ; C₁-C₈-halogénoalkylsulfinyle ; C₁-C₈-alkylsulfonyle ; C₁-C₈-halogénoalkylsulfonyle ; C₁-C₈-alkylsulfonyloxy ; C₁-C₈-halogénoalkylsulfonyloxy ; C₁-C₈-alkylaminosulfamoyle ; di-C₁-C₈-alkylaminosulfamoyle ; (C₁-C₈-alcoxyimino)-C₁-C₈-alkyle ; (C₃-C₇-cycloalcoxyimino) -C₁-C₈-alkyle ; hydroxyimino-C₁-C₈-alkyle ; (C₁-C₈-alcoxyimino) -C₃-C₇-cycloalkyle ; hydroxyimino-C₃-C₇-cycloalkyle ; (C₁-C₈-alkylimino)-oxy ; (C₁-C₈-alkylimino)-oxy-C₁-C₈-alkyle ; (C₃-C₇-cycloalkylimino) -oxy-C₁-C₈-alkyle ; (C₁-C₆-alkylimino)-oxy-C₃-C₇-cycloalkyle ; (C₁-C₈-alcényloxyimino)-C₁-C₈-alkyle ; (C₁-C₈-alcynyloxyimino)-C₁-C₈-alkyle ; (benzyloxyimino)-C₁-C₈-alkyle ; C₁-C₈-alcoxyalkyle ; C₁-C₈-alkylthioalkyle ; C₁-C₈-alcoxyalcoxyalkyle ; C₁-C₈-halogénoalcoxyalkyle ; benzyle ; phényle ; hétéroaryle de 5 chaînons ; hétéroaryle de 6 chaînons ; benzyloxy ; phényloxy ; benzylsulfanyle ; benzylamino ; phénylsulfanyle ; ou phénylamino ;
ainsi que leurs sels ou N-oxydes.
